# FASCICULE DE BREVET EUROPEEN

(11) **EP 3 728 632 B1**
(45) Date de publication et mention de la délivrance du brevet: **12.07.2023**
(21) Numéro de dépôt: 18842809.8
(22) Date de dépôt: 21.12.2018
(51) Int. Cl.: C12Q 1/6806, G01N 33/53

(54) **MOLECULE D'ADN DOUBLE-BRIN POUR LA DETECTION ET LA CARACTERISATION DES INTERACTIONS MOLECULAIRES**
DOPPELSTRÄNGIGES DNA-MOLEKÜL ZUR DETEKTION UND CHARAKTERISIERUNG VON MOLEKULAREN WECHSELWIRKUNGEN
DOUBLE-STRANDED DNA MOLECULE FOR DETECTING AND CHARACTERIZING MOLECULAR INTERACTIONS

(30) Priorité: 21.12.2017 FR 1762848
(43) Date de publication de la demande: 28.10.2020
(73) Titulaire: Paris Sciences et Lettres, 75006 Paris (FR); Centre National de la Recherche Scientifique (CNRS), 75016 Paris (FR); Ecole Normale Supérieure, 75005 Paris (FR); Université Paris Cité, 75006 Paris (FR); Institut National de la Santé et de la Recherche Médicale (INSERM), 75013 Paris (FR)
(72) Inventeur: STRICK, Térence, 75007 PARIS (FR); GOSSE, Charlie, 75014 PARIS (FR); KOSTRZ, Dorota, 75012 PARIS (FR); WANG, Jinglong, 75013 PARIS (FR); NADAL, Marc, 75012 PARIS (FR)
(74) Mandataire: Regimbeau
(86) Numéro de dépôt international: PCT/FR2018/053533
(87) Numéro de publication internationale: WO 2019/122793

(56) Documents cités:
- WO-A1-2016/089588
- WO-A1-2017/055463
- JONGSEONG KIM ET AL: "A mechanically stabilized receptor-ligand flex-bond important in the vasculature", NATURE, vol. 466, no. 7309, 1 août 2010 (2010-08-01), pages 992-995, XP055501167, GB ISSN: 0028-0836, DOI: 10.1038/nature09295 cité dans la demande
- RYU TASHIRO ET AL: "Linking two DNA duplexes with a rigid linker for DNA nanotechnology", NUCLEIC ACIDS RESEARCH, vol. 43, no. 14, 30 juin 2015 (2015-06-30) , pages 6692-6700, XP055501482, ISSN: 0305-1048, DOI: 10.1093/nar/gkv662

## Description

### DOMAINE DE L'INVENTION

La présente invention se rapporte au domaine de la micromanipulation des molécules. Plus particulièrement, la présente invention se rapporte à des molécules et des procédés permettant de détecter et de caractériser des interactions moléculaires à l'échelle d'une seule molécule.

### ART ANTERIEUR

Les techniques de micromanipulation, tels que les pinces optiques et la microscopie à force atomique, permettent de caractériser les interactions moléculaires entre différentes molécules à tester et de déterminer des informations thermodynamiques et cinétiques à l'échelle d'une seule molécule, ceci notamment en fonction d'une force appliquée. Ces techniques sont en plein essor du fait qu'elles nécessitent peu de réactifs par rapport aux méthodes plus classiques (telle que celles faisant intervenir des « *biosensors* » basés sur la résonance plasmonique de surface ou l'interférométrie optique), et qu'elles permettent d'obtenir des informations mécanistiques de meilleure qualité du fait de l'individualisation des suivis moléculaires.

La détection des interactions moléculaires par des techniques de micromanipulation nécessite le plus souvent un accrochage des molécules à tester entre deux supports. S'il est envisageable qu'au moins une des molécules à tester puisse être fixée directement à un support, cela n'est pas souhaitable car ceci est source d'artefacts compte tenu des interactions non-spécifiques de type support-support ou molécule-support qui peuvent avoir lieu. Afin de réduire les artéfacts, différentes molécules ont été décrites permettant d'accrocher des molécules à tester de façon indirecte aux supports, notamment par le biais de molécules peptidiques et/ou nucléotidiques (voir, par exemple, Gao et al., 2012). Ces molécules peuvent elles-mêmes être reliées l'une à l'autre de façon directe ou indirecte, par exemple par une tierce molécule, appelée ici une « *longe* » (voir, par exemple, Kim et al., 2010 ; Halvorsen et al., 2011 ; Rognoni et al., 2012 ; Kilchherr et al., 2016). Ainsi une molécule utilisée pour étudier des interactions moléculaires par la micromanipulation correspond généralement à une molécule dans laquelle plusieurs *« sous-molécules* » sont combinées, dont au moins deux molécules à tester.

Les molécules actuellement utilisées pour étudier des interactions moléculaires peuvent être regroupées dans quatre catégories distinctes en fonction de la composition de la molécule (e.g. comprenant des éléments polypeptidiques et/ou polynucléotidiques), comme décrit ci-après.

La première catégorie de molécules comprend des molécules qui sont composées uniquement d'acides aminés, dont des polypeptides et/ou des protéines. Plus précisément, les molécules à tester sont fixées à deux supports différents au moyen de protéines globulaires (voir, par exemple, Wiita et al., 2006 ; Popa et al., 2011). Généralement, les molécules à tester sont liées à des protéines globulaires et/ou à une longe polypeptidique au sein d'une protéine de fusion. Cependant, l'utilisation de protéines de fusion limite le type de molécules à tester pouvant être intégrées aux seules molécules de nature protéique. De plus, ces molécules nécessitent un important effort de préparation à chaque fois que les molécules à tester sont modifiées du fait que la longe et/ou les protéines globulaires sont exprimées et purifiées en même temps que les molécules à tester. De plus, en fonction de la conformation des protéines, le taux d'interaction positive entre les molécules à tester peut être diminué de façon artificielle à cause de contraintes conformationnelles (e.g. d'orientation, d'accessibilité) liées à la structure tridimensionnelle de la molécule. Enfin, afin de réduire les interactions entre la longe et les autres éléments de la molécule, et d'empêcher la formation de structures secondaires parasites, particulièrement lors de l'expression, la longueur de la longe polypeptidique est limitée à environ 100 acides aminés et sa séquence doit être soigneusement choisie. Cette limitation de longueur augmente également la concentration effective en molécules à tester, qui est déterminée par la distance moyenne entre ces deux molécules. À titre d'exemple, la concentration effective des molécules à tester est de l'ordre de 1 mM lorsqu'une longe polypeptidique d'une cinquantaine d'acides aminés est utilisée, ce qui fait qu'uniquement les associations moléculaires ayant des constantes de vitesse suffisamment lentes peuvent être étudiées. À titre d'exemple, pour une résolution temporelle donnée, qui permet de détecter les changements de conformations en 10 ms, la valeur limite au-delà de laquelle les constantes de vitesse ne peuvent plus être mesurées est de 10⁵ M⁻¹s⁻¹.

La deuxième catégorie de molécules comprend des molécules d'ADN double-brin, reliées l'une à l'autre par une longe polypeptidique (voir, par exemple, Rognoni et al, 2012 ; Kim et al., 2010). Les contraintes associées à l'utilisation d'une longe polypeptidique restent donc les mêmes ici que pour les molécules de nature peptidique de la première catégorie. De plus, compte tenu du faible nombre d'outils disponibles à ce jour permettant de modifier les liens peptidiques, les molécules à tester doivent être, encore une fois, exclusivement de nature protéique et exprimées en même temps que la longe (e.g. dans des protéines de fusion).

Ainsi, ces deux types de molécules sont peu souples dans leur utilisation, d'une part par la nature des molécules à tester qui peuvent être incorporées, et d'autre part par la limite imposée aux concentrations effectives les plus faibles pouvant être étudiées.

Une troisième catégorie de molécules comprend des molécules composées d'ADN simple-brin recouvert d'oligonucléotides (voir, par exemple, WO 2013/067489 ; Halvorsen et al., 2011). Dans ce cas, l'ADN simple-brin fonctionne comme une « *molécule support* » pour les oligonucléotides, qui s'hybrident et transforment l'ADN simple-brin en une structure tridimensionnelle discontinue comprenant uniquement des liaisons de type phosphodiester, selon une approche de synthèse de type « *origami* » (voir, par exemple, Rothemund, 2006). Cependant, la longueur de ce type de molécule peut difficilement dépasser la limite pratique d'environ 25 000 nucléotides, ce qui correspond à la longueur du plus grand génome d'ADN simple-brin présent dans la nature, et qui pourrait potentiellement fonctionner comme « *molécule support* » (même si, pour l'instant la technique n'a été illustrée qu'avec des « *molécules support* » dont la longueur était, au maximum, d'environ 7250 nucléotides). De plus, lorsque les oligonucléotides s'hybrident à la molécule d'ADN simple-brin il reste encore de nombreuses entailles sur toute la longueur de la structure obtenue.

Enfin, la technique d'« *origami* » reste délicate à mettre en oeuvre. Les oligonucléotides peuvent notamment avoir des structures secondaires et/ou interagir entre eux lorsqu'ils sont mélangés, et ce d'autant plus quand ils présentent une longueur importante (e.g. > 20 bases). À titre d'exemple, pour un support composé d'environ 7250 nucléotides, 121 oligonucléotides différents ayant une longueur de 60 bases ont été hybridés (Halvorsen et al., 2011). Si la probabilité qu'un oligonucléotide individuel soit correctement hybridé est élevée, même avec une probabilité de 99%, la probabilité que la molécule support comprenne bien 121 oligonucléotides hybridés n'est que de 0.99¹²¹, soit environ 30%. Si la probabilité qu'un oligonucléotide individuel soit correctement hybridé atteint les 99,9% alors la probabilité que la molécule support comprenne bien 121 oligonucléotides hybridés n'est que de 89%. Ces considérations indiquent que l'approche « *origami* » produit fréquemment des molécules d'ADN possédant :
(1) Des propriétés chimiques hétérogènes (régions hybridées double-brin intactes, régions non-hybridées simple-brin, et entailles entre deux oligonucléotides hybridés) ;
(2) Des propriétés mécaniques variables d'une molécule à une autre, la réponse mécanique précise d'une molécule donnée dépendant du rapport exact entre longueur totale des régions hybridées double-brin et longueur totale des régions non-hybridées simple brin ;
(3) Des propriétés topologiques défavorables (la molécule ne peut pas être superenroulée par exemple) ;
(4) Un risque de séparation important entre la « *molécule support* » et les oligonucléotides, si des forces élevées sont appliquées à la structure.

Il résulte de ceci une grande variabilité, d'une molécule à l'autre, des propriétés intrinsèques des molécules utilisées pour ces études. La complexité de l'assemblage de ce type de molécule fait qu'il est impossible de garantir l'homogénéité d'un ensemble de molécules d'ADN ainsi construit. Or, pour mesurer des interactions à l'échelle d'une seule molécule, il est nécessaire de pouvoir s'assurer d'un très haut niveau de qualité pour chaque molécule individuelle incorporant lesdites molécules à tester.

La quatrième catégorie de molécules comprend deux faisceaux de molécules d'ADN double-brin liées l'un à l'autre par une longe en ADN simple-brin (Kilchherr et al., 2016), comprenant ainsi uniquement des liaisons de type phosphodiester. L'assemblage d'un faisceau de molécules d'ADN dépendant également de la technique dite d'« *origami* »*,* cette molécule possède les mêmes désavantages associés à cette technique que ceux décrits ci-dessus. De plus, compte tenu du grand nombre d'oligonucléotides qui compose les faisceaux, ces compositions sont particulièrement rigides ; la liberté conformationnelle des molécules à tester est donc très éloignée de ce qu'elle est en solution. En contraste de la rigidité des faisceaux, la longe d'ADN simple-brin est flexible, composée d'une simple succession de liaisons phosphodiesters. De ce fait, il est impossible d'effectuer des études impliquant l'application d'un couple sur des molécules comprenant une longe d'ADN simple-brin. Une longe d'ADN simple-brin est également susceptible de former des structures secondaires et/ou d'interagir avec d'autres éléments de la molécule. WO 2016/089588 décrit un système pour étudier des interactions intermoléculaires, qui exploite la flexibilité de longes à ADN simple brin et leur capacité de répondre à des forces externes en changeant de structure secondaire. Cette méthode utilise une seule molécule d'ADN, ce qui diffère totalement d'un complexe comprenant deux bras et une longe de connexion.

Il existe donc, à l'heure actuelle, un besoin pour de nouvelles molécules stables et homogènes, dans lesquelles une grande diversité de molécules à tester pourrait être intégrée. Il existe également un besoin pour de nouvelles molécules dans lesquelles les interactions non-spécifiques (e.g. entre une molécule à tester et un ou plusieurs autres éléments de la molécule, telle qu'une longe) sont réduites, avantageusement dans lesquelles les interactions non-spécifiques sont entièrement absentes. Il existe également un besoin pour de nouvelles molécules qui sont à la fois flexibles, afin de minimiser les contraintes conformationnelles subies par les molécules à tester et ainsi éviter une réduction artificielle dans le taux d'interactions entre les molécules à tester, mais aussi rigides, afin de pouvoir avoir accès facilement au domaine des basses forces commençant sous environ 2 pN. En effet, les molécules actuellement utilisées, par exemple qui sont composées de faisceaux de molécules d'ADN ou de polypeptides, ne peuvent pas être utilisées pour caractériser des interactions moléculaires qui ont lieu à des basses forces (e.g. en dessous de 2 pN).

Il existe également un besoin pour de nouvelles molécules qui seraient facilement modulables. Plus précisément, il existe un besoin particulier pour de nouvelles molécules qui permettraient de mesurer des interactions entre des molécules à tester ayant une grande diversité de structures et/ou de compositions, notamment au-delà des acides nucléiques et acides aminés (e.g. polymères, petites molécules chimiques, nanoparticules, etc.). Il existe également un besoin pour de nouvelles molécules comprenant des éléments facilement ajustables en longueur (e.g. pas limitée à 25 000 nucléotides), tels que la longe et/ou les molécules reliant les molécules à tester aux supports, afin de pouvoir moduler les concentrations effectives des molécules à tester et ainsi pouvoir mesurer des constantes de vitesse d'association supérieures à environ 10⁵ M⁻¹s⁻¹.

Il existe également un besoin pour un nouveau dispositif pour la mesure des interactions moléculaires, ledit dispositif comprenant une molécule ayant au moins l'un des avantages décrits ci-dessus, ainsi qu'un nouveau procédé permettant de caractériser les interactions moléculaires par le biais d'une telle molécule ou d'un tel dispositif. Enfin, il existe un besoin pour une méthode de fabrication desdites molécules et dispositifs qui soit rapide, simple, peu coûteuse, et/ou qui implique un minimum d'étapes.

### RESUME DE L'INVENTION

La présente invention a pour objet une nouvelle molécule qui permet de répondre à l'ensemble des besoins cités ci-dessus. Plus particulièrement, la présente invention a pour objet une molécule d'ADN double-brin comprenant une première molécule d'ADN double-brin (1) reliée à une deuxième molécule d'ADN double-brin (2) par au moins une liaison covalente qui n'est pas une liaison phosphodiester, phosphorothioate, phosphoramidate ou phosphorodiamidate, avantageusement par une longe, ladite longe étant avantageusement une molécule d'ADN double-brin, ladite liaison covalente n'ayant pas lieu aux nucléotides ultimes des extrémités de la première molécule d'ADN double-brin (1).

En effet, les inventeurs ont démontré de façon avantageuse que la molécule d'ADN double-brin selon l'invention est plus homogène que les molécules connues jusqu'à présent, ce qui permet d'améliorer la qualité des résultats obtenus lorsqu'une telle molécule est utilisée pour effectuer des études d'interactions moléculaires. L'homogénéité chimique de la molécule permet d'éliminer un grand nombre d'artefacts potentiels liés aux interactions différentielles entre protéines et différents types d'acides nucléiques (simple-brin par exemple). L'homogénéité chimique de la molécule renforce également la stabilité de la molécule en réduisant le nombre de façons dont la molécule peut se dégrader. Avantageusement, la molécule d'ADN double-brin selon l'invention ne comprend ni structure secondaire, ni structure tertiaire. Les inventeurs ont également démontré, de façon surprenante, que la molécule d'ADN double-brin selon l'invention peut être utilisée pour étudier un grand nombre d'interactions différentes, avec différents types de molécules à tester (e.g. des protéines, des polynucléotides, des petites molécules chimiques) sans avoir besoin d'adapter les différents éléments de la molécule (e.g. les molécules d'ADN double-brin (1) et (2) et/ou la longe). Ceci est particulièrement avantageux par rapport aux molécules comprenant des éléments polypeptidiques telles que décrites ci-dessus (selon la première ou la deuxième catégorie de molécules).

De façon surprenante, les inventeurs ont également démontré que la molécule d'ADN double-brin selon l'invention est flexible, ce qui permet de façon avantageuse de minimiser les contraintes conformationnelles subies par les molécules à tester, et ainsi d'éviter une réduction artificielle dans le taux d'interactions entre les molécules à tester. De plus, de façon surprenante, les inventeurs ont démontré que la molécule d'ADN double-brin est aussi suffisamment rigide afin de pouvoir caractériser des interactions moléculaires ayant lieu à des basses forces commençant en-dessous environ 2 pN.

Avantageusement, l'invention a pour objet une molécule d'ADN double-brin dans laquelle ladite longe est attachée par au moins une liaison covalente qui n'est pas une liaison phosphodiester à la première molécule d'ADN double-brin (1) et par au moins une liaison covalente qui n'est pas une liaison phosphodiester à la deuxième molécule d'ADN double-brin (2).

Avantageusement, l'invention a pour objet une molécule d'ADN double-brin comprenant une première molécule d'ADN double-brin (1) reliée à une deuxième molécule d'ADN double-brin (2) par une longe comprenant de l'ADN double-brin, dans laquelle ladite longe est attachée par (i) au moins une liaison covalente à une nucléotide de la première molécule d'ADN double-brin (1) et par (ii) au moins une liaison covalente à une nucléotide de la deuxième molécule d'ADN double-brin (2), selon laquelle ladite liaison covalente de (i) et ladite liaison covalente de (ii) ne sont pas des liaisons phosphodiesters, phosphorothioate, phosphoramidates ou phosphorodiamidates, et ladite nucléotide de (i) et ladite nucléotide de (ii) ne sont pas des nucléotides ultimes desdites molécules d'ADN double-brin (1) et (2).

Avantageusement, l'invention a pour objet une molécule d'ADN double-brin dans laquelle ladite longe est attachée à la première molécule d'ADN double-brin (1) par une première liaison covalente entre la première extrémité de ladite longe et une région intermédiaire de la première molécule d'ADN double-brin (1) et à la deuxième molécule d'ADN double-brin (2) par une deuxième liaison covalente entre la deuxième extrémité de ladite longe et une région intermédiaire de la deuxième molécule d'ADN double-brin (2).

Avantageusement, l'invention a pour objet une molécule d'ADN double-brin dans laquelle une première molécule à tester est liée à une première extrémité de la ladite première molécule d'ADN double-brin (1) et une deuxième molécule à tester est liée à une première extrémité de la ladite deuxième molécule d'ADN double-brin (2).

Avantageusement, l'invention a pour objet une molécule d'ADN double-brin dans laquelle la deuxième extrémité de ladite première molécule d'ADN double-brin (1) est liée à un premier support et la deuxième extrémité de ladite deuxième molécule d'ADN double-brin (2) est liée à un deuxième support, avantageusement dans laquelle au moins un des deux supports est un support mobile.

De façon avantageuse, il y a une grande liberté dans le choix des supports auxquels la molécule d'ADN double-brin peut être attachée.

De façon avantageuse, la longueur de ladite première molécule d'ADN double-brin (1) et/ou ladite deuxième molécule d'ADN double-brin (2) et/ou ladite longe peuvent être facilement ajustée. Ceci permet notamment d'optimiser la résolution des mesures d'extension lors de la caractérisation des interactions entre molécules à tester. Ceci permet également de faciliter la distinction entre les signaux qui résultent d'une interaction spécifique entre deux molécules à tester et les signaux qui résultent d'une interaction non-spécifique (e.g. entre des molécules à tester et les surfaces ou supports environnantes). De façon particulièrement avantageuse, la longueur de ladite longe peut être facilement ajustée afin de pouvoir moduler les concentrations effectives des molécules à tester (C*_{eff}*) et notamment permettre de déterminer des constantes de vitesse d'association supérieures à environ 10⁵ M⁻¹s⁻¹. Ceci est d'ailleurs d'autant plus facile que l'ADN double-brin a une grande longueur de persistance (environ 50 nm, soit environ 160 paires de bases).

Selon un mode préférentiel, l'invention a pour objet une molécule d'ADN double-brin dans laquelle :
- ladite première molécule d'ADN double-brin (1) et/ou ladite deuxième molécule d'ADN double-brin (2) a une longueur de 300 à 5000 paires de bases, de préférence de 650 à 1500 paires de bases ;
- la première extrémité de la première molécule d'ADN double-brin (1) et/ou la première extrémité de la deuxième molécule d'ADN double-brin (2) à une longueur de 10 à 150 paires de bases, avantageusement de 30 à 50 paires de bases ; et/ou
- ladite longe à une longueur d'environ 300 à environ 50 000 paires de bases, avantageusement d'environ 500 à 10 000 paires de bases, plus avantageusement d'environ 600 à 3000 paires de bases.

Avantageusement, l'invention a pour objet une molécule d'ADN double-brin dans laquelle ladite première et/ou ladite deuxième molécule à tester est sélectionnée dans le groupe composé des molécules suivantes : polymères, acides aminés, peptides, polypeptides, protéines, nucléosides, nucléotides, polynucléotides, oligonucléotides, sucres, polysaccharides, petites molécules, drogues, aptamères, antigènes, anticorps, lipides, lectines, hormones, vitamines, virus, fragments de virus, nanoparticules, molécules cellulaires de surface, et facteurs de transcription.

Avantageusement, l'invention a pour objet une molécule d'ADN pour une utilisation dans la caractérisation des interactions entre au moins deux molécules à tester.

Avantageusement, l'invention a pour objet un dispositif comprenant la molécule d'ADN double-brin décrit ici avec ses supports.

Avantageusement, l'invention a pour objet une molécule d'ADN double-brin comprenant une première molécule d'ADN double-brin (A) et une deuxième molécule d'ADN double-brin (B), ladite molécule (A) comprenant un site de clivage qui est présent uniquement dans ladite molécule d'ADN double-brin (A), ladite molécule d'ADN double-brin (A) étant reliée à la molécule d'ADN double-brin (B) par deux liaisons covalentes qui ne sont pas des liaisons phosphodiesters, phosphorothioates, phosphoramidates ou phosphorodiamidates, de part et d'autre dudit site de clivage, dans laquelle lesdites liaisons covalentes n'ont pas lieu aux nucléotides ultimes des extrémités de ladite molécule d'ADN double-brin (A).

La molécule d'ADN double-brin comprenant une première molécule d'ADN double-brin (1) reliée à une deuxième molécule d'ADN double-brin (2) par au moins une liaison covalente qui n'est pas une liaison phosphodiester, une liaison phosphorothioate, une liaison phosphoramidate ou une liaison phosphorodiamidate selon la présente invention est avantageusement fabriquée à partir de la molécule d'ADN double-brin comprenant une première molécule d'ADN double-brin (A) et une deuxième molécule d'ADN double-brin (B) telle que décrite ci-dessus, appelée la molécule d'ADN « *intermédiaire* »*.*

Aussi, l'invention a pour objet un procédé de fabrication de la molécule d'ADN double-brin telle que décrite ici, caractérisé en ce qu'il comprend l'étape suivante :
a) le clivage de la molécule d'ADN double-brin (A) (présente dans la molécule d'ADN « *intermédiaire* » telle que décrite ci-dessus) audit site de clivage, générant ainsi une molécule d'ADN comprenant une première molécule d'ADN double-brin (1) et une deuxième molécule d'ADN double-brin (2).

Un tel procédé de fabrication est très avantageux car très facile à mettre en oeuvre. De plus, de façon avantageuse, la molécule « *intermédiaire* » est obtenue avec un rendement satisfaisant (e.g. d'au moins 8 %). De façon avantageuse, la molécule « *intermédiaire* » ouvre sur une succession d'étapes de synthèse qui conduisent, avec des rendements de plus de 90 %, à la molécule d'ADN double-brin selon l'invention (données non démontrées).

Selon un aspect particulier, l'invention a pour objet la molécule d'ADN double-brin obtenue par ce procédé.

La présente invention a également pour objet un procédé de caractérisation d'une interaction entre au moins deux molécules à tester liées à une molécule d'ADN double-brin ou telle que comprise dans le dispositif, comprenant :
a) l'application d'une force physique basse, F_{LF}, à la molécule d'ADN double-brin, qui permet aux molécules à tester de s'associer ;
b) l'application d'une force physique haute, F_{HF}, à la molécule d'ADN double-brin, qui permet de déterminer si les molécules à tester sont associées ou dissociées ; et
c) la détection d'un changement dans les propriétés conformationnelles de la molécule d'ADN comprenant :
   - la détermination de l'extension z_{LF} entre la deuxième extrémité de la première molécule d'ADN double-brin (1) et la deuxième extrémité de la deuxième molécule d'ADN double-brin (2) à l'étape a) ;
   - la détermination des extensions z_{HF-A} et z_{HF-D} entre la deuxième extrémité de la première molécule d'ADN double-brin (1) et la deuxième extrémité de la deuxième molécule d'ADN double-brin (2), à l'étape b) ; et
   - la comparaison des extensions z_{LF}, z_{HF-A}, et z_{HF-D}, en fonction du temps t.

Avantageusement, l'invention a pour objet un procédé selon lequel la force physique à l'étape a) est de 0,01 picoNewton (pN) à 0,4 pN et/ou selon lequel la force physique à l'étape b) est de 0,5 à 70 pN, avantageusement de 0,5 à 40 pN.

Avantageusement, l'invention a pour objet un procédé selon lequel la caractérisation de l'interaction comprend la détermination d'au moins une des caractéristiques choisies parmi : le temps caractéristique d'association, le temps caractéristique de dissociation, la constante de vitesse de dissociation, l'énergie d'activation de dissociation, la distance séparant l'état de transition du complexe lors de la dissociation, et la constante d'équilibre de dissociation.

En effet, les inventeurs ont mis en évidence que la molécule d'ADN double-brin selon l'invention est particulièrement adaptée à une utilisation dans des dispositifs et des méthodes permettant de caractériser des interactions moléculaires entre au moins deux molécules à tester.

### DESCRIPTION DES FIGURES

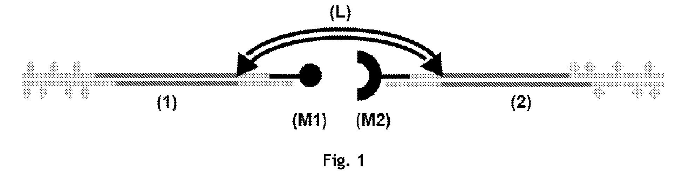

**Figure 1****.** Représentation conceptuelle d'une molécule d'ADN double-brin selon l'invention comprenant une première molécule d'ADN double-brin (1) reliée à une deuxième molécule d'ADN double-brin (2) par une longe (L), dans laquelle une première molécule à tester (M1) est liée à une première extrémité de la première molécule d'ADN double-brin (1) et une deuxième molécule à tester (M2) est liée à une première extrémité de la deuxième molécule d'ADN double-brin (2). Des groupes fonctionnels sont présents sur la deuxième extrémité de la première molécule d'ADN double-brin (1) et sur la deuxième extrémité de la deuxième molécule d'ADN double-brin (2) (ovales et losanges, respectivement), permettant leur attachement aux supports. Les deux extrémités des molécules d'ADN double-brin (1) et (2) sont illustrées en gris clair, et la région intermédiaire de chaque molécule est illustrée en gris foncé. La longe est représentée en noir. Les liaisons covalentes non-phosphodiesters entre la longe (L) et les molécules d'ADN double-brin (1) ou (2) sont illustrées par les triangles.

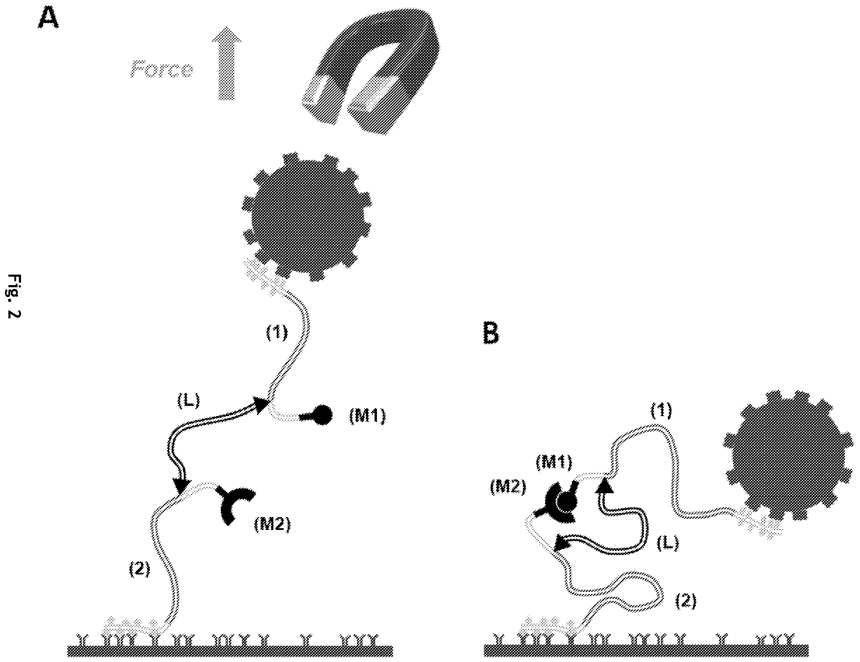

**Figure 2****.** Représentation conceptuelle d'un dispositif comprenant une molécule d'ADN double-brin selon la **Figure 1** et ses supports, dans lequel **A**) les deux molécules à tester, (M1) et (M2), sont dissociées et **B**) les deux molécules à tester, (M1) et (M2), sont associées.

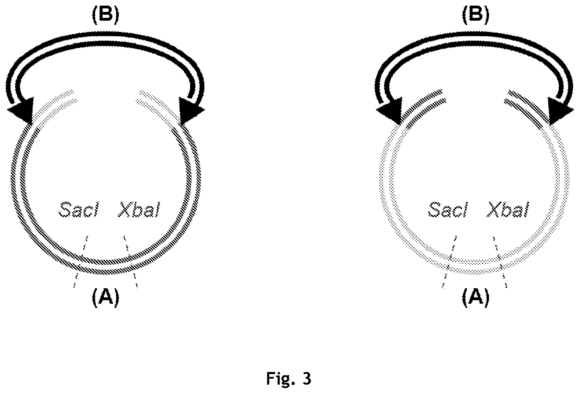

**Figure 3****.** Représentations conceptuelles d'une molécule d'ADN double-brin « *intermédiaire* » comprenant une première molécule d'ADN double-brin (A) et une deuxième molécule d'ADN double-brin (B). La molécule (B) correspond à la longe. La molécule (A) correspond au précurseur des molécules d'ADN double brin (1) et (2). À titre d'exemple, les régions de la molécule d'ADN double-brin (A) pouvant correspondre aux extrémités des deux molécules d'ADN double-brin (1) et (2) à lesquelles des molécules à tester peuvent être liées sont illustrées en gris clair, selon deux configurations différentes.

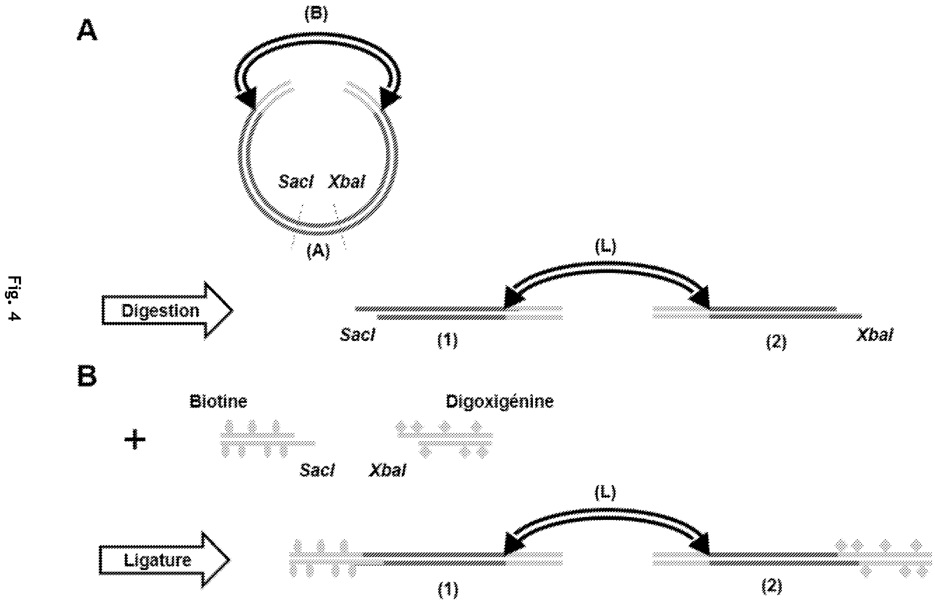

**Figure 4****.** Schéma d'une méthode de fabrication d'une molécule d'ADN double-brin à partir d'une molécule d'ADN double-brin « *intermédiaire* », comprenant les étapes **A**) Synthèse des molécules d'ADN double-brin (1) et (2) ; **B**) Synthèse et assemblage des extrémités fonctionnalisées destinées aux supports.

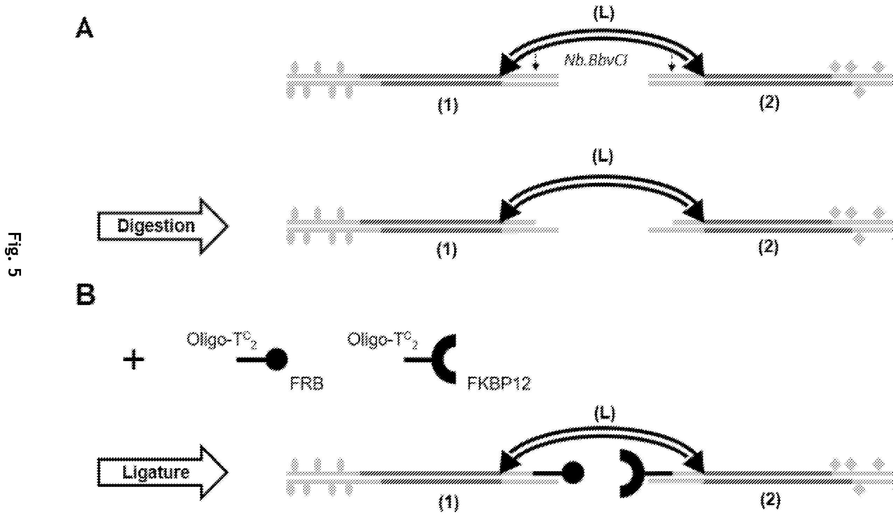

**Figure 5****.** Schéma de l'aménagement des extrémités destinées aux molécules à tester, comprenant les étapes **A**) Digestion des extrémités (par exemple par l'enzyme *Nb.BbvCI*) ; **B**) Liaison des molécules à tester aux extrémités.

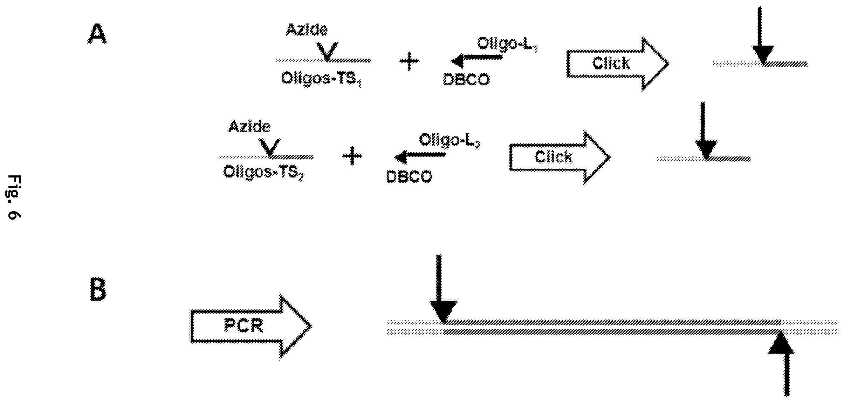

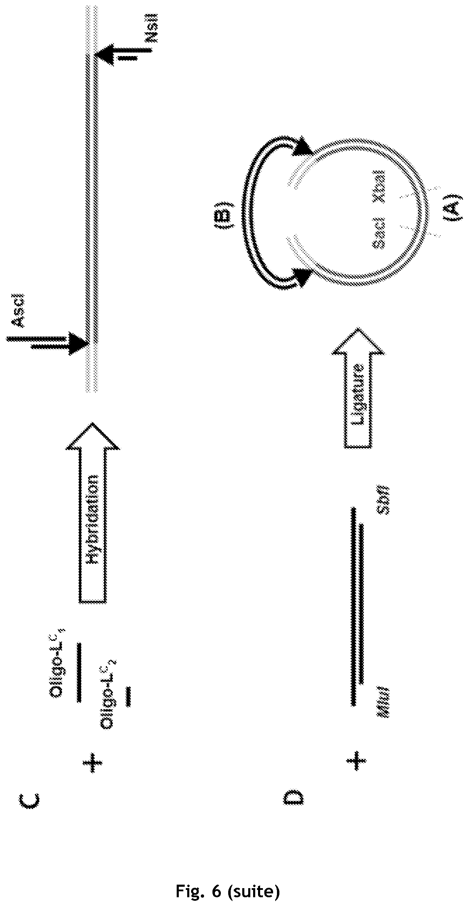

**Figure 6****.** Schéma d'un procédé de fabrication d'une molécule d'ADN double-brin « *intermédiaire* », comprenant les étapes **A**) Synthèse des jonctions ; **B**) Synthèse de la molécule (A), correspondant à un précurseur des première et deuxième molécules d'ADN double-brin (1) et (2) ; **C**) Synthèse et assemblage de la molécule (B), correspondant à la longe.

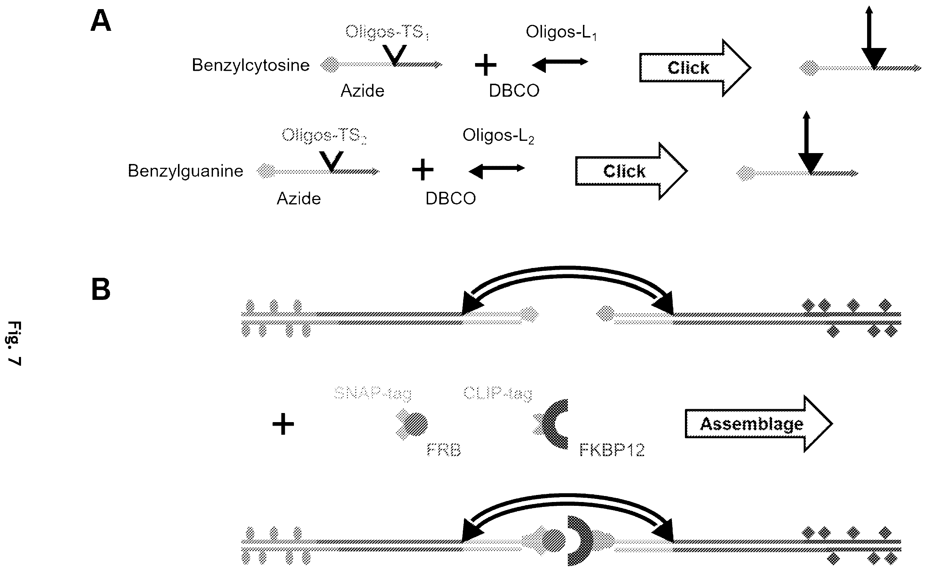

**Figure 7****.** Schéma de l'aménagement des extrémités destinées aux molécules à tester, comprenant les étapes **A**) Synthèse des jonctions, **B**) Liaison des molécules à tester aux extrémités.

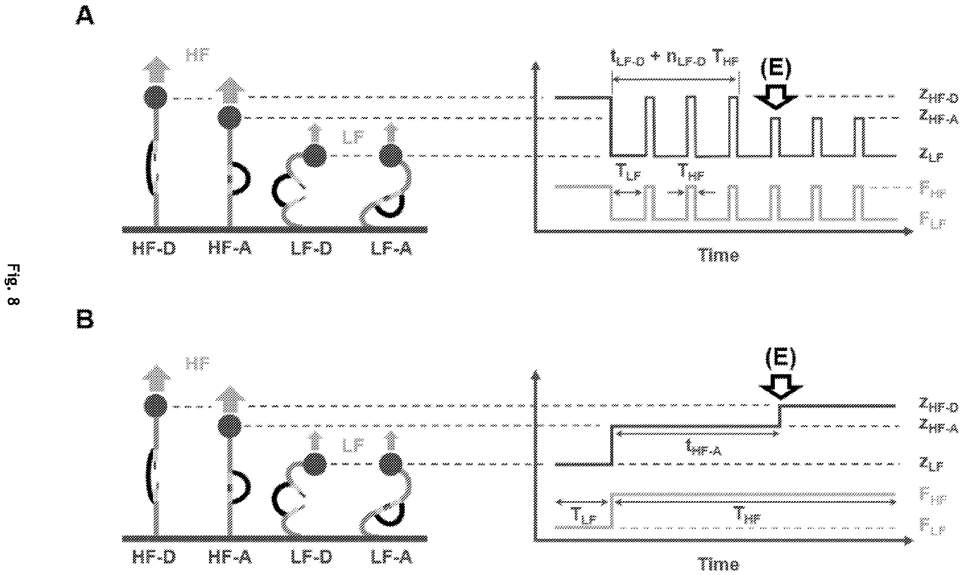

**Figure 8****.** Principe de mesure, par cyclage de la force, du temps de vie, t, **A)** pour les molécules à tester sous leur forme dissociée et **B)** pour les molécules à tester sous leur forme associée. Un seul cycle de mesure est ici représenté pour chacune des techniques ; on obtient donc un unique temps de vie pour les molécules à tester accrochées à la molécule unique d'ADN considérée. La conformation de la molécule d'ADN peut être déterminée à partir de l'enregistrement au cours du temps de l'extension, z, lorsque la force F est cyclée entre une valeur haute (F_{HF}) et une valeur basse (F_{LF}), pour des durées T (T_{HF}/T_{LF}, respectivement). HF correspond à l'application d'une force haute (« *High Force* ») ; LF correspond à l'application d'une force basse (« *Low Force* »). Les molécules à tester peuvent être soit associées (A pour « *Associated* ») ; soit dissociées (D pour « *Dissociated* »). Le repérage sur les traces temporelles d'événement de changement d'extension (E) permet de déterminer t. « n » correspond au nombre de cycles effectués avant détection d'un événement dans les expériences d'association. Plus précisément la mesure du temps de vie pour les molécules à tester sous forme dissociée, t_{LF-D}, passe par la détermination de n_{LF-D}, le nombre de cycles effectués avant détection d'un événement et la multiplication de celui par T_{LF}. La mesure du temps de vie pour les molécules sous forme associée, t_{HF-A}, se fait quant à elle directement sur le suivi temporel.

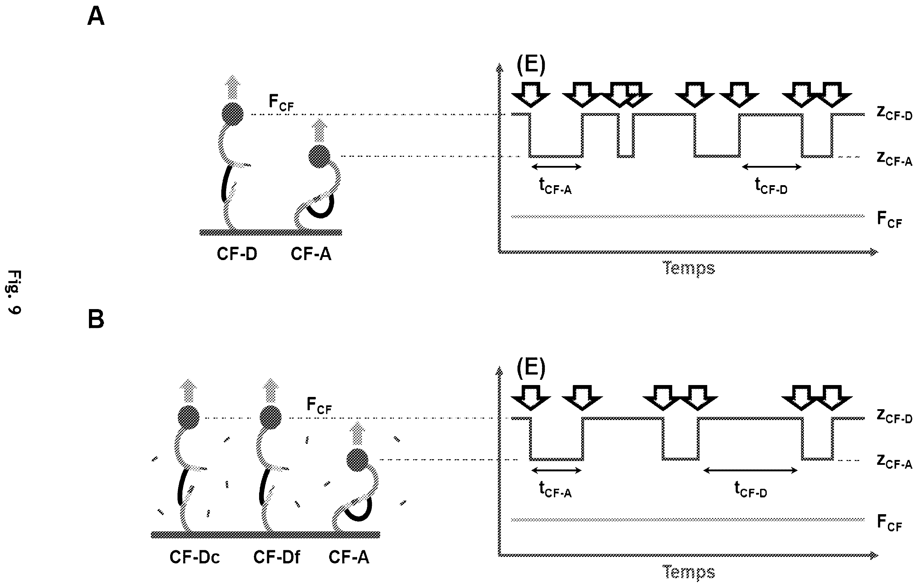

**Figure 9****.** Principe de mesure, par enregistrement des fluctuations spontanées observées à une force constante, F_{CF}, du temps de vie pour les molécules à tester sous leur forme dissociée, t_{CF-D}, et pour les molécules à tester sous leur forme associée, t_{CF-A}. Une fraction de suivi temporel est ici représentée pour chacun des deux modes d'acquisition considérés **A)** en l'absence et **B)** en présence, en solution, de molécules similaires à l'une des molécules à tester. La conformation de la molécule d'ADN peut être déterminée à partir de l'enregistrement au cours du temps de l'extension, z. CF correspond à l'application d'une force constante (« *Constant Force* »). Les molécules à tester peuvent être soit associées (A pour « *Associated* ») ; soit dissociées (D pour « *Dissociated* »). Lorsque les molécules à tester sont dissociées il se peut qu'elles soient toutes les deux non complexées (Df, D pour « *Dissociated* » et f pour « *free* ») ou que l'une d'entre-elles forme un complexe avec l'une des molécules présentes en solution (Dc, D pour « *Dissociated »* et c pour « *complexed* »)*.* Le repérage sur les traces temporelles d'événement de changement d'extension (E) permet de déterminer t.

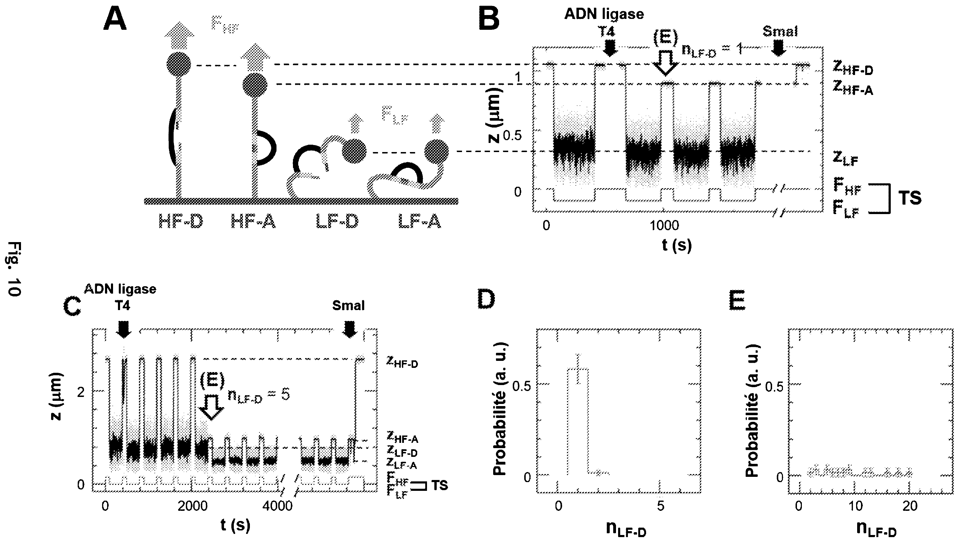

**Figure 10****.** Étude de l'interaction covalente entre deux bouts cohésifs des extrémités d'ADN double-brin en présence de l'ADN ligase du phage T4 puis de l'enzyme de restriction Smal. Ces deux bouts cohésifs, où des groupes phosphates terminaux sont présents en 5' et des groupes hydroxyles terminaux sont présents en 3', constituent eux-mêmes ici les deux molécules à tester. La molécule d'ADN utilisée correspond à celle schématisée dans la **Figure 1** dans un dispositif tel qu'illustré dans la **Figure 2****,** et obtenu par le procédé de fabrication décrit dans les Exemples 3, 1 puis 8. Les expériences sont réalisées à 34° C dans le tampon RB. **A**) Schéma des quatre configurations sous lesquelles peut se trouver la molécule d'ADN. Le dispositif peut être soumis soit à une force haute (HF pour « *High Force* ») ; soit à une force basse (LF pour « *Low Force* »). Les molécules à tester peuvent être soit associées (A pour « *Associated* ») ; soit dissociées (D pour « *Dissociated* »). B) Suivi temporel de l'extension (z) de la molécule d'ADN possédant une longe de ~ 0,7 kbp lorsque la force est cyclée entre une force haute F_{HF} = 1,4 pN pendant un temps T_{HF} = 100 s et une force basse F_{LF} = 0,04 pN pendant un temps T_{LF} = 300 s (TS : tracé schématique des cycles F_{HF}/F_{LF}). C) Suivi temporel de l'extension (z) de la molécule d'ADN possédant une longe de ~ 6 kbp lorsque la force est cyclée entre une force haute F_{HF} = 1,1 pN pendant un temps T_{HF} = 100 s et une force basse F_{LF} = 0,04 pN pendant un temps T_{LF} = 300 s (TS : tracé schématique des cycles F_{HF}/F_{LF}). **D**) Histogramme du nombre de passages à force basse, n_{LF-D}, nécessaire pour obtenir la ligature lorsque la longe mesure ~ 0,7 kbp. **E**) Histogramme du nombre de passages à force basse, n_{LF-D}, nécessaire pour obtenir la ligature lorsque la longe mesure ~ 6 kbp.

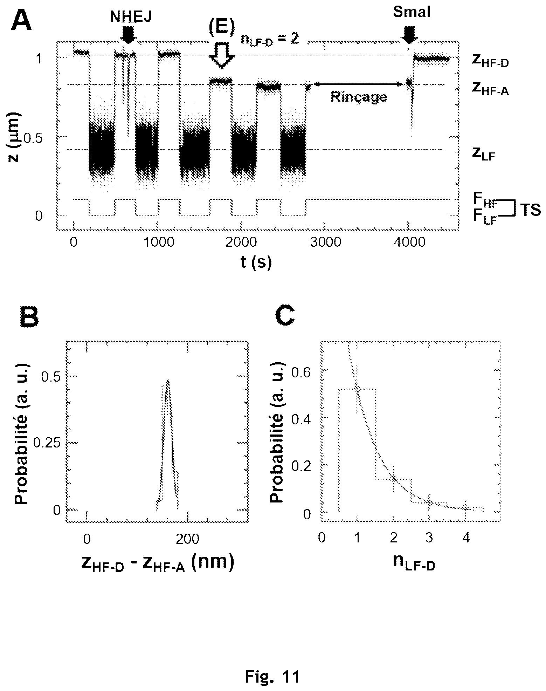

**Figure 11****.** Mesure, par cyclage de la force, du temps caractéristique d'association / ligature de deux bouts francs des extrémités de la molécule d'ADN double-brin en présence des protéines participant au système de réparation NHEJ humain. Ces deux bouts francs, où des groupes phosphates terminaux sont présents en 5' et des groupes hydroxyles terminaux sont présents en 3', constituent eux-mêmes ici les deux molécules à tester. La molécule d'ADN utilisée correspond à celle schématisée dans la **Figure 1** dans un dispositif tel qu'illustré dans la **Figure 2****,** et obtenu par le procédé de fabrication, et obtenu par le procédé de fabrication décrit dans les Exemples 3, 1 puis 9. Les expériences sont réalisées à 34°C dans le tampon RB. **A**) Suivi temporel de l'extension de la molécule d'ADN double-brin lorsque la force est cyclée entre une force haute F_{HF} = 1,4 pN pendant un temps T_{HF} = 300 s et une force basse F_{LF} = 0,05 pN pendant un temps T_{LF} = 300 s (TS : tracé schématique des cycles F_{HF}/F_{LF}). HF correspond à l'application d'une force haute (« *High Force* ») ; LF correspond à l'application d'une force basse (« *Low Force* »). Les molécules à tester peuvent être soit associées (A pour « *Associated* ») ; soit dissociées (D pour « *Dissociated* »). Les molécules à tester sont tout d'abord seules puis les protéines composant le système NHEJ sont introduites dans le capillaire (indiqué par la flèche). Ceci conduit à la formation de liens covalents entre les bouts francs après un certain nombre de passages à basse force, réaction qui se traduit par un raccourcissement de l'extension mesurée à haute force. Ici deux passages à basse force auront été nécessaires pour obtenir la ligature. Finalement, l'ajout de l'enzyme de restriction Smal permet de cliver les liens unissant les bouts francs et de retrouver l'extension maximale à haute force. Le repérage sur la trace temporelle de l'événement de changement d'extension (E) est indiqué par la flèche blanche. **B**) Histogramme des différences d'extension, « *z_{HF-D} - z_{HF-A}* », entre les états HF-A (état de force haute - molécules à tester associées) et HF-D (état de force haute - molécules à tester dissociées), et extraction de la valeur moyenne, *« z_{HF-D}* - *z_{HF-A}* » = 161 nm, par ajustement gaussien. **C**) Histogramme du nombre de passages à force basse, n_{LF-D}, nécessaire pour obtenir l'association / ligature et extraction du temps caractéristique d'association / ligature, τ_{A}, par ajustement exponentiel. Le résultat exprimé en nombre de passages, n_{LF-D}, est égal à 0,8 ± 0,2 ; il peut être converti en temps par multiplication par T_{LF}, ce qui conduit à τ_{A} = 240 ± 60 s.

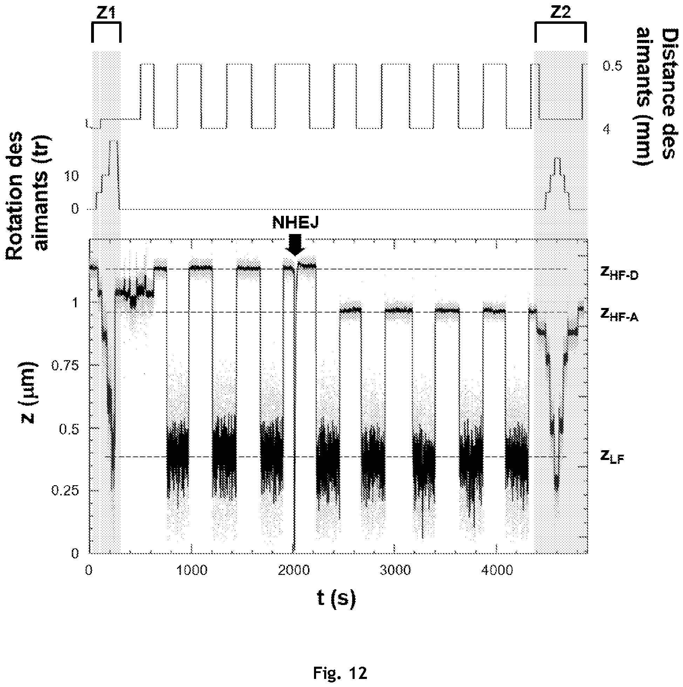

**Figure 12****.** Mise en évidence de la capacité du dispositif à appliquer un couple aux molécules à tester lorsqu'elles sont associées, ceci lors de l'étude des interactions entre deux bouts francs des extrémités de la molécule d'ADN double-brin et les protéines participant au système de réparation NHEJ humain. Ces deux bouts francs, où des groupes phosphates terminaux sont présents en 5' et des groupes hydroxyles terminaux sont présents en 3', constituent eux-mêmes ici les deux molécules à tester. La molécule d'ADN utilisée correspond à celle schématisée dans la **Figure 1** dans un dispositif tel qu'illustré dans la **Figure 2****,** et obtenu par le procédé de fabrication décrit dans les Exemples 3, 1 puis 9. Les expériences sont réalisées à 34°C dans le tampon RB. L'extension de la molécule d'ADN double-brin (bas) est suivie en fonction des modulations de la position des aimants, qui correspond à une modulation de la force (haut), et de l'orientation des aimants (milieu). Un premier essai de surenroulement à basse force (environ 0,4 pN, première zone, Z1) est effectué avant introduction des protéines de réparation et aucune variation de hauteur de la bille n'est observée car les jonctions permettent une libre rotation des différents éléments fonctionnels de la molécule d'ADN (e.g. la longe et les molécules d'ADN double-brin (1) et (2)). Un second essai de surenroulement (seconde zone, Z2) est effectué après introduction des protéines du système NHEJ et réparation aux niveaux des bouts francs, la hauteur de la bille varie maintenant en fonction de l'angle des aimants, signe que les deux extrémités ont été ligaturées et qu'aucune entaille n'existe le long de l'enchaînement de la molécule d'ADN double-brin (1) ou de la molécule d'ADN double-brin (2).

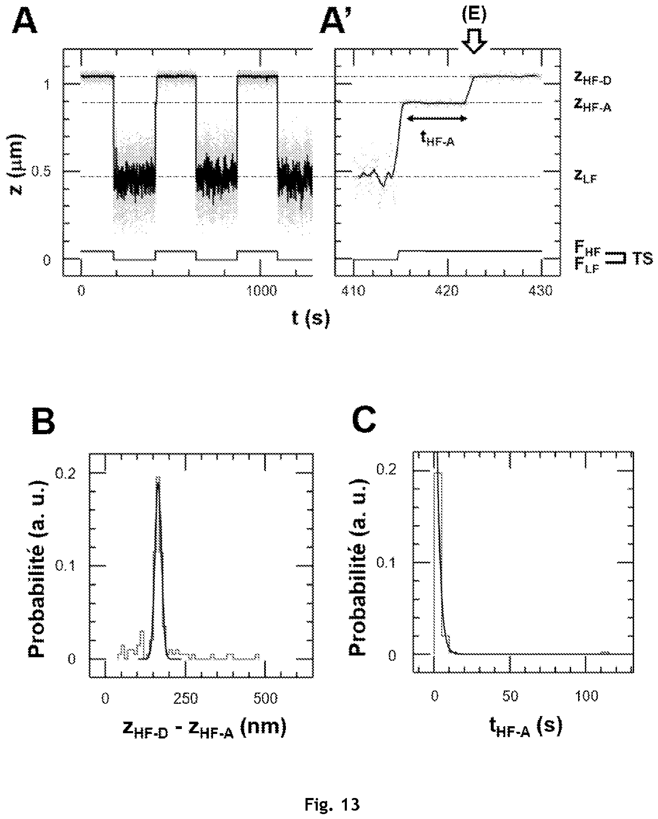

**Figure 13****.** Mesure, par cyclage de la force, du temps caractéristique de dissociation de la liaison non-covalente formée par deux bouts francs des extrémités d'ADN double-brin en présence des protéines participant au système de réparation NHEJ humain. Ces bouts francs, qui ne présentent que des groupes hydroxyles terminaux, constituent eux-mêmes ici les deux molécules à tester. La molécule d'ADN utilisée correspond à celle schématisée dans la **Figure 1** dans un dispositif tel qu'illustré dans la **Figure 2****,** et obtenu par le procédé de fabrication décrit dans les Exemples 3, 1 puis **9.** Les expériences sont réalisées à 34 °C dans le tampon RB. **A**) Suivi temporel de l'extension, z, de la molécule d'ADN lorsque la force F est cyclée entre une valeur haute F_{HF} et une valeur basse F_{LF} (TS : tracé schématique des cycles F_{HF}/F_{LF}). HF correspond à l'application d'une force haute (« *High Force* ») ; LF correspond à l'application d'une force basse (« *Low Force* »).
Les molécules à tester peuvent être soit associées (A pour « *Associated* ») ; soit dissociées (D pour « *Dissociated* »). À chaque passage à F_{LF} = 0,05 pN pendant un temps T_{LF} = 250 s les molécules à tester s'associent puis après passage à F_{HF} = 1,4 pN elles se séparent au bout d'un temps noté t_{HF-A}. La force haute est appliquée pendant T_{HF} = 250 s. **A'**) Zoom sur une mesure de t_{HF-A}. Le repérage sur la trace temporelle de l'événement de changement d'extension (E) est indiqué par la flèche blanche. **B**) Histogramme des différences d'extension, « *z_{HF-D}* - *z_{HF-A}* », entre les états HF-D (état de force haute - molécules à tester dissociées) et HF-D (état de force haute - molécules à tester associées), et extraction de la valeur moyenne, « *z_{HF-D}* - *z_{HF-A}* » = 166 nm, par ajustement gaussien. C) Histogramme des temps de vie des synapses, t_{HF-A}, sous une force élevée F_{HF} = 1,4 pN, et extraction du temps caractéristique de dissociation, τ_{D} = 2,2 ± 0,3 s, par ajustement exponentiel.

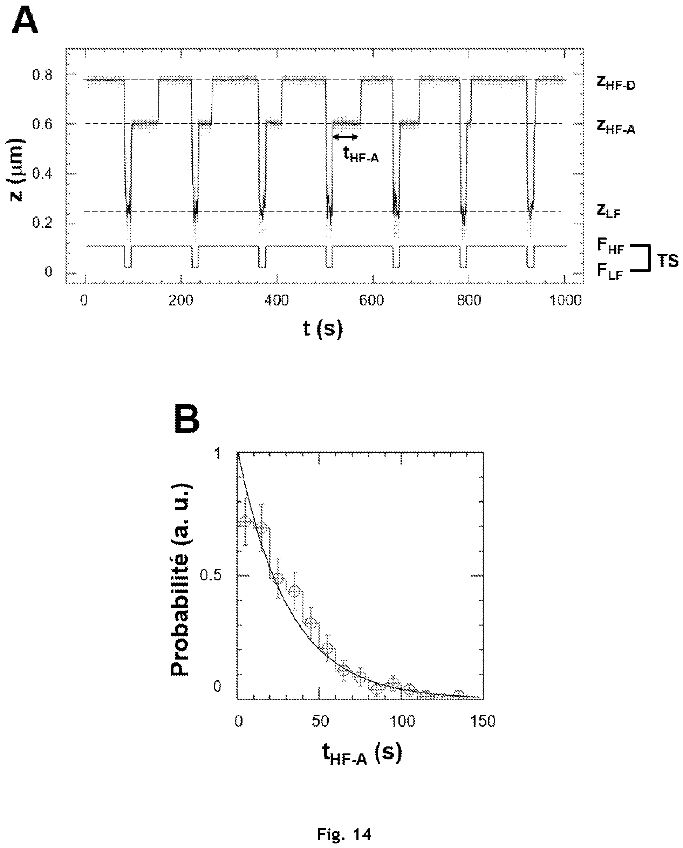

**Figure 14****.** Mesure, par cyclage de la force, du temps caractéristique de dissociation de la liaison non-covalente unissant les protéines FKBP12 et FRB en présence de la rapamycine à 500 nM. Les protéines FKBP12 et FRB constituent ici les deux molécules à tester. La molécule d'ADN utilisée correspond à celle schématisée dans la **Figure 1** dans un dispositif tel qu'illustré dans la **Figure 2****,** et obtenu par le procédé de fabrication illustré dans la **Figure 6** suivi par les procédés illustrés dans les Figures **4** puis **5** (décrits dans les Exemples 3, 1 puis 2). Les expériences sont réalisées à 25 °C dans le tampon DB. **A**) Suivi temporel de l'extension de molécule d'ADN double-brin lorsque la force est cyclée entre valeur haute et valeur basse (TS : tracé schématique des cycles F_{HF}/F_{LF}). HF correspond à l'application d'une force haute (« *High Force* »); LF correspond à l'application d'une force basse (« *Low Force* »). Les molécules à tester peuvent être soit associées (A pour « *Associated* ») ; soit dissociées (D pour « *Dissociated* »). À chaque passage à F_{LF} = 0,05 pN pendant un temps T_{LF} = 13 s les molécules à tester s'associent puis après passage à F_{HF} = 1,4 pN elles se séparent au bout d'un temps noté t_{HF-A}. La force haute est appliquée pendant T_{HF} = 125 s. **B**) Histogramme des t_{HF-A} sous une force haute de 1,4 pN et extraction du temps caractéristique de dissociation, τ_{D} = 31,1 ± 2,3 s, par ajustement exponentiel.

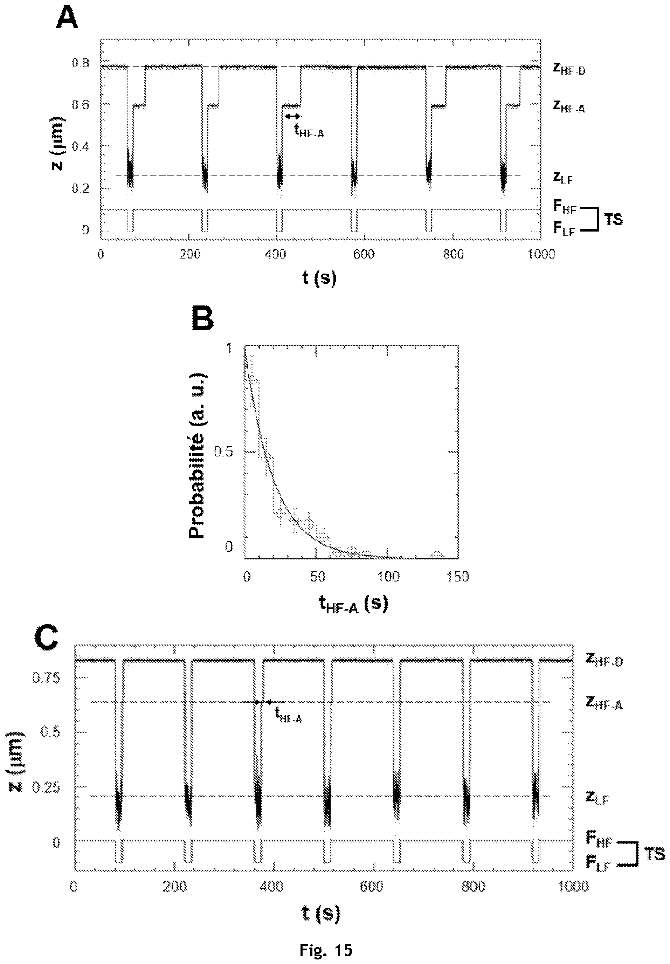

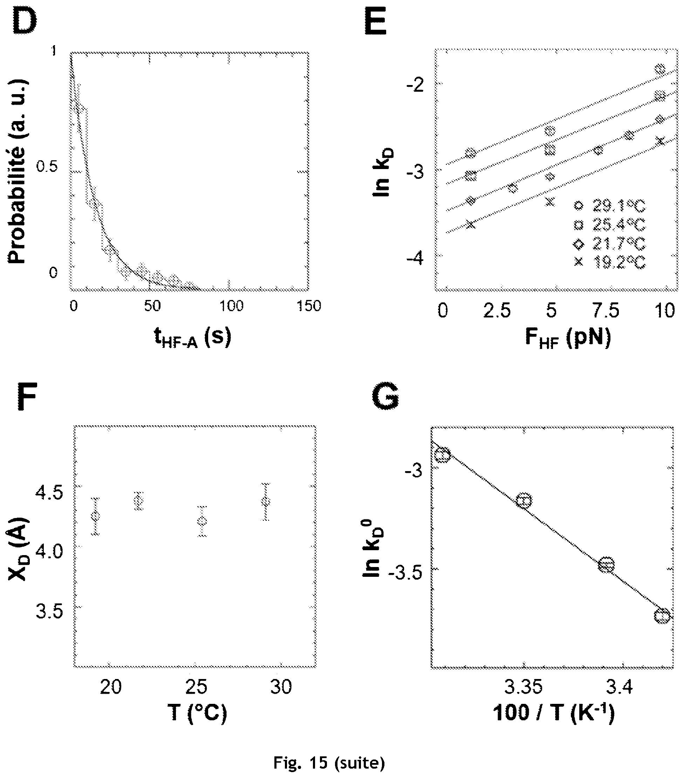

**Figure 15****.** Mesure, par cyclage de la force, du temps caractéristique de dissociation de la liaison non-covalente unissant les protéines FKBP12 et FRB en présence de la rapamycine à 500 nM, ceci pour d'autres valeurs de la force haute, F_{HF}, et/ou de la température que celles utilisées dans la **Figure 12****.** Les protéines FKBP12 et FRB constituent ici les deux molécules à tester. La molécule d'ADN utilisée correspond à celle schématisée dans la **Figure 1** dans un dispositif tel qu'illustré dans la **Figure 2****,** et obtenu par le procédé de fabrication illustré dans la **Figure 6** suivi par les procédés illustrés dans les Figures **4** puis 5 (décrits dans les Exemples 3, 1 puis 2). Les expériences sont réalisées à 30 °C dans le tampon DB. A) Suivi temporel de l'extension de la molécule d'ADN double-brin lorsque la force est cyclée entre valeur haute et valeur basse (TS : tracé schématique des cycles F_{HF}/F_{LF}). HF correspond à l'application d'une force haute (« *High Force* ») ; LF correspond à l'application d'une force basse (« *Low Force* »). Les molécules à tester peuvent être soit associées (A pour « *Associated* ») ; soit dissociées (D pour « *Dissociated* »). À chaque passage à F_{LF} = 0,05 pN pendant un temps T_{LF} = 13 s les molécules à tester s'associent puis après passage à F_{HF} = 1,4 pN elles se séparent au bout d'un temps noté t_{HF-A}. La force haute est appliquée pendant un temps T_{HF} = 155 s. **B**) Histogramme des t_{HF-A} sous une force haute de 1,4 pN et extraction du temps caractéristique de dissociation τ_{D} = 20,6 ± 2,6 s, par ajustement exponentiel. **C**) Suivi temporel de l'extension de la molécule d'ADN double-brin lorsque la force est cyclée entre valeur haute et valeur basse (TS : tracé schématique des cycles F_{HF}/F_{LF}). À chaque passage à F_{LF} = 0,05 pN pendant un temps T_{LF} = 14 s les molécules à tester s'associent puis après passage à F_{HF} = 6 pN elles se séparent au bout d'un temps noté t_{HF-A}. La force haute est appliquée pendant un temps T_{HF} = 125 s. **D**) Histogramme des t_{HF-A} sous une force haute de 6 pN et extraction du temps caractéristique de dissociation, τ_{D} = 15,4 ± 1,9 s, par ajustement exponentiel. **E**) Variation du logarithme des constante de vitesse de dissociation, calculé comme ln[k_{D}] = ln[1/<τ_{D}>], en fonction de F_{HF}, la force haute appliquée (<τ_{D}> indique que les temps caractéristiques ont été moyenné sur plusieurs molécules d'ADN). Les courbes sont données pour plusieurs température : OT = 29,1 °C ; □T = 25,4 °C ; ◇ T = 21,7 °C ; x T = 19,2 °C. Les ajustements linéaires en accord avec le modèle d'Arrhenius-Bell, ln[k_{D}] = ln[k_{D}⁰] + X_{D}F_{HF}/K_{B}T, permettent de déterminer ln[k_{D}⁰] et X_{D}. **F)** Variation de X_{D}, la distance séparant l'état de transition du complexe lors de la dissociation, en fonction de la température. La moyenne des valeurs est égale à 4,31 ± 0,03 Å. **G)** Variation de ln[k_{D}⁰], le logarithme de la vitesse de dissociation extrapolée à force nulle, en fonction de la température. Un ajustement linéaire en accord avec le modèle d'Arrhenius-Bell, ln[k_{D}⁰] = A - E_{D}/k_{B}T, permet de déterminer E_{D}, l'énergie d'activation de la réaction de dissociation. On trouve E_{D} = 58,8 ± 1,7 kJ mol⁻¹.

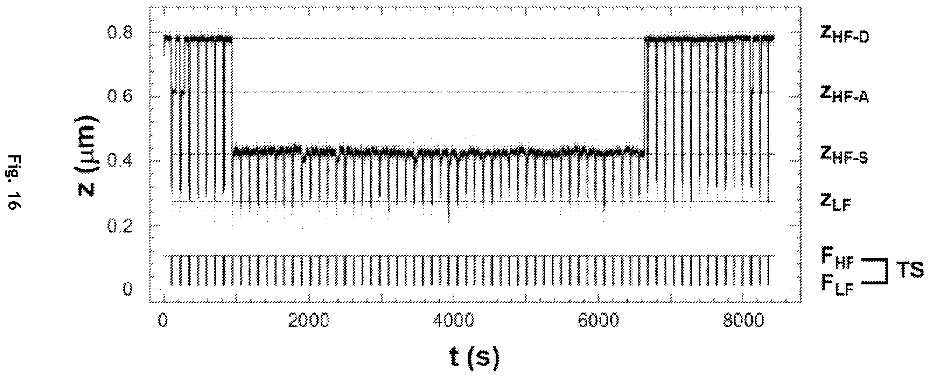

**Figure 16****.** Mise en évidence de la capacité du dispositif à distinguer les interactions spécifiques entre une des molécules à tester et l'un des supports (z_{HF-S}, où « S » signifie support), en plus des interactions entre les molécules à tester (z_{HF-D}, z_{HF-A}), ceci lors de l'étude des interactions entre les protéines FKBP12 et FRB en présence de la rapamycine.

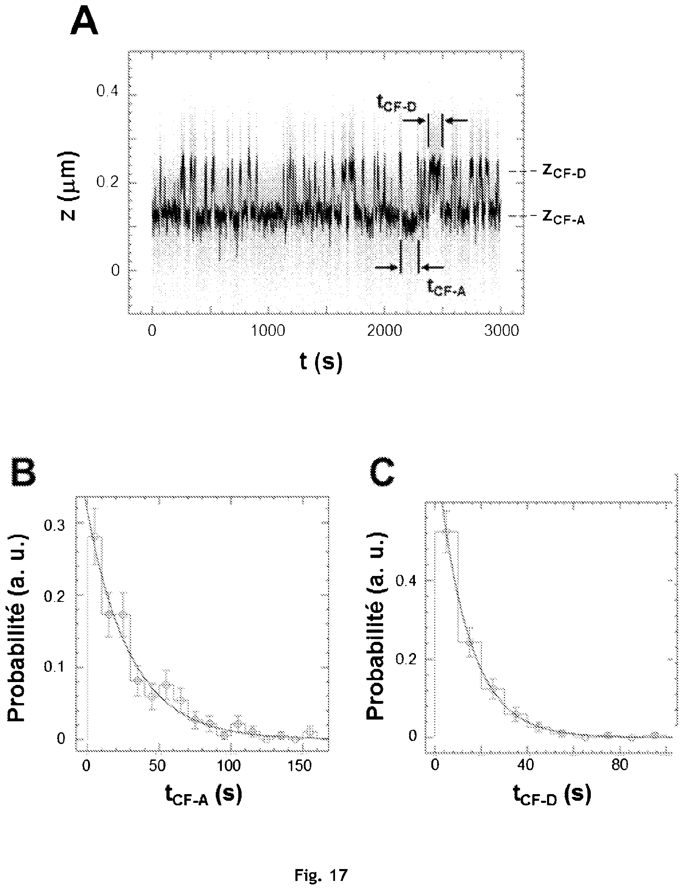

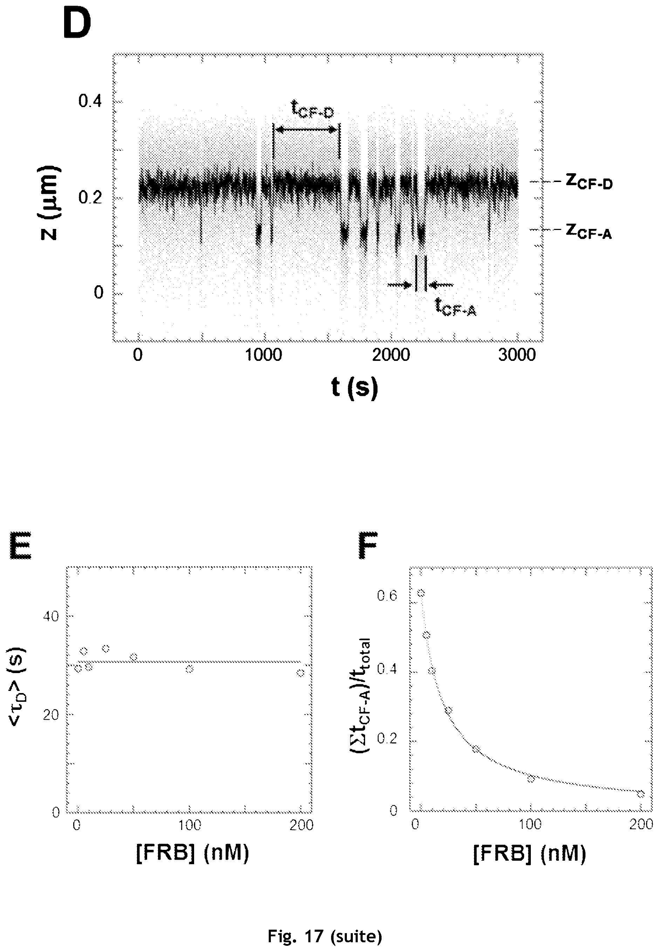

**Figure 17****.** Mesure, par étude des fluctuations spontanées, du temps caractéristique de dissociation et du temps caractéristique d'association de la liaison non-covalente unissant les protéines FKBP12 et FRB en présence de la rapamycine à 500 nM. Les protéines FKBP12 et FRB constituent ici les deux molécules à tester. La molécule d'ADN utilisée correspond à celle schématisée dans la **Figure 1** dans un dispositif tel qu'illustré dans la **Figure 2****,** et obtenu par le procédé de fabrication illustré dans la **Figure 6** suivi par les procédés illustrés dans les Figures **4** puis **5** (décrits dans les Exemples 3, 1 puis 2). Les expériences sont réalisées à 25 °C dans le tampon DB. **A**) Suivi temporel de l'extension de molécule d'ADN double-brin lorsque la force est maintenue à une valeur constante (F_{CF} = 0,04 pN). CF correspond à l'application d'une force constante (« *Constant Force* »). Les molécules à tester peuvent être soit associées (A pour « *Associated* ») ; soit dissociées (**D** pour « *Dissociated* »). La dissociation des molécules à tester se produit au bout d'un temps noté t_{CF-A} et l'association au bout d'un temps noté t_{CF-D}. **B**) Histogramme des t_{CF-A} sous une force constante de 0,04 pN et extraction du temps caractéristique de dissociation, τ_{D} = 30,4 ± 2,7 s, par ajustement exponentiel. **C**) Histogramme des t_{CF-D} sous une force constante de 0,04 pN et extraction du temps caractéristique d'association, τ_{A} = 13,7 ± 1,1 s, par ajustement exponentiel. **D**) Suivi temporel de l'extension de molécule d'ADN double-brin lorsque la force est maintenue à une valeur constante (TS : tracé schématique F_{CF}) et que des molécules identiques à l'une des molécules à tester sont présentes en solution. CF correspond à l'application d'une force constante (« *Constant Force* »). Les molécules à tester peuvent être soit associées (A pour « *Associated* ») ; soit dissociées (D pour « *Dissociated* »), dans ce dernier cas l'une des molécules à tester peut être soit libre, soit complexée par une des molécules en solution. F_{CF} = 0,04 pN en permanence et [FRB] = 100 nM. Les molécules à tester s'associent et se dissocient spontanément, l'une des deux peut aussi réagir spontanément avec les molécules en présentent en solution. La dissociation des molécules à tester se produit au bout d'un temps noté t_{CF-A} et l'association au bout d'un temps noté T_{CF-D}. **E**) Variation du temps caractéristique dissociation moyenné sur plusieurs molécules, <τ_{D}>, en fonction de la concentration en [FRB], obtenu sous une force constante de 0,04 pN. La ligne continue horizontale correspond à la valeur moyenne obtenue sur toutes les concentrations, on obtient 30,6 s. **F)** Variation de la fraction de temps passée par les molécules à tester sous forme associée, (Σt_{CF-A})/tₜₒₜₐₗ, en fonction de la concentration en [FRB], obtenu sous une force constante de 0,04 pN. Les données sont ensuite ajustées à l'aide de (Σt_{CF-A})/tₜₒₜₐₗ = ((1+K₀) + [FRB]/C_{eff} )⁻¹, ce qui conduit à C_{eff} = 12,3 nM et K₀ = 0,59.

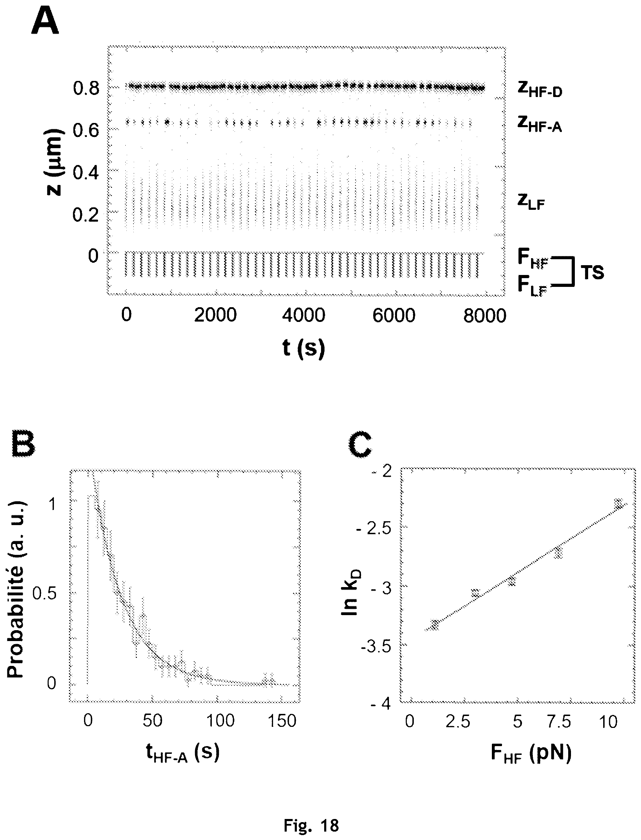

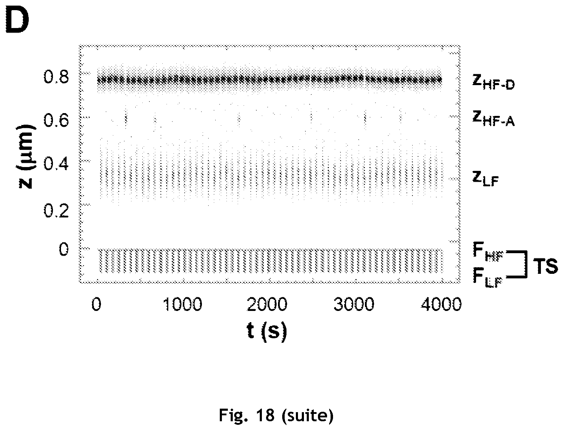

**Figure 18****.** Mesure, par cyclage de la force, du temps caractéristique de dissociation de la liaison non-covalente de deux molécules à tester attachées à la molécule d'ADN grâce au système d'étiquetage par SNAP et CLIP. La molécule d'ADN utilisée correspond à celle schématisée dans la **Figure 7B** dans un dispositif tel qu'illustré dans la **Figure 2****,** et obtenu par le procédé de fabrication illustré dans la **Figure 7A** suivi par les procédés illustrés dans les Figures 6B-D, 4, puis 7B (décrits dans les Exemples 3 et 1 avec les variations décrites dans l'Exemple 4). **A-C).** Les molécules à tester sont les protéines FKBP12 et FRB qui interagissent en présence de la rapamycine. Les expériences sont réalisées à 21,7 °C dans le tampon DB. **A**) Suivi temporel de l'extension de la molécule d'ADN double-brin lorsque la force est cyclée entre valeur haute et valeur basse (TS : tracé schématique des cycles F_{HF}/F_{LF}). HF correspond à l'application d'une force haute (« *High Force* ») ; LF correspond à l'application d'une force basse (« *Low Force* »). Les molécules à tester peuvent être soit associées (A pour « *Associated* ») ; soit dissociées (D pour « *Dissociated* »). À chaque passage à F_{LF} = 0,01 pN pendant un temps de 13 s les molécules à tester s'associent puis après passage à F_{HF} = 1,1 pN elles se séparent au bout d'un temps noté t_{HF-A} (voir **B**)). La force haute est appliquée pendant un temps de 155 s. **B**) Histogramme des t_{HF-A} sous une force haute de 1,1 pN et extraction du temps caractéristique de dissociation τ_{D} = 25,3 ± 2,4 s, par ajustement exponentiel. C) Variation du logarithme des constantes de vitesse de dissociation, calculé comme ln[k_{D}] = ln[1/<τ_{D}>], en fonction de F_{HF}, la force haute appliquée (<τ_{D}> indique que les temps caractéristiques ont été moyenné sur plusieurs molécules d'ADN). L'ajustement linéaire est en accord avec le modèle d'Arrhenius-Bell, ln[k_{D}] = ln[k_{D}⁰] + X_{D}F_{HF}/k_{B}T, permettant de déterminer ln[k_{D}⁰] = -3.45 ± 0.03 et X_{D} = 4.7 ± 0.2 Å. D) Les molécules à tester sont la géphyrine et la boucle β du récepteur de la glycine. Les expériences sont réalisées à 19,2 °C dans le tampon GB. Suivi temporel de l'extension de la molécule d'ADN double-brin lorsque la force est cyclée entre valeur basse F_{LF} = 0,01 pN pendant 13 s et une valeur haute F_{HF} = 1,1 pN pendant 52 s.

### DESCRIPTION DETAILLEE DE L'INVENTION

Comme indiqué précédemment, dans le cadre de la présente invention, les inventeurs ont mis en évidence de nouvelles molécules d'ADN double-brin comprenant une première molécule d'ADN double-brin (1) reliée à une deuxième molécule d'ADN double-brin (2) par au moins une liaison covalente qui n'est pas une liaison phosphodiester, une liaison phosphorothioate, une liaison phosphoramidate ou une liaison phosphorodiamidate, avantageusement par une longe. Ces nouvelles molécules double-brin sont très avantageuses car elles présentent une stabilité, une flexibilité et une homogénéité améliorées, grâce notamment à l'utilisation de molécules d'ADN double-brin et à leur configuration.

### MOLECULE D'ADN

Un premier aspect de l'invention concerne donc une molécule d'ADN double-brin comprenant une première molécule d'ADN double-brin (1) reliée, par au moins une liaison covalente qui n'est pas une liaison phosphodiester, une liaison phosphorothioate, une liaison phosphoramidate ou une liaison phosphorodiamidate, à une deuxième molécule d'ADN double-brin (2).

Par « *comprenant* » ou « *contenant* », on entend ici que les éléments énumérés sont requis ou obligatoires, mais que d'autres éléments optionnels peuvent être ou non présents. Ainsi, à titre d'exemple non-limitatif, une molécule d'ADN double-brin selon l'invention peut notamment comprendre d'autres molécules, dont d'autres molécules d'ADN double-brin, en surplus des molécules (1) et (2).

Les termes « *un*/*une* » et « *le*/*la »* tels qu'utilisés ici comprennent des formes plurielles sauf si le contenu de la présente demande indique clairement le contraire. À titre d'exemple, « *un site de restriction* » peut donc comprendre deux sites de restriction ou plus.

Par « *acide nucléique* », « *molécule d'acide désoxyribonucléique* » ou « *molécule d'ADN* », on entend, au sens de la présente invention, un polymère composé de monomères de désoxyribonucléotides ou des analogues de ceux-ci. Plus particulièrement, les monomères désoxyribonucléotidiques décrits ici se réfèrent à des monomères comprenant un groupe triphosphate, la base azotée adénine (« *A* »), cytosine (« *C* »), guanine (« *G* »), ou thymine (« *T* »), et un sucre de désoxyribose. De façon générale, un analogue d'un nucléotide aura la même spécificité d'appariement de bases (e.g. un analogue de « *A* » s'apparie avec « *T* »). Des nucléotides modifiés sont également inclus ici, et peuvent comprendre, par exemple, des modifications dans les moitiés base, sucres, et/ou phosphate (i.e. des squelettes phosphorothioates). Ladite molécule d'ADN peut également comprendre un ou plusieurs nucléotides modifiés, tels qu'un acide nucléique bloqué (LNA), qui est un nucléotide dans lequel la fraction ribose est modifiée par un pont supplémentaire reliant l'oxygène 2' et le carbone 4', un acide nucléique peptidique (PNA), le squelette étant composé de motifs répétés de N-(2-aminoéthyl)-glycine liés par des liaisons peptidiques, des nucléotides modifiés tels que l'hypoxanthine, la xanthine, la 7-méthylguanine, ou la 5-méthylcytosine, ou des analogues de type « morpholino ».

La molécule d'ADN double-brin selon l'invention est un polymère composé majoritairement de désoxyribonucléotides (i.e. plus de 50 %). Avantageusement, la molécule d'ADN double-brin selon l'invention est un polymère composé d'au moins 50% de désoxyribonucléotides, plus avantageusement d'au moins 60%, d'au moins 70%, d'au moins 75%, d'au moins 80%, d'au moins 85%, encore plus avantageusement d'au moins 90%, d'au moins 95%, d'au moins 96%, d'au moins 97%, d'au moins 98%, ou d'au moins 99% de désoxyribonucléotides. Avantageusement, la molécule d'ADN double-brin selon l'invention est un polymère composé entièrement (i.e. 100%) de désoxyribonucléotides. Ainsi, selon un mode préférentiel, la première molécule d'ADN double-brin (1) et/ou la deuxième molécule d'ADN double-brin (2), compris dans la molécule d'ADN double-brin selon l'invention, sont avantageusement des polymères composés d'au moins 50%, d'au moins 60%, d'au moins 70%, d'au moins 75%, d'au moins 80%, d'au moins 85%, d'au moins 90%, d'au moins 95%, d'au moins 96%, d'au moins 97%, d'au moins 98%, ou d'au moins 99%, plus avantageusement composés entièrement de désoxyribonucléotides. La première molécule d'ADN double-brin (1) et la deuxième molécule d'ADN double-brin (2) peuvent être des polymères composés de différentes proportions de désoxyribonucléotides, par exemple selon les pourcentages décrits ci-dessus. Lorsque d'autres molécules d'ADN double-brin sont présentes dans la composition (e.g. une molécule d'ADN (3)), elles sont aussi avantageusement des polymères composés d'au moins 50% de désoxyribonucléotides voir plus, selon les pourcentages décrits ci-dessus, et ont les mêmes caractéristiques que les molécules d'ADN double-brin (1) et/ou (2) décrites ici. Le plus souvent, l'acide nucléique double-brin sera une molécule d'ADN, mais il est compris que l'invention s'applique également à des duplex de deux molécules d'ADN simple-brin, parfaitement appariées ou non parfaitement appariées. En outre, le duplex peut consister en l'appariement au moins partiel de deux brins uniques obtenus à partir d'échantillons d'origines différentes. Enfin, l'invention s'applique également aux structures secondaires d'un seul ADN simple-brin, formant ainsi des structures en forme double-brin.

La molécule d'ADN double-brin selon l'invention peut être d'origine naturelle (e.g. d'origine eucaryote ou procaryote), ou artificielle, ou comprendre un mélange d'ADN d'origine naturelle et artificielle selon n'importe quel ratio. La première molécule d'ADN double-brin (1) et la deuxième molécule d'ADN double-brin (2) peuvent donc être composées d'ADN de la même origine ou d'origine différente. Le plus souvent, l'origine de l'ADN dépendra des besoins expérimentaux (e.g. besoin d'une molécule d'ADN ayant une longueur particulière, possédant certain(s) site(s) de restriction, etc.). De même, les séquences des première et deuxième molécules d'ADN double-brin (1) et (2) peuvent être identiques ou différentes. De façon avantageuse, la première molécule d'ADN double-brin (1) et de la deuxième molécule d'ADN double-brin (2) ont des séquences différentes.

Les longueurs des première et deuxième molécules d'ADN double-brin (1) et (2) peuvent être choisies selon les besoins expérimentaux, tels que détaillés ci-dessous, et/ou en fonction des autres éléments de la molécule d'ADN (e.g. pour que les deux molécules d'ADN double-brin (1) et (2) aient chacune une longueur particulière ou selon la longueur d'une longe). Les longueurs de la première molécule d'ADN double-brin (1) et de la deuxième molécule d'ADN double-brin (2) peuvent être identiques ou différentes. La longueur de la première molécule d'ADN double-brin (1) peut, par exemple, être plus courte que celle de la deuxième molécule d'ADN double-brin (2), ou plus longue. Une différence dans la longueur de la première molécule d'ADN double-brin (1) par rapport à la deuxième molécule d'ADN double-brin (2) est particulièrement avantageuse lorsque la molécule d'ADN est utilisée pour détecter et/ou mesurer des interactions moléculaires. En effet, les inventeurs ont démontré de façon surprenante que, lorsque la molécule d'ADN est utilisée pour détecter et/ou mesurer des interactions moléculaires, une différence de longueur de la première molécule d'ADN double-brin (1) par rapport à la deuxième molécule d'ADN double-brin (2) permet de mieux différencier les signaux qui résultent d'une interaction spécifique entre deux molécules à tester aux signaux qui résultent d'une interaction non-spécifique (i.e. entre un support et une molécule à tester (voir par exemple la **Figure 16** et l'Exemple 11).

Alors que la « *longueur* » d'une molécule d'ADN est généralement exprimée en nombre de nucléotides ou paires de bases (pb), elle peut également être exprimée selon sa longueur physique, par exemple en nanomètres ou micromètres (1 paire de base correspond à environ 0,32 nm). De façon préférée, la longueur d'une molécule d'ADN est exprimée ici en paires de bases. Par exemple, la première molécule d'ADN double-brin (1) et/ou la deuxième molécule d'ADN double-brin (2) peut avoir une longueur de 300 à 5 000 paires de bases, de 500 à 5 000 paires de bases, ou de 650 à 1 500 paires de bases. Ainsi, la longueur totale des molécules d'ADN double-brin (1) et (2) peut être comprise entre 600 et 10 000 paires de bases, entre 1 000 et 10 000, ou entre environ 1 300 et 3 000 paires de bases (± 50 paires de bases).

Selon un mode préféré de l'invention, la première molécule d'ADN double-brin (1) et/ou la deuxième molécule d'ADN (2) a une longueur de 300 à 5 000 paires de bases, de préférence de 650 à 1 500 paires de bases. Selon un mode de réalisation particulier, la première molécule d'ADN double-brin (1) et la deuxième molécule d'ADN double-brin (2) ont chacune une longueur de 1500 paires de bases. Selon un autre mode de réalisation particulier, la première molécule d'ADN double-brin (1) a une longueur de 650 paires de bases alors que la deuxième molécule d'ADN (2) a une longueur de 1 350 paires de bases.

Avantageusement, la longueur totale des molécules d'ADN double-brin (1) et (2) est comprise entre 600 et 10 000 paires de bases, entre 1 000 et 10 000, ou entre 1300 et 3000 paires de bases. Selon un mode particulier de l'invention, la longueur totale des molécules d'ADN double-brin (1) et (2) est comprise entre 2 050 paires de bases et 3 000 paires de bases, avantageusement d'environ 2 050 ou d'environ 3 000 paires de bases (± 50 paires de bases).

Dans certains cas, l'ADN est sélectionné pour ne pas comprendre d'éléments qui pourraient interagir avec une ou plusieurs des molécules à tester. À titre d'exemple, lorsqu'une molécule à tester est une protéine susceptible d'interagir avec une molécule d'ADN double-brin ayant une séquence et/ou une structure particulière (e.g. MutS, Zα), la molécule d'ADN double-brin selon l'invention ne comprend pas ces séquences et/ou structures (e.g. absence de mismatch, pas de structure de type Z-ADN) sauf sur le site conçu pour accueillir la deuxième molécule à tester (e.g. séquence avec un mismatch, Z-ADN).

La molécule d'ADN double-brin peut notamment comprendre une partie (e.g. 0,6, 1, 5, 20, 30, 40, 50, 60, 70, 80 ou 90 %) ou la totalité (100%) d'un génome d'ADN double-brin d'un virus de groupe I, tel qu'un génome d'un virus de l'ordre *Caudovirales, Herpesvirales*, ou *Ligamenvirales,* plus particulièrement de la famille *Siphoviridae.* À titre d'exemple non-limitatif, la molécule d'ADN double-brin comprend une partie ou la totalité d'un génome d'ADN double-brin d'un bactériophage, tel que le phage lambda, le phage P1 de *Mycoplasma,* le phage c2 de *Lactococcus* phage c2, le phage F108 de *Pasteurella*, ou tout autre génome d'ADN double-brin de phage. À titre d'exemple non-limitatif, la longueur du génome incorporé dans la molécule d'ADN double-brin selon l'invention peut être comprise environ 11 500 et environ 50 000 paires de bases (± 50 paires de bases). Lorsque la molécule d'ADN double-brin selon l'invention comprend un génome entier, ledit génome est avantageusement sous forme linéaire. Lorsqu'une molécule d'ADN double-brin est sous forme circulaire (e.g. un génome circulaire, un plasmide), elle est avantageusement linéarisée avant d'être incorporée dans la molécule d'ADN double-brin selon l'invention.

La molécule d'ADN double-brin selon l'invention peut être continue ou discontinue. Une molécule d'ADN est discontinue lorsqu'au moins une liaison phosphodiester, phosphorothioate, phosphoramidate ou phosphorodiamidate, liant deux nucléotides localisés l'un à côté de l'autre sur un seul brin est absente, ou lorsque la molécule d'ADN comprend une région d'ADN simple-brin. Dans certains cas, une molécule d'ADN peut avoir un ou plusieurs sites de discontinuité sur les deux brins, à condition que les sites de discontinuité ne soient pas présents entre deux nucléotides appariés sur les deux brins. Cependant, dans la cadre de la présente invention, l'ADN double-brin de la première molécule d'ADN double-brin (1) et/ou de la deuxième molécule d'ADN double-brin (2) est avantageusement continu. Selon un mode de réalisation préféré, la molécule d'ADN double-brin selon l'invention comprenant une première molécule d'ADN double-brin (1) reliée à une deuxième molécule d'ADN double-brin (2) par une liaison covalente qui n'est pas une liaison phosphodiester, phosphorothioate, phosphoramidate ou phosphorodiamidate est continue. En effet, la continuité de la molécule permet avantageusement de garantir les propriétés mécaniques de la molécule d'ADN double-brin, dont sa longueur de persistance et sa capacité à être superenroulée (« *supercoiled* »).

Avantageusement, tout site de discontinuité dans la molécule d'ADN double-brin selon l'invention est fermé, par exemple par une ligase lors de la fabrication de ladite molécule d'ADN double-brin, avant son utilisation. Avantageusement, la molécule d'ADN double-brin selon l'invention est parfaitement appariée. La molécule d'ADN double-brin selon l'invention comprend plus particulièrement au moins deux molécules d'ADN double-brin distinctes, appelés ici les molécules d'ADN double-brin (1) et (2), qui sont reliées l'une à l'autre par au moins une liaison covalente.

Par « *liaison covalente* » on entend ici une liaison chimique dans laquelle au moins une paire d'électrons est partagée entre deux atomes. De façon avantageuse, selon la présente invention, au moins une liaison covalente est formée entre un atome d'un premier groupe fonctionnel et un atome d'un deuxième groupe fonctionnel. À titre d'exemple non-limitatif, une liaison covalente peut être une ou plusieurs liaison(s) amide ou ester, ou un pont disulfure. Le terme « *la liaison covalente* » inclut donc plusieurs types de liaisons covalentes. À titre d'exemple non-limitatif, ladite liaison covalente peut également comprendre d'autres éléments, tels qu'un ou plusieurs polymères, composés cycliques, ou particules, pouvant lier les deux sous-unités en formant une liaison covalente avec chaque sous-unité.

Selon un mode de réalisation préféré, ladite au moins une liaison covalente n'est pas une liaison phosphodiester. Une liaison phosphodiester est une liaison entre le carbone 3' d'un premier désoxyribose et le carbone 5' d'un deuxième désoxyribose par la formation d'une liaison phosphoester de chacun des carbones avec un groupe phosphate. Au sens de la présente invention, une liaison phosphodiester est plus particulièrement une liaison entre deux désoxyribonucléotides, tel que connu par l'homme de métier. Selon un autre mode de réalisation préféré, ladite au moins une liaison covalente n'est pas une liaison phosphorothioate. Une telle liaison est une liaison phosphodiester où un soufre vient substituer un oxygène non liant dans le groupe phosphate. Selon un autre mode de réalisation préféré, ladite au moins une liaison covalente n'est pas une liaison phosphoramidate ou une liaison phosphorodiamidate. Une liaison phosphoramidate est une liaison phosphodiester dans laquelle le groupe phosphate comprend un phosphore lié à trois atomes d'oxygène et un atome d'azote. Une liaison phosphorodiamidate est une liaison phosphodiester dans laquelle le groupe phosphate comprend un phosphore lié à deux atomes d'oxygène et deux atomes d'azote. Les liaisons phosphorothioates, phosphoramidates et phosphorodiamidates se retrouvent principalement dans des acides nucléiques synthétiques (tels que ceux décrits plus haut), auxquels elles confèrent des propriétés avantageuses. A titre d'exemple, ces liaisons confèrent généralement une stabilité accrue aux acides nucléiques dans lesquelles elles se retrouvent. À titre d'exemple non-limitatif, une liaison covalente autre qu'une liaison phosphodiester, une liaison phosphorothioate, une liaison phosphoramidate ou une liaison phosphorodiamidate, peut être une liaison formée sur la base de la chimie click, telle qu'une liaison formée par la cycloaddition d'un azoture à une alcyne (voir, par exemple, Hein et al., 2008). Avantageusement, ladite liaison covalente reliant les deux molécules d'ADN double-brin (1) et (2) est formée par une réaction entre une fonction azoture et une fonction alcyne, entre une fonction azoture et une fonction dibenzocyclooctyne (DBCO), entre une fonction tétrazine et une fonction transcyclooctène, entre une fonction thiol et une fonction alcyne, entre une fonction thiol et une fonction alcène, entre une fonction thiol et une fonction thiol (générant ainsi un pont disulfure), entre une fonction thiol et une fonction maléimide, entre une fonction amine et un acide activé (e.g. par des réactifs tels que le N-hydroxysuccinimide, le *N*,*N*'-dicyclohexylcarbodiimide ou le 1-éthyl-3-(3-diméthylaminopropyl)carbodiimide), entre une fonction hydroxyle et une fonction maléimide, et/ou entre une fonction azoture et une fonction phosphine (réaction de Staudinger).

Dans certains cas, ladite première molécule d'ADN double-brin (1) est liée à ladite deuxième molécule d'ADN double-brin (2) par une longe. Par « *longe* » on entend au sens de la présente invention une troisième molécule distincte, qui est liée à la fois à la première (1) et à la deuxième (2) molécule d'ADN double-brin. De façon avantageuse, lorsque la molécule d'ADN double-brin selon l'invention est utilisée pour caractériser des interactions moléculaires entre au moins deux molécules à tester, la longe permet de garder les deux molécules à tester à proximité l'une de l'autre après leur dissociation.

Lorsque les deux molécules d'ADN double-brin (1) et (2) sont liées par une longe, ladite longe peut être liée à chaque molécule d'ADN par au moins une liaison covalente qui n'est pas une liaison phosphodiester, phosphorothioate, phosphoramidate ou phosphorodiamidate. Ainsi, la molécule d'ADN double-brin selon l'invention comprend avantageusement au moins deux liaisons covalentes qui ne sont pas des liaisons phosphodiesters, phosphorothioates, phosphoramidates ou phosphorodiamidates (voir, par exemple, la **Figure 1**). De façon alternative, ladite longe peut être composée de plusieurs molécules distinctes, qui sont elles-mêmes avantageusement reliées les unes aux autres par des liaisons covalentes qui ne sont pas des liaisons phosphodiesters, phosphorothioates, phosphoramidates ou phosphorodiamidates. Selon un mode de réalisation préféré, ladite première molécule d'ADN double-brin (1) est donc liée à ladite deuxième molécule d'ADN double-brin (2) par une longe.

La longe peut être composée de tout type de matière et/ou comprendre tout type de molécule, tels que des polypeptides, des polynucléotides, tels que l'ADN double-brin, et/ou des polymères. À titre d'exemple non-limitatif, la longe est un polymère composé d'au moins 50%, 60%, 70% de désoxyribonucléotides, avantageusement au moins 75%, 80%, 85%, plus avantageusement au moins 90%, 95%, 96%, 97%, 98%, encore plus avantageusement au moins 99% de désoxyribonucléotides. À titre d'exemple non-limitatif, la longe est un polymère composé de désoxyribonucléotides et un ou plusieurs autres polymères tels que le polyéthylène glycol (PEG), le propylène glycol, un polyamide, et/ou une ou plusieurs régions d'acide nucléique simple brin (e.g. d'ADN simple-brin). Avantageusement, la longe est un polymère composé entièrement (i.e. 100%) de désoxyribonucléotides. Lorsque la longe comprend de l'ADN double-brin, ledit ADN double-brin peut comprendre n'importe quelle caractéristique telle que détaillée ci-dessus. À titre d'exemple, la longe d'ADN double-brin est continue. De préférence, lorsque la longe comprend de l'ADN double-brin, la longueur de ladite longe est d'au moins 150 paires de bases, plus préférentiellement d'au moins 200, 300, 400, 500 ou 600 paires de bases. Avantageusement, une telle longueur en ADN double-brin permet aux molécules à tester de se rencontrer avec une efficacité significative et limite les contraintes mécaniques s'appliquant au complexe une fois celui formé. Selon un mode de réalisation préférentiel, la longe comprend de l'ADN double-brin ayant une longueur d'au moins 150 paires de bases, de préférence d'au moins 600 paires de bases, ledit ADN double-brin étant continue (i.e. sans entaille). Lorsque la longe est une molécule d'acide nucléique double-brin (e.g. une molécule d'ADN ou d'ARN double-brin), elle comprend elle-même des liaisons phosphodiesters, phosphorothioates, phosphoramidates ou phosphorodiamidates. Toutefois, comme indiqué ci-dessus, l'au moins une liaison covalente entre la longe et ladite première ou deuxième molécule d'ADN double-brin (1) ou (2) n'est pas une liaison phosphodiester, phosphorothioate, phosphoramidate ou phosphorodiamidate. Dans certains cas, ladite longe peut elle-même comprendre des liaisons covalentes (e.g. du « *crosslinking* » entre les deux brins d'une molécule ADN double-brin, lorsque la longe est un polymère d'ADN double-brin). Ceci est notamment avantageux lorsque des forces supérieures à 70 pN seront appliquées à la molécule d'ADN double-brin.

Selon un mode de réalisation préféré, la longe est une molécule d'ADN double-brin. De façon avantageuse, la structure double hélice d'une longe d'ADN double-brin est uniquement présente sous forme linéaire. La longe ne peut donc se replier en aucune structure tertiaire. De plus, lorsque la longe est une molécule d'ADN double-brin, elle est stable et est donc peu encline à interagir avec l'une ou plusieurs des molécules à tester. La longueur de la longe est également avantageusement facilement ajustable.

L'utilisation d'une longe d'ADN double-brin est aussi avantageuse car des molécules d'ADN double-brin ont une longueur de persistance d'environ 50 nm dans des conditions de laboratoire usuelles. Par « *longueur de persistance* » on entend une propriété mécanique permettant de caractériser la rigidité d'un polymère linéaire (Doi, 1996, et Bouchiat et al., 1999). Il s'agit plus particulièrement de la longueur typique sur laquelle un polymère linéaire peut rester aligné malgré les déformations dues à l'agitation thermique. Avec une telle longueur de persistance, une variation de longueur de la longe se traduit en une variation conséquente de la distance moyenne entre les jonctions avec les molécules d'ADN double-brin (1) et (2), ce qui permet d'ajuster la concentration effective en molécules à tester, *C_{eff}.* À titre d'exemple, avec une molécule d'ADN double-brin selon l'invention comprenant une longe en ADN double-brin d'environ 50 000 paires de bases, la C*_{eff}* est de l'ordre des nM. Ceci veut dire qu'il est possible de mesurer les temps caractéristiques d'association, τ_{A}, pour des réactions dont les constantes cinétiques d'association entre molécules à tester sont aussi grandes que 3×10⁹ M⁻¹s⁻¹, par exemple, avec une acquisition vidéo fonctionnant à quelques dizaines de Hz (e.g. 10 à 30 Hz).

La longueur de persistance de la longe en ADN double-brin permet en outre, lorsque la molécule d'ADN double-brin est utilisée pour mesurer des interactions entre au moins deux molécules à tester, de sonder la dépendance du temps de vie d'un complexe moléculaire (e.g. entre deux molécules à tester) en fonction de la force physique. En effet la molécule d'ADN double-brin s'étire facilement, ce qui permet de mesurer la différence d'extension entre les conformations associées et dissociée de molécules à tester lorsque des forces faibles, en dessous d'environ 2 pN, sont appliquées. De façon avantageuse, il est possible de mesurer la différence d'extension entre les conformations associées et dissociée de molécules à tester lorsque des forces aussi faibles que 0,1 pN sont appliquées (par exemple avec une longe d'environ 700 pb). En revanche pour parvenir au même résultat avec des molécules ayant une longueur de persistance plus petite (e.g. des molécules d'ADN simple-brin, de polypeptide), il faut appliquer des forces plus importantes pour étendre significativement la longe dans l'état dissocié, et donc avoir une chance de pouvoir le distinguer de l'état associé. Ceci empêche donc toute étude utilisant des forces en dessous de l'ordre de quelques pN avec ces molécules.

La longueur de la longe est un paramètre important dans la mesure d'interactions entre des molécules et elle peut varier selon les besoins expérimentaux (i.e. selon le but de l'expérience, selon les longueurs des molécules d'ADN double-brin (1) et (2) et/ou les molécules à tester). Une longe trop courte (e.g. inférieure à 300 pb) peut notamment faire qu'une interaction qui vient de se terminer entre deux molécules à tester se reforme si rapidement qu'il n'est pas possible de la distinguer de la précédente. À l'inverse, une augmentation de la longueur de la longe réduit la fréquence à laquelle les interactions entre des molécules à tester sont observées. Ceci peut être utile pour l'étude des réactions où l'association se fait très rapidement, mais peut être rédhibitoire pour l'étude des réactions où l'association se fait lentement. La longueur appropriée de la longe peut être déterminée par l'homme du métier en fonction des besoins expérimentaux, notamment en tenant compte des aspects détaillés ci-dessus. À titre d'exemple, dans le cadre de la présente invention, la longe peut avoir une longueur d'environ 300 à environ 50 000 paires de bases (± 50 paires de bases).

De façon préférée, la longe d'ADN double-brin a une longueur d'environ 300 à environ 50 000 paires de bases, avantageusement d'environ 500 à 10 000 paires de bases, avantageusement de 1 000 à 10 000 paires de bases, de 600 à 3 000 paires de bases (± 50 paires de bases), plus avantageusement de 600 à 1 000 paires de bases, encore plus avantageusement d'environ 700 paires de bases (± 50 paires de bases) ou d'environ 6 000 paires de bases (± 50 paires de bases). Avantageusement, lorsque la longe est assez longue, par exemple lorsque sa longueur est de 6 000 kbp, la distance z_{LF-A} peut être facilement distinguée de la distance z_{LF-D}.

La longe peut être attachée à la première molécule (1) et/ou à la deuxième molécule (2) d'ADN double-brin par un ou plusieurs liaisons covalentes qui ne sont pas des liaisons phosphodiesters, phosphorothioates, phosphoramidates ou phosphorodiamidates. Avantageusement, au moins une liaison covalente non-phosphodiester, non-phosphorothioate, non-phosphoramidate ou non-phosphorodiamidate, est formée entre un nucléotide de la longe comprenant un groupe fonctionnel et un nucléotide de la première molécule (1) et/ou de la deuxième molécule (2), comprenant un autre groupe fonctionnel. À titre d'exemple non-limitatif, la première molécule d'ADN double-brin (1) peut comprendre une fonction azoture qui réagit avec une fonction DBCO présente sur la longe par la technique de chimie click, formant ainsi une liaison covalente non-phosphodiester non-phosphorothioate, non-phosphoramidate ou non-phosphorodiamidate entre les deux molécules.

La longe peut être attachée aux molécules d'ADN double-brin (1) et (2) par ses deux extrémités, respectivement, ou par des nucléotides localisés dans la région intermédiaire de la longe. Par « *région intermédiaire* » on entend au sens de la présente invention un segment d'une molécule qui est localisé entre les deux extrémités de ladite molécule. De telles régions intermédiaires sont notamment schématisées pour les molécules d'ADN (1) et (2), à titre d'exemple, dans la **Figure 1** (segments en gris foncé).

Par « *extrémité* » on entend, au sens de la présente invention, les 30 à 150 paires de bases localisées à l'extrémité d'une molécule (e.g. de la longe, de la première molécule d'ADN double-brin (1), de la deuxième molécule d'ADN double-brin (2), avantageusement les 30 à 50 paires de bases localisées à l'extrémité d'une molécule. De telles extrémités sont notamment schématisées pour les molécules d'ADN (1) et (2), à titre d'exemple, dans la **Figure 1** (segments en gris clair). Ainsi, à titre d'exemple, une longe, (ou la première ou deuxième molécule d'ADN double-brin (1) ou (2)), comprenant 1 000 paires de bases, comprend deux extrémités, une première extrémité correspondante aux paires de bases 1 à 150 et une deuxième extrémité correspondant aux paires de bases 850 à 1 000. Dans le présent contexte, les termes première extrémité et deuxième extrémité ne doivent pas être interprétés comme correspondant à des extrémités spécifiques de la longe, mais permettre simplement de différencier une extrémité de la longe de l'autre. Ainsi, il n'a y pas de notion de direction ou d'orientation associée auxdites première et deuxième extrémités. L'homme de métier saura facilement identifier les extrémités d'une molécule d'ADN double-brin telle qu'elle est définie ici.

Une liaison entre une extrémité d'une longe et une molécule d'ADN double-brin (1) ou (2) peut donc avoir lieu à une ou plusieurs bases nucléotidiques d'une extrémité d'une longe. Avantageusement, au moins un nucléotide ultime, de préférence une base, sucre ou phosphate ultime, d'une extrémité de la longe est attachée par une liaison covalente non-phosphodiester, non-phosphorothioate, non-phosphoramidate ou non-phosphorodiamidate, à la première ou deuxième molécule d'ADN double-brin (1) ou (2).

Ainsi, selon un mode de réalisation préféré, la longe est attachée par sa première extrémité à la première molécule d'ADN double-brin (1) et par sa deuxième extrémité à la deuxième molécule d'ADN double-brin (2), avantageusement par un nucléotide ultime, de préférence une base, sucre ou phosphate ultime, de chaque extrémité de la longe. De façon avantageuse, la longe est attachée par au moins deux nucléotides, de préférence d'au moins deux bases nucléotidiques, dans chaque extrémité à la première et la deuxième molécule d'ADN double-brin (1) et (2), respectivement, lesdites au moins deux bases nucléotidiques comprenant, de façon plus avantageuse, un nucléotide ultime, de préférence une base, sucre ou phosphate ultime, à chaque extrémité de la longe.

Selon un mode de réalisation alternatif, la longe peut être attachée à la première molécule d'ADN double-brin (1) et/ou à la première molécule d'ADN double-brin (2) par au moins une base localisée dans la région intermédiaire de la longe, lesdites bases étant avantageusement séparés par au moins 300 paires de bases, plus avantageusement par au moins 500 paires de bases, encore plus avantageusement par au moins 600 paires de bases. À titre d'exemple non-limitatif, la longe peut être attachée à la première molécule d'ADN double-brin (1) par au moins une première base, ladite base étant localisée entre le point central de la longe et sa première extrémité et/ou attachée à la deuxième molécule d'ADN double-brin (2) par au moins une deuxième base, ladite base étant localisée entre le point central de la longe et sa deuxième extrémité. Par « *point central* » on entend une localisation qui est sensiblement équidistante entre la première et la deuxième extrémité de la molécule. Ceci est particulièrement avantageux lorsqu'au moins une extrémité de la longe comprend un autre élément de la molécule d'ADN double-brin selon l'invention (e.g. est liée à une autre molécule à tester).

Dans certains cas, le site de la liaison covalente entre la longe et la première molécule d'ADN double-brin (1) et/ou la deuxième molécule d'ADN double-brin (2) est localisé dans la région intermédiaire de ladite molécule d'ADN double-brin (1) ou (2), c'est-à-dire entre les deux extrémités de ladite molécule d'ADN (1) ou (2). À titre d'exemple non-limitatif, une liaison covalente non-phosphodiester, non-phosphorothioate, non-phosphoramidate ou non-phosphorodiamidate, est formée entre une extrémité de la longe (ou tout autre point de la longe qui sera approprié, tel que décrit ci-dessus), et un point central de ladite molécule d'ADN double-brin (1). La liaison covalente non-phosphodiester, non-phosphorothioate, non-phosphoramidate ou non-phosphorodiamidate, entre la longe et ladite première molécule d'ADN double-brin (1) forme ainsi une jonction avec trois embranchements (voir par exemple la **Figure 1**).

Dans d'autres cas, une liaison covalente entre la longe et la première molécule d'ADN double-brin (1) et/ou la deuxième molécule d'ADN double-brin (2) peut être localisée dans une extrémité de ladite molécule d'ADN double-brin (1) ou (2), ou à une base nucléotidique, qui permet de distinguer l'extrémité de la molécule d'ADN double-brin (1) ou (2) de sa région intermédiaire (voir, par exemple, la **Figure 1**). Dans le contexte de la présente invention, aucune liaison covalente n'est avantageusement formée entre un nucléotide ultime, de préférence une base, sucre ou phosphate ultime, de la molécule d'ADN (1) ou (2) et une base quelconque de la longe.

Ainsi, selon un mode de réalisation préféré de l'invention, la longe est attachée à la première molécule d'ADN double-brin (1) par une première liaison covalente entre la première extrémité de ladite longe et une région intermédiaire de la première molécule d'ADN double-brin (1) et à la deuxième molécule d'ADN double-brin (2) par une deuxième liaison covalente entre la deuxième extrémité de ladite longe et une région intermédiaire de la deuxième molécule d'ADN double-brin (2).

Les extrémités des molécules d'ADN double-brin (1) et (2) n'étant pas attachées à la longe, elles restent avantageusement disponibles pour d'autres finalités (e.g. pour d'autres interactions). À titre d'exemple, les deux extrémités de la première molécule d'ADN double-brin (1) ou de la deuxième molécule d'ADN double-brin (2) peuvent notamment être liées respectivement à une molécule à tester et à un support.

De façon avantageuse, une première extrémité de ladite première molécule d'ADN double-brin (1) est liée à une première molécule à tester et une première extrémité de la ladite deuxième molécule d'ADN double-brin (2) est liée à une deuxième molécule à tester. La molécule à tester peut être liée de façon directe ou indirecte à l'extrémité. La liaison d'une première ou d'une deuxième molécule à tester à une première extrémité de la première ou de la deuxième molécule d'ADN double-brin (1) ou (2) est avantageuse car elle permet d'éloigner les molécules à tester de la longe et du reste de la molécule d'ADN double-brin (1) et/ou (2). Les molécules à tester sont notamment moins susceptibles de subir une gêne stérique et de voir les caractéristiques de leur interaction modifiées dans cette configuration. De plus, l'utilisation d'une molécule d'ADN double-brin selon l'invention permet de laisser la longe et les molécules d'ADN double-brin (1) et (2) relativement libres de se réorienter dans l'espace indépendamment l'une de l'autre, de telle sorte que les molécules à tester ne se trouvent privées d'aucun degré de liberté de mouvement. Enfin, la liaison des molécules à tester aux extrémités des molécules d'ADN double-brin (1) et (2) permet également d'éloigner les molécules à tester des supports, ce qui diminue les interactions non-spécifiques. Dans certains cas, une molécule à tester peut être liée de de façon directe ou indirecte à un nucléotide ultime, de préférence une base, sucre ou phosphate ultime, d'une extrémité de la molécule d'ADN double-brin (1) ou (2). Lorsque la molécule à tester est liée de façon indirecte, elle est avantageusement liée par un « *espaceur* ». L'espaceur peut correspondre, par exemple, à une séquence de bases nucléotidiques double-brin ou simple-brin, à un polymère, un peptide, ou une petite molécule, et peut être aisément sélectionné par l'homme de métier. L'addition d'espaceurs aux molécules d'ADN double-brin (1) et (2) peut être effectuée avec n'importe laquelle des méthodes couramment utilisées en biologie moléculaire.

Selon une mode de réalisation préféré de l'invention, au moins une molécule à tester est liée de façon directe ou indirecte à un nucléotide ultime, de préférence une base, sucre ou phosphate ultime, d'une extrémité d'une molécule d'ADN double-brin donnée. Selon un autre mode de réalisation préféré, une molécule à tester est liée de façon indirecte à un nucléotide ultime, de préférence une base, sucre ou phosphate ultime, d'une extrémité d'une molécule d'ADN (1) ou (2), avantageusement par un espaceur.

Dans une configuration typique de l'invention, les deux molécules à tester sont liées de façon spécifique à la première et la deuxième molécule d'ADN à double-brin (1) et (2), respectivement. A titre d'exemple, une extrémité de la première molécule d'ADN double-brin (1) et une extrémité de la deuxième molécule d'ADN double-brin (2) sont digérées avec une ou plusieurs nucléases, par exemple l'enzyme *Nb.BbvCI,* afin de générer deux bouts cohésifs différents (voir aussi la **Figure 5A**). Des oligonucléotides synthétiques auxquelles des molécules à tester ont été liées, peuvent ensuite être hybridées et ligaturées de façon spécifique aux extrémités par des bouts cohésifs complémentaires (voir aussi la **Figure 5B**). De façon alternatif, chaque molécule à tester peut être liée de façon spécifique à une extrémité de la molécule d'ADN double-brin (1) ou (2) par une liaison covalente, par exemple une liaison covalente non-phosphodiester, non-phosphorothioate, non-phosphoramidate ou non-phosphorodiamidate, selon les méthodes décrites ici, par exemple par la technique de chimie click.

À titre d'exemple non-limitatif, au moins une des molécules à tester, telle que ladite première et/ou ladite deuxième molécule à tester, est sélectionnée dans le groupe composé des molécules suivantes : polymères, acides aminés, peptides, polypeptides, protéines, nucléosides, nucléotides, polynucléotides, oligonucléotides, sucres, polysaccharides, petites molécules, drogues, aptamères, antigènes, anticorps, lipides, lectines, hormones, vitamines, virus, fragments de virus, nanoparticules, molécules cellulaires de surface, et facteurs de transcription, ou des analogues ou peptidomimétiques de l'une de celles-ci. Dans certains cas, la première et la deuxième molécule à tester sont de même nature (e.g. deux protéines, deux polymères, deux molécules d'ADN double-brin, etc.), voire sont identiques (e.g. deux sous-unités d'une protéine, deux protéines formant un homodimère telles que des fragments de cadhérines). Dans d'autres cas, la première molécule à tester est de nature différente de la deuxième molécule à tester (e.g. antigène/anticorps, virus/récepteur, facteur de transcription/ADN, protéine-petite molécule, etc.). Lorsque au moins une des molécules à tester est une molécule d'ADN double-brin, ladite molécule est avantageusement liée directement à l'extrémité d'une des molécules d'ADN (1) ou (2).

Dans certains cas, une ou plusieurs autres molécules à tester peuvent être liées à la molécule d'ADN double-brin selon l'invention. Avantageusement, l'une ou plusieurs autres molécules à tester peut également être choisie dans le groupe de molécules ci-dessus. À titre d'exemple, une troisième molécule à tester peut être liée à une extrémité d'une molécule d'ADN double-brin (3) ou à une extrémité de la longe lorsque celle-ci est libre. Dans certains cas, au moins une autre molécule à tester peut être mise en contact avec la molécule d'ADN double-brin selon l'invention comprenant au moins deux molécules à tester, par exemple dans une suspension aqueuse. À titre d'exemple non-limitatif, ladite troisième molécule peut être un cofacteur de la première et/ou de la deuxième molécule à tester.

Selon un mode préférentiel de l'invention, ladite première et/ou ladite deuxième molécule à tester est sélectionnée dans le groupe composé des molécules suivantes : polymères, acides aminés, peptides, polypeptides, protéines, nucléosides, nucléotides, polynucléotides, oligonucléotides, sucres, polysaccharides, petites molécules, drogues, aptamères, antigènes, anticorps, lipides, lectines, hormones, vitamines, virus, fragments de virus, nanoparticules, molécules cellulaires de surface, et facteurs de transcription. Avantageusement, ladite première molécule à tester est de nature différente de ladite deuxième molécule à tester. Avantageusement, ladite première molécule à tester est de même nature, voire est identique, à ladite deuxième molécule à tester.

Dans une configuration typique de l'invention, la première et la deuxième molécule d'ADN à double-brin (1) et (2) peuvent être spécifiquement attachées à des supports. Par « *support* » on entend tout type de surface ou substrat solide, ladite support pouvant être avantageusement fonctionnalisée pour pouvoir réagir avec une extrémité fonctionnalisée de la molécule d'ADN double-brin selon l'invention. À titre d'exemple non-limitatif, le support peut être une bille (e.g. des billes de silice, verre à pores contrôlés, billes magnétiques, billes de séparation biomagnétiques telles que Dynabeads^{®}, résine Wang, résine Merrifield, résine copolystyrène chlorométhylée - divinylbenzène (DVB), billes Sephadex^{®} / Sepharose^{®}, billes de cellulose, etc.), un support plat tel qu'un filtre en fibre de verre, une surface diélectrique (e.g. verre, silice, nitrure de silicium, alumine), une surface métallique (e.g. acier, or, argent, aluminium, et cuivre), une surface semi-conductrice (e.g. silicium, semi-conducteur III-V, semi-conducteur II-VI), des matériaux plastiques, y compris plaques ou membranes multipuits (e.g. en polyéthylène, polypropylène, polyamide, polyfluorure de vinylidène), une aiguille, une micropipette, ou un cantilever utilisé en microscopie à force atomique. Une bille selon l'invention peut avoir n'importe quelle structure en trois dimensions et n'importe quelle taille. Avantageusement, la taille de la bille est comprise entre 0,5 et 100 µm en diamètre.

Les supports sont avantageusement des substrats solides (par exemple une surface de verre telle qu'une lame de microscope, une micropipette, une microparticule), qui peuvent être du même type (i.e. deux microparticules) ou de type différent (e.g. une surface de verre et une microparticule). De façon avantageuse, dans le cadre de la présente invention, lorsque des molécules à tester sont liées aux premières extrémités des première et deuxième molécules d'ADN (1) et (2), la deuxième extrémité de ladite première molécule d'ADN double-brin (1) est liée à un premier support et la deuxième extrémité de la ladite deuxième molécule d'ADN double-brin (2) est liée à un deuxième support.

De façon avantageuse, au moins un des deux supports est une bille telle qu'une microbille, une microparticule, une surface en verre, une micropipette, ou un cantilever utilisé en microscopie à force atomique.

Afin d'attacher des molécules d'ADN double-brin à des supports, on peut utiliser l'une quelconque des techniques connues dans le domaine. À titre d'exemple, l'ADN peut être lié directement sur un support, par exemple une microbille, ce qui implique une fonctionnalisation de ce support, par exemple en l'enrobant de streptavidine, d'un polymère portant des groupes COOH, etc., capable de réagir avec une extrémité fonctionnalisée de l'ADN double-brin.

De telles méthodes nécessitent en général de fonctionnaliser une molécule d'ADN double-brin, notamment à l'une de ses extrémités, c'est-à-dire de greffer au moins un groupe fonctionnel approprié sur celle-ci. À cette fin, différentes procédures peuvent être adoptées. Dans le cadre de la présente invention, le plus simple est de fonctionnaliser, à l'aide d'oligonucléotides synthétiques, une extrémité de la première molécule d'ADN double-brin (1) avec un groupe fonctionnel et une extrémité de la deuxième molécule d'ADN double-brin (2) avec un groupe fonctionnel. De façon avantageuse, dans le cadre de la présente invention, l'extrémité de la première molécule d'ADN (1) est fonctionnalisée avec un premier groupe fonctionnel et la deuxième molécule d'ADN (2) est fonctionnalisée avec un deuxième groupe fonctionnel, ce qui permet d'attacher une extrémité sur chacune des deux supports qui ont été prétraitées de façon différente (e.g. une première extrémité fonctionnalisée avec la biotine s'attache à un support recouvert de streptavidine alors que une deuxième extrémité, fonctionnalisée avec un group amine, s'attache à un support recouvert d'un polymère portant des groupes COOH, respectivement).

L'avantage de cette méthode réside dans sa capacité à fonctionnaliser tout type de molécule d'ADN double-brin ayant n'importe quelle longueur en conservant les mêmes réactifs. Dans ce cas, les deux extrémités des molécules d'ADN double-brin (1) et (2) destinées à être attachées à des supports sont clivées en utilisant par exemple deux enzymes de restriction (ou plus), ce qui permet d'obtenir une première molécule d'ADN ayant un premier site de restriction à l'une de ses extrémités et une deuxième molécule d'ADN ayant un deuxième site de restriction à l'une de ses extrémités. Ceci permet de traiter les deux extrémités différemment.

Dans certains cas, il peut être avantageux d'ajouter un « *espaceur* » à la suite duquel on ajoute un groupe fonctionnel à une extrémité de la première molécule d'ADN double-brin (1) et/ou à une extrémité de la deuxième molécule d'ADN double-brin (2) ; les deux séquences espaceur donnant ainsi à chaque groupe fonctionnel de l'espace supplémentaire pour se lier à son support dédié. L'espaceur peut correspondre à une séquence de bases nucléotidiques double-brin ou simple-brin, un polymère, un peptide, ou une petite molécule, tel que décrit ci-dessus. L'espaceur comprend avantageusement au moins un groupe fonctionnel. L'addition d'espaceurs aux molécules d'ADN double-brin (1) et (2) peut être effectuée avec n'importe laquelle des méthodes couramment utilisées en biologie moléculaire. Ces méthodes sont bien connues de l'homme du métier et il n'y a donc pas lieu de les détailler ici.

En ce qui concerne les techniques d'attachement elles-mêmes, elles sont nombreuses et elles dérivent des techniques d'attachement de macromolécules (protéines, ADN, etc.) à des supports prétraités qui sont disponibles dans le commerce ou facilement réalisable en laboratoire. La plupart de ces techniques ont été développées pour des tests d'immunologie, et relient des protéines (immunoglobulines) à des supports portant des groupes (-COOH, -NH2, -OH, etc.) capables de réagir avec le carboxyle (-COOH) ou les extrémités amine (-NH2) des protéines.

L'attachement de la molécule d'ADN double-brin selon l'invention à un support peut être réalisé directement, via le phosphate libre de l'extrémité 5' de la molécule, qui réagit avec une amine secondaire (surface Covalink -NH commercialisée par Polylabo à Strasbourg) pour former une liaison covalente. Il est également possible de fonctionnaliser l'ADN avec un groupe amine puis de procéder comme avec une protéine. À titre d'exemple alternatif, une molécule ADN fonctionnalisée avec un group thiol (S-H) se lie de façon covalente à un support en or par la formation d'une liaison thiolate S-Au.

Il existe également des supports revêtus de streptavidine (e.g. billes de Dynal et similaires), qui permettent un attachement quasi-covalent entre la streptavidine et une molécule d'ADN biotinylée. Enfin, en greffant un anticorps dirigé contre la digoxigénine sur un support (par les méthodes mentionnées ci-dessus), une molécule d'ADN fonctionnalisée par la digoxigénine peut y être attachée. Ceci représente simplement un exemple d'une des nombreuses techniques d'attachement possibles. Parmi les techniques de fixation et d'attachement, on citera par exemple les techniques décrites dans le brevet EP 152 886 utilisant un couplage enzymatique pour la fixation de l'ADN sur un support solide tel que la cellulose. Le brevet EP 146 815 décrit également diverses méthodes de fixation d'ADN à un support. De même, la demande de brevet WO 92/16659 propose une méthode utilisant un polymère pour fixer l'ADN. Naturellement, une molécule d'ADN peut être fixée directement sur un support mais, le cas échéant, et notamment en vue de limiter l'influence des supports, la molécule d'ADN peut être fixée à la fin d'un bras inerte de nature peptidique ou autre (autrement dit, un espaceur), tel que par exemple cela est décrit dans le brevet EP 329 198. Les deux molécules d'ADN double-brin (1) et (2) étant liées à des supports différents, elles peuvent être liées à leurs supports respectifs par des moyens différents.

Selon un mode de réalisation préféré de l'invention, lesdites deuxièmes extrémités desdites première et deuxième molécules d'ADN double-brin (1) et (2) sont attachées auxdits premier et deuxième supports de façon directe ou indirecte. De façon avantageuse, au moins un des deux supports est un support mobile, avantageusement une bille, plus avantageusement une bille magnétique. De façon avantageuse, lesdites deuxièmes extrémités desdites première et deuxième molécules d'ADN double-brin (1) et (2) sont attachées auxdits premier et deuxième supports à un seul point ou à plusieurs points. De façon avantageuse, lesdites deuxièmes extrémités desdites première et deuxième molécules d'ADN double-brin (1) et (2) sont attachées au premier et au deuxième supports respectivement par une base nucléotidique, ladite base étant avantageusement fonctionnalisée. De façon avantageuse, ladite deuxième extrémité desdites première et deuxième molécules d'ADN double-brin (1) et (2) sont attachées par au moins deux bases nucléotidiques, lesdites bases étant plus avantageusement fonctionnalisées.

Selon un aspect particulier de l'invention, la molécule d'ADN double-brin selon l'invention comprend deux molécules à tester. Cependant, selon la configuration de la molécule (i.e. selon le nombre d'extrémités dans les différentes molécules d'ADN et/ou la longe comprise dans la molécule d'ADN double-brin selon l'invention, ladite molécule peut comprendre au moins trois, quatre, cinq, ou six molécules à tester. Lesdites molécules à tester pourraient notamment être liées aux extrémités de la longe ou à d'autres molécules d'ADN double-brin (e.g. une troisième molécule d'ADN double-brin (3), une quatrième molécule d'ADN double-brin (4), etc.). De préférence, lorsque la molécule d'ADN double-brin selon l'invention comprend au moins trois molécules d'ADN double-brin, lesdites molécules sont également attachées à la longe, avantageusement à des bases différentes.

L'invention a pour autre objet la molécule d'ADN double-brin telle que décrite ici, pour une utilisation dans la détection et/ou la caractérisation des interactions entre au moins deux molécules à tester, avantageusement la détermination des propriétés thermodynamiques et/ou cinétiques de ces interactions. À titre d'exemple non-limitatif, la caractérisation de l'interaction comprend la détermination d'au moins une des caractéristiques choisies parmi : le temps caractéristique d'association, le temps caractéristique de dissociation, la constante de vitesse de dissociation, l'énergie d'activation de dissociation, la distance séparant l'état de transition du complexe lors de la dissociation, et la constante d'équilibre de dissociation. Ainsi, selon un mode préféré, la molécule d'ADN double-brin est utilisée pour la caractérisation d'au moins une interaction moléculaire, avantageusement choisi parmi les caractéristiques décrites ci-dessus, entre au moins deux molécules à tester.

### DISPOSITIF

L'invention a pour autre objet un dispositif comprenant la molécule d'ADN double-brin telle que décrite ici avec ses supports. Un exemple d'un tel dispositif est notamment illustré dans la **Figure 2****.** Avantageusement, le dispositif selon l'invention est utilisé pour détecter et/ou mesurer des interactions, avantageusement pour mesurer des propriétés thermodynamiques et/ou cinétiques, entre au moins deux molécules à tester, telles que celles décrites ci-dessus.

Alors que la caractérisation d'une interaction s'effectue à l'échelle d'une molécule unique, il est possible de mesurer des interactions individuelles de façon simultanée, au sein d'au moins deux molécules d'ADN double-brin différents. Une méthode de détection ayant un niveau de résolution suffisamment élevée pour distinguer entre les interactions ayant lieu au sein des différentes molécules d'ADN double-brin peut notamment être utilisée. Afin de mesurer plusieurs (e.g. au moins deux) interactions individuelles de façon simultanée, plusieurs molécules d'ADN double-brin peuvent être liées à un même support. La distribution des molécules d'ADN double-brin peut être faite de façon régulière, par exemple, sur un support de type réseau ou puce, ou de façon aléatoire, de préférence à une densité permettant de pouvoir mesurer les interactions au sein de chaque molécule d'ADN double-brin séparément. Dans certains cas, il peut être avantageux de séparer les molécules d'ADN double-brin au sein du dispositif (e.g. par une répartition dans des puits individuels). Les molécules d'ADN double-brin peuvent être liées à un même support fixe et à un même support mobile (e.g. un système multicantilevers, comprenant par exemple une surface de verre en tant que support fixe et un réseau de cantilevers microfabriqués sur un même substrat en tant que support mobile). De façon alternative, les molécules d'ADN double-brin peuvent être liées à un même support fixe et à différents supports mobiles (e.g. des pinces magnétiques, comprenant par exemple une surface de verre en tant que support fixe et des billes en tant que supports mobiles, chaque molécule d'ADN double-brin étant liée à une bille différente). De façon alternative, les molécules d'ADN double-brin peuvent être liées à différents supports fixes et à différents supports mobiles (e.g. des pinces optiques multiplexées, comprenant par exemple des billes en tant que supports fixes et en tant que supports mobiles, chaque molécule d'ADN double-brin étant liée à un couple de billes différent).

Dans une configuration typique, la molécule d'ADN double-brin selon l'invention est attachée de manière spécifique entre deux supports solides, l'une des extrémités de la première molécule d'ADN double-brin (1) pouvant être fixée directement ou indirectement à un support, tandis que l'une des extrémités de la deuxième molécule d'ADN double-brin (2) est attachée directement ou indirectement à un support mobile. L'une des extrémités de la première molécule d'ADN double-brin (1) peut plus particulièrement être fixée directement ou indirectement à un support fixe ou mobile.

Ledit dispositif selon l'invention comprend avantageusement au moins deux molécules d'ADN double-brin selon l'invention, lesdites molécules étant liées aux supports, les différentes molécules d'ADN double brin pouvant être liées aux mêmes supports et/ou à des supports différents. Avantageusement, lors de la liaison des molécules d'ADN double-brin aux supports, lesdites molécules sont liées à une densité permettant à chaque molécule individuelle d'être résolue individuellement, avantageusement dans laquelle chaque molécule individuelle est ou peut devenir adressable spatialement.

Comme l'extension des au moins deux molécules d'ADN double-brin peut être mesurée de façon simultanée mais indépendante, les molécules à tester et donc les interactions moléculaires à caractériser dans les au moins deux molécules d'ADN double-brin peuvent être différentes. La caractérisation de différentes interactions est particulièrement avantageuse lorsqu'un grand nombre de molécules à tester doivent être évaluées, par exemple lors d'un criblage destiné à identifier une molécule interagissant avec un récepteur particulier ou modulant l'interaction entre deux protéines. À titre d'exemple, différentes molécules à tester peuvent être liées à l'une des molécules d'ADN (1) ou (2) ou introduites en solution aqueuse pour caractériser les interactions entre ladite molécule et le(s) molécule(s) à tester liée(s) à la molécule d'ADN double-brin. Dans certains cas, il est possible qu'aucune interaction moléculaire ne soit détectée entre les molécules à tester.

Dans certains cas, la molécule d'ADN selon l'invention, ainsi que le dispositif comprenant ladite molécule et ses supports, peuvent être utilisées dans le contexte d'études à haut-débit, par exemple, lors d'un criblage pharmaceutique pour la recherche de nouvelles molécules ayant des caractéristiques d'interaction particulières avec une molécule particulière (e.g. un récepteur, un complexe protéique). En effet, il est connu que différentes drogues peuvent interagir avec un même récepteur avec des cinétiques différentes, engendrant des effets différents. Il est aussi connu que différentes drogues peuvent interagir avec des assemblées de protéines et en moduler la stabilité. Ainsi, l'utilisation du présent dispositif est très avantageuse pour la détermination de différentes caractéristiques cinétiques et/ou thermodynamiques.

De façon alternative, une même interaction moléculaire peut être caractérisée en parallèle pour chaque molécule d'ADN, lorsque celles-ci comprennent les mêmes molécules à tester. Ceci permet notamment de moyenner les mesures, et ainsi d'avantageusement d'améliorer la précision. Ceci permet aussi de s'assurer de la reproductibilité des mesures. Lorsque les au moins deux molécules d'ADN double-brin comprennent les mêmes molécules à tester, elles sont avantageusement liées aux deux supports selon une même orientation (e.g. la première molécule à tester est liée à la première molécule d'ADN double-brin (1), lui-même liée à un premier support pour chaque la molécule d'ADN double-brin).

### MOLECULE D'ADN « INTERMEDIAIRE »

Un autre aspect de l'invention concerne une molécule « *intermédiaire* » à partir de laquelle la molécule d'ADN double-brin selon l'invention peut être obtenue. L'invention a donc pour autre objet une molécule d'ADN double-brin comprenant une première molécule d'ADN double-brin (A) et une deuxième molécule d'ADN double-brin (B), ladite molécule d'ADN double-brin (A) comprenant un site de clivage qui est présent uniquement dans ladite molécule d'ADN double-brin (A), ladite molécule d'ADN double-brin (A) étant reliée à la molécule d'ADN double-brin (B) par deux liaisons covalentes qui ne sont pas des liaisons phosphodiesters, phosphorothioates, phosphoramidates ou phosphorodiamidates, de part et d'autre dudit site de clivage. Des exemples d'un telle molécule « *intermédiaire* » sont notamment illustrés dans la **Figure 3****.**

Avantageusement, lesdites premières extrémités des molécules d'ADN double-brin (1) et (2) sont différentes des deuxièmes extrémités des molécules d'ADN double-brin (1) et (2). Par première et deuxième extrémité, on fait référence aux deux extrémités différentes au sein de la molécule d'ADN double-brin (1) ou (2). Cependant, les termes « *première et deuxième extrémités* » ne doivent pas être interprétés comme correspondant à des extrémités spécifiques, mais permettre simplement de différencier une extrémité d'une molécule de l'autre extrémité. Ainsi, il n'a y pas de notion de direction ou d'orientation associée auxdites première et deuxième extrémités.

Avantageusement, les quatre extrémités de la molécule d'ADN double-brin (e.g., les deux extrémités de la molécule d'ADN double-brin (1) et les deux extrémités de la molécule d'ADN double-brin (2)) sont différentes.

La molécule d'ADN double-brin (A) a avantageusement une longueur comprise entre environ 600 et 10 000 paires de bases, avantageusement entre environ 1 000 et 4 000 paires de bases, avantageusement entre environ 1 500 et 3 000 paires de bases (± 50 paires de bases). La molécule d'ADN double-brin (B) a avantageusement une longueur comprise entre environ 300 à environ 50 000 paires de bases, avantageusement choisi selon le but de l'expérience et les molécules à tester d'environ 500 à 10 000 paires de bases, avantageusement de 600 à 3 000 paires de bases, plus avantageusement de 600 à 1 000 paires de bases, encore plus avantageusement d'environ 700 paires de bases (± 50 paires de bases).

Par « *site de clivage* » on entend au sens de la présente invention une structure ou séquence polynucléotidique qui est capable d'être clivée de façon spécifique par un agent de clivage, tel qu'une enzyme de restriction, une nucléase, une nickase, un ribozyme, une DNAzyme, et des fragments de celles-ci. À titre d'exemple non-limitatif, le site de clivage peut donc être un site d'enzyme de restriction, un site de ribozyme, un site de nickase, un site de DNAzyme ou un site de clivage de nucléase. De tels agents et de tels sites sont bien connus par l'homme de métier. Par « *enzyme de restriction* » on entend plus particulièrement une enzyme qui coupe l'ADN double-brin au niveau ou à proximité d'une séquence nucléotidique spécifique. Les spécificités de nombreuses enzymes de restriction sont bien connues dans l'art et un grand nombre d'enzymes de restriction sont disponibles dans le commerce et leurs conditions de réaction, la nécessité de la présence de cofacteurs et autres exigences établies par les fournisseurs d'enzymes sont bien connues. À titre d'exemple non-limitatif, l'enzyme de restriction peut être une enzyme de restriction de type II, de type III ou artificielle (telle qu'une nucléase à doigts de zinc, une nucléase effectrice de type activateur de transcription (TALEN), une méga-nucléase, ou une endonucléase de type CRISPR), encore plus préférablement une enzyme de restriction de type II. Les enzymes de restriction de type II comprennent les catégories IIP, IIS, IIC, IIT, IIG, IIE, IIF, IIG, IIM et IIB, comme décrit par exemple dans Pingoud et Jeltsch, 2001. L'enzyme de restriction peut générer des bouts francs (i.e. les deux brins ayant le même longueur) ou des bouts cohésifs (un brin étant plus long que l'autre brin, généralement de quelques nucléotides).

Selon un mode préféré de l'invention, le site de clivage dans la molécule d'ADN double-brin (A) de la molécule d'ADN double-brin « *intermédiaire* » est un site d'enzyme de restriction, avantageusement un site d'enzyme de restriction de type II, plus avantageusement deux sites de restriction, encore plus avantageusement deux sites de restriction générant deux bouts différents (e.g. un bout franc et un bout cohésif, ou deux bouts cohésifs non-complémentaires). À titre d'exemple non-limitatif, le site de clivage peut consister en un site de restriction de l'enzyme Sacl et un site de restriction de l'enzyme Xbal. Lorsque le site de clivage comprend au moins deux sites différents, lesdits sites peuvent être séparés l'un de l'autre, par exemple par au moins 1, au moins 2, au moins 3, au moins 4, au moins 5, au moins 6, au moins 12, au moins 25, au moins 50, au moins 100, au moins 200, au moins 500, ou au moins 1000 paires de bases. De façon avantageuse, lesdits sites sont séparés par au moins 6 paires de bases (« *Restriction Endonucleases Technical Guide* », New England Biolabs).

### PROCEDE DE FABRICATION DE LA MOLECULE D'ADN

Un autre aspect de l'invention concerne un procédé de fabrication d'une molécule d'ADN double-brin à partir d'une molécule d'ADN double-brin « *intermédiaire ».* L'invention a donc pour autre objet un procédé de fabrication caractérisé en ce qu'il comprend l'étape suivante :
a) le clivage de la molécule d'ADN double-brin (A) audit site de clivage, générant ainsi une molécule d'ADN double-brin comprenant une première molécule d'ADN double-brin (1) et une deuxième molécule d'ADN double-brin (2).

L'étape de clivage peut être effectuée par des techniques bien connues de l'homme de métier, tel qu'une digestion double par deux enzymes de restriction de façon simultanée ou séquentielle. Avantageusement, le clivage est effectué par au moins un agent de clivage telle que définie ci-dessus, plus avantageusement par au moins une enzyme de restriction telle que définie ci-dessus. Avantageusement le clivage est effectué par deux enzymes de restriction différentes permettant d'obtenir deux bouts cohésifs différents, avantageusement lesdits bouts cohésifs étant non-complémentaires. Avantageusement, les deux sites de clivage sont séparés l'un de l'autre selon l'une des modes de réalisation décrit ci-dessus.

Avantageusement, après l'étape a), au moins une étape supplémentaire peut être effectuée. Une telle étape pourrait comprendre, par exemple, une étape de fonctionnalisation d'une extrémité de la première et/ou de la deuxième molécule d'ADN double-brin (1) et (2), et/ou la liaison d'une molécule à tester à une extrémité de la première et/ou de la deuxième molécule d'ADN double-brin (1) et (2).

Avantageusement, le procédé de fabrication de la molécule d'ADN double-brin selon l'invention comprend, en outre après l'étape a), l'étape suivante :
b) l'attachement d'au moins une base fonctionnalisée à une extrémité de ladite première molécule d'ADN double-brin (1) et l'attachement d'une autre base fonctionnelle à une extrémité de ladite deuxième molécule d'ADN double-brin (2).

Selon un premier mode de réalisation, les extrémités fonctionnalisées sont celles générées lors de l'étape a). Selon un deuxième mode de réalisation, les extrémités fonctionnalisées sont celles déjà présentes dans la molécule d'ADN double-brin (A) de la molécule d'ADN double-brin « *intermédiaire* » avant l'étape a). De façon avantageuse, l'extrémité de la première molécule d'ADN double-brin (1) et l'extrémité de la deuxième molécule d'ADN double-brin (2) sont fonctionnalisées par deux fonctions différentes, de façon à ce qu'elles puissent être liées à des supports différents. À titre d'exemple non-limitatif, une extrémité de la première molécule d'ADN double-brin (1) est fonctionnalisée par l'ajout d'au moins un groupe biotine alors qu'une extrémité de la deuxième molécule d'ADN double-brin (2) est fonctionnalisée par l'ajout d'au moins un groupe digoxigénine. Ainsi, l'extrémité biotinylée peut se lier de façon quasi-covalente à une surface ou un support recouvert de streptavidine alors que l'extrémité fonctionnalisée par la digoxigénine peut se lier à une surface ou un support recouvert d'anticorps dirigé contre la digoxigénine. De façon préférée, l'extrémité de la première molécule d'ADN double-brin (1) et/ou l'extrémité de la deuxième molécule d'ADN double-brin (2) sont fonctionnalisés par l'ajout de plusieurs groupes afin d'améliorer l'accrochage de l'extrémité au support. Selon un mode de réalisation particulier, le(s) groupe(s) fonctionnel(s) sont présent(s) dans une molécule d'ADN simple-brin ou double-brin qui est incorporée à l'extrémité de la première molécule d'ADN double-brin (1) et dans une autre molécule d'ADN simple-brin ou double-brin qui est incorporée à l'extrémité de la deuxième molécule d'ADN double-brin (2) en utilisant des techniques bien connues par l'homme du métier (e.g. hybridation et ligation). À titre d'exemple non-limitatif, la **Figure 4** illustre les étapes a) et b) telles que décrites ici.

Selon un mode préférentiel de l'invention, ladite au moins une base fonctionnalisée est comprise dans une troisième (3) et/ou quatrième molécule d'ADN double-brin (4), ladite troisième molécule d'ADN double-brin (3) étant avantageusement liée à la deuxième extrémité de ladite première molécule d'ADN double-brin (1), et ladite quatrième molécule d'ADN double-brin (4) étant avantageusement liée à la deuxième extrémité de la deuxième première molécule d'ADN double-brin (2). Avantageusement, ladite au moins une base fonctionnalisée de ladite molécule d'ADN (3) double-brin est fonctionnalisée par un groupe fonctionnel différent de ladite au moins une base fonctionnalisée de ladite molécule d'ADN double-brin (4).

Avantageusement, la molécule d'ADN double-brin (1), (2), (3) et/ou (4) a une longueur comprise entre 600 et 10 000 paires de bases, plus avantageusement entre 650 à 1500 paires de bases.

Lorsqu'un fragment d'ADN simple-brin est présent à l'extrémité d'une des molécules d'ADN double-brin (1) ou (2) après incorporation d'une molécule d'ADN fonctionnalisée, ledit fragment peut être avantageusement :
1) enlevé, par exemple par une enzyme ayant une activité exonucléase, ou
2) complété par l'ajout d'un deuxième fragment complémentaire, par exemple par l'activité d'une polymérase ADN (e.g. fragment Klenow, le polymérase T4, etc.) en présence des dNTPs.
Avantageusement, le procédé de fabrication de la molécule d'ADN double-brin selon l'invention comprend, en outre après l'étape a), l'étape suivante :
c) la liaison d'une première molécule à tester à une extrémité de la ladite première molécule d'ADN double-brin (1) et la liaison d'une deuxième molécule à tester à une extrémité de la ladite deuxième molécule d'ADN (2).

À titre d'exemple non-limitatif, la **Figure 5** illustre cette étape.

Avantageusement, la liaison selon l'étape c) comprend la liaison d'une cinquième (5) molécule d'ADN comprenant ladite première molécule à tester à ladite première extrémité de la première molécule d'ADN double-brin (1), et/ou la liaison d'une sixième (6) molécule d'ADN comprenant ladite deuxième molécule à tester à ladite première extrémité de la deuxième molécule d'ADN double-brin (2).

Ladite cinquième (5) molécule d'ADN et/ou sixième (6) molécule d'ADN comprenant une molécule à tester peut avantageusement être une molécule d'ADN double-brin comprenant une extrémité débordante ou un oligonucléotide. Dans ce cas, la liaison selon l'étape c) comprend avantageusement des étapes d'hybridation et de ligation afin de lier les molécules d'ADN ensemble. Toutefois, les molécules à tester peuvent être liées auxdites extrémités des molécules d'ADN double-brin (1) et (2) selon n'importe quelle technique connue par l'homme de métier.

L'étape c) peut être effectuée avant ou après l'étape b). Selon un autre mode de réalisation, une des étapes b) et c) est effectuée avec l'étape a) (i.e. en ajoutant les molécules à tester ou les groupes fonctionnels aux extrémités déjà présentes dans la molécule d'ADN double-brin précurseur (A), et l'autre des étapes b) et c) est effectuée après l'étape a), lorsque les deux molécules d'ADN double-brin (1) et (2) ainsi que leurs deuxièmes extrémités sont générées. Ceci peut être avantageux lorsque la première extrémité de la molécule d'ADN double-brin (1) et/ou la première extrémité de la molécule d'ADN double-brin (2), comprise dans la molécule d'ADN précurseur double-brin (A) ont un bout identique (i.e. un même bout cohésif ou franc) à au moins une des extrémités qui est formée par le clivage de la molécule d'ADN double-brin (A). Ceci permet avantageusement d'attacher chaque groupe fonctionnel et chaque molécule à tester à une extrémité particulière, tout en utilisant un nombre d'enzymes de restriction réduit. Avantageusement, avant l'étape c) les extrémités auxquelles seront fixées les molécules à tester sont clivées, plus avantageusement par une enzyme de restriction, telle que décrite ci-dessus. Toutefois, cette étape n'est pas nécessaire lorsque les extrémités concernées ont déjà subi une étape de clivage auparavant (par exemple avant l'étape a) ou avant l'étape b)).

De façon alternative, les molécules à tester peuvent être liées directement aux molécules d'ADN double-brin (1) et (2) à des bouts francs (par exemple selon la méthode de ligation à bout franc). Cette technique est bien connue de l'homme du métier. À titre d'exemple non-limitatif, la ligation à bout franc comprend des étapes de déphosphorylation des extrémités des molécules d'ADN double-brin (1) et (2) et de phosphorylation de l'extrémité libre des molécules d'ADN comprenant les molécules à tester avant de mettre les molécules en contact dans un tampon approprié et en présence d'une ligase.

Avantageusement, l'attachement d'au moins une des molécules d'ADN fonctionnalisées à l'étape b) et/ou l'attachement d'au moins une des molécules à tester à l'étape c) comprend des étapes d'hybridation et de ligation, avantageusement par hybridation de deux bouts cohésifs complémentaires (i.e. l'un étant présent sur la molécule d'ADN double-brin (1) ou (2) et l'autre étant présent sur une molécule d'ADN comprenant au moins une base fonctionnalisée ou une molécule à tester). À titre alternatif, l'attachement d'au moins une des molécules d'ADN fonctionnalisées à l'étape b) et/ou l'attachement d'au moins une des molécules à tester à l'étape c) comprend une étape de conjugaison, avantageusement entre au moins deux groupes fonctionnels, plus avantageusement selon des méthodes de la chimie click.

Dans le cas d'études d'interactions entre des molécules à tester qui sont des protéines, il est envisageable de faire appel à des réactions de marquage où des « *protéines étiquettes* » sont fusionnées avec les molécules à tester et viennent réagir de façon spécifique avec des ligands se situant aux extrémités des molécules d'ADN (1) et/ou (2) (comme illustré **Figure 7B**). De nombreux systèmes sont aujourd'hui commercialement disponibles, qui permettent autant de réactions orthogonales. Les adduits formés peuvent, par exemple, être covalents comme dans le cas de la fixation par la protéine SNAP-tag du ligand benzylguanine, par CLIP-tag de la benzylcytosine, par HaloTag d'un groupe chloroalkane, ou par une combinaison d'au moins deux de celles-ci. De façon alternative, on pourrait avoir recours à la formation de complexes non-covalents comme ceux résultant de l'interaction entre la streptavidine et la biotine, entre un épitope et un antigène ou encore entre une cible protéique et son aptamère. Dans ces trois derniers cas, il serait avantageux que la complexation soit suffisamment forte pour conduire à la fonctionnalisation d'un nombre important des extrémités par les molécules à tester et pour qu'il n'y ait pas rupture à la traction.

De façon avantageuse, l'utilisation des « *protéines étiquettes* » rend superflue l'étape de digestion des extrémités, illustré à la **Figure 5A**. Avantageusement, il n'y a plus à synthétiser de conjugués « *oligonucléotide* - *molécule à tester* », ce qui est assez long et qui peut parfois être complexe à cause de de problématique liées à la purification et à la stabilité des espèces obtenues. Ainsi, de façon avantageuse, on se repose simplement sur l'expression et la purification de protéines de fusion.

Avantageusement, lesdites premières extrémités des molécules d'ADN double-brin (1) et (2) sont différentes des deuxièmes extrémités des molécules d'ADN double-brin (1) et (2).

Avantageusement, le procédé de fabrication de la présente invention utilise des techniques de synthèse biochimiques, et non un assemblage de type « *origami* ». La structure de la molécule d'ADN double-brin selon l'invention peut être garantie à la base près, et est donc de très haute qualité. En effet, les inventeurs ont notamment montré lors des études de surenroulement, qu'aucune liaison phosphodiester, phosphorothioate, phosphoramidate ou phosphorodiamidate, n'est manquante sur les environ 3600 bases qui sont comprises dans la molécule d'ADN double-brin exemplifié dans l'Exemple 9 et la **Figure 12**.

### PROCEDE DE FABRICATION - ETAPES SUPPLEMENTAIRES

Le procédé de fabrication selon l'invention peut comprendre en outre une ou plusieurs étapes supplémentaires, telle qu'une ou plusieurs étapes de synthèse en amont de l'étape a), permettant avantageusement de générer la molécule d'ADN double-brin « *intermédiaire* », à partir de laquelle la molécule d'ADN double-brin selon l'invention peut être obtenue. À titre d'exemple non-limitatif, le procédé de fabrication comprend en outre, en amont de l'étape a), les étapes suivantes :
- la synthèse d'un oligonucléotide (1) comprenant une jonction obtenue par réaction d'un premier groupe fonctionnel présent sur l'extrémité d'un premier oligonucléotide avec un deuxième groupe fonctionnel présent sur l'un des nucléotides intermédiaires d'un deuxième oligonucléotide ;
- la synthèse d'un oligonucléotide (2) comprenant une jonction obtenue par réaction d'un premier groupe fonctionnel présent sur l'extrémité d'un troisième oligonucléotide avec un deuxième groupe fonctionnel présent à un point intermédiaire d'un quatrième oligonucléotide (voir aussi les exemples illustrés aux **Figures 6A** **et** **7A**) ;
- la synthèse de ladite molécule d'ADN double-brin (A), ladite molécule d'ADN (A) comprenant l'oligonucléotide (1) à sa première extrémité et l'oligonucléotide (2) à sa deuxième extrémité (voir aussi l'exemple illustré à la **Figure 6B**) ; et
- l'attachement d'une longe, par la première et la deuxième extrémité de ladite longe, à la jonction de l'oligonucléotide (1) et de l'oligonucléotide (2), respectivement (voir aussi l'exemple illustré à la **Figure 6C** et **6D**).

Selon un premier mode de réalisation, l'étape de synthèse de la molécule d'ADN double-brin (A) est effectuée par PCR en utilisant les oligonucléotides (1) et (2) en tant qu'amorces. Avantageusement, la molécule (A) est produite par PCR, avantageusement suivi par la digestion enzymatique de chacune de ses extrémités par une enzyme de restriction, avantageusement par deux enzymes de restriction différentes.

Selon un deuxième mode de réalisation, l'étape de synthèse de la molécule d'ADN double-brin (A) comprend des étapes d'hybridation et de ligature de l'oligonucléotide (1) à la première extrémité d'une molécule d'ADN double-brin, tel qu'un génome de phage, et de l'oligonucléotide (2) à la deuxième extrémité. Avantageusement, la molécule d'ADN double-brin (A) comprend un surplomb à chacune de ses extrémités, avantageusement comprend deux surplombs différents. Avantageusement l'oligonucléotide (1) et l'oligonucléotide (2) comprennent des régions d'ADN complémentaires auxdits surplombs.

Avantageusement, l'attachement de la molécule (B) comprend l'hybridation et la ligature d'un cinquième oligonucléotide sur la jonction de l'oligonucléotide (1) et d'un sixième oligonucléotide sur la jonction de l'oligonucléotide (2), respectivement, afin de générer des surplombs sur chaque jonction (voir par exemple, **Figure 6C**).

Avantageusement, la molécule d'ADN double-brin (B) est synthétisée par PCR, plus avantageusement suivi par la digestion enzymatique de chacune de ses extrémités par une enzyme de restriction, encore plus avantageusement par deux enzymes de restriction différentes (voir par exemple, **Figure 6C**).

La molécule d'ADN double-brin (A) et/ou la molécule d'ADN double-brin (B) a avantageusement au moins une des caractéristiques (e.g. longueur), telles que décrites ci-dessus. Avantageusement, l'invention a pour autre objet la molécule d'ADN double-brin comprenant une première molécule d'ADN double-brin (A) et une deuxième molécule d'ADN double-brin (B), ladite molécule (A) comprenant un site de clivage qui est présent uniquement dans ladite molécule (A), ladite molécule (A) étant reliée à la molécule (B) par deux liaisons covalentes qui ne sont pas des liaisons phosphodiesters, phosphorothioates, phosphoramidates ou phosphorodiamidates, de part et d'autre dudit site de clivage obtenu par le procédé décrit ci-dessus.

Avantageusement, la longueur de la première molécule d'ADN double-brin (1), de la deuxième molécule d'ADN (2), et de la longe peuvent être facilement ajustées lors de la fabrication de la molécule d'ADN double-brin selon le procédé de fabrication tel que décrit ici.

Avantageusement, l'ajustement de la longueur de la longe permet de moduler la concentration effective en molécules à tester (aussi appelé « *C_{eff}* »). Ceci est d'ailleurs d'autant plus facile que l'ADN double-brin a une grande longueur de persistance. La longueur de la longe peut notamment être déterminée en fonction de la longueur des molécules d'ADN double-brin (1) et (2), ou vice-versa, et selon la concentration effective des molécules à tester et/ou la résolution spatiotemporelle souhaitée. En effet, dans certains cas, une réduction de la longueur totale de la molécule d'ADN double-brin selon l'invention d'un facteur numérique N, augmente la résolution spatiotemporelle de l'expérience d'environ le même facteur N, ledit facteur N étant avantageusement compris entre 1 et 6. Cependant, il peut être avantageuse de conserver une longueur de la longe d'au moins 300 pb. Il peut également être avantageux de conserver une longueur des molécules d'ADN double-brin (1) et (2) d'au moins 100 pb afin d'éviter les interactions entre les supports et les molécules à tester. Pour des constructions comprenant déjà une longe très courte, (e.g. moins d'environ 300 paires de bases), la seule façon de réduire la longueur de la construction est de raccourcir la longueur des première et deuxième molécules d'ADN (1) et (2).

### PROCEDE DE CARACTERISATION D'INTERACTIONS MOLECULAIRES

L'invention a pour autre objet un procédé de détection et/ou de caractérisation d'au moins une interaction moléculaire entre au moins deux molécules à tester, lesdites molécules à tester étant avantageusement liées à une molécule d'ADN double-brin selon l'invention. La caractéristique déterminée peut être, par exemple, de nature thermodynamique ou cinétique. À titre d'exemple, un temps caractéristique d'association, un temps caractéristique de dissociation, une constante de vitesse de dissociation, une énergie d'activation de dissociation, une distance séparant l'état de transition du complexe lors de la dissociation, et/ou une constante d'équilibre de dissociation peut être déterminé.

Plus particulièrement, l'invention a pour objet un procédé de caractérisation d'une interaction entre au moins deux molécules à tester, lesdites molécules à tester étant liées à une molécule d'ADN double-brin, ou à une molécule d'ADN double-brin telle que comprise dans le dispositif de l'invention, comprenant :
a) l'application d'une force physique basse, F_{LF}, à la molécule d'ADN double-brin, qui permet aux molécules à tester de s'associer ;
b) l'application d'une force physique haute, F_{HF}, à la molécule d'ADN double-brin, qui permet de déterminer si les molécules à tester sont associées ou dissociées ; et
c) la détection d'un changement dans les propriétés conformationnelles de la molécule d'ADN comprenant :
   - la détermination de l'extension z_{LF} entre la deuxième extrémité de la première molécule d'ADN double-brin (1) et la deuxième extrémité de la deuxième molécule d'ADN double-brin (2) à l'étape a) ;
   - la détermination des extensions z_{HF-A} et z_{HF-D} entre la deuxième extrémité de la première molécule d'ADN double-brin (1) et la deuxième extrémité de la deuxième molécule d'ADN double-brin (2), à l'étape b) , dans laquelle z_{HF-A} est l'extension quand les molécules sont associées et z_{HF-D} est l'extension quand les molécules sont dissociées ; et
   - la comparaison des extensions z_{LF}, z_{HF-A}, et z_{HF-D}, en fonction du temps t.

Il pourra être avantageux pour mesurer l'association entre les deux molécules à tester, d'initier le cycle par un palier à haute force dont la valeur peut être plus élevée que F_{HF}. Selon ce mode de réalisation préféré, le procédé comprendra une étape préalable d'application d'une force physique supérieure à la force F_{HF} de l'étape c).

Dans un autre mode de réalisation préférentiel, le procédé de l'invention permet avantageusement de distinguer les extensions z_{LF-A} et z_{LF-D}. Ceci est notamment le cas lorsque la longe a une longueur d'au moins 700 bp. A cet égard, on notera qu'il est particulièrement intéressant d'utiliser une longe d'au moins 6 000 bp, laquelle permet de distinguer aisément les extensions z_{LF-A} et z_{LF-D}.

Selon ce mode de réalisation préféré, ledit procédé comprend en outre l'étape supplémentaire :
d) la détection d'un changement dans les propriétés conformationnelles de la molécule d'ADN comprenant :
- la détermination des extensions z_{LF-A} et z_{LF-D} entre la deuxième extrémité de la première molécule d'ADN double-brin (1) et la deuxième extrémité de la deuxième molécule d'ADN double-brin (2) à l'étape a), dans laquelle z_{LF-A} est l'extension quand les molécules sont associées et z_{LF-D} est l'extension quand les molécules sont dissociées;
- la détermination des extensions z_{HF-A} et z_{HF-D} entre la deuxième extrémité de la première molécule d'ADN double-brin (1) et la deuxième extrémité de la deuxième molécule d'ADN double-brin (2), à l'étape b), dans laquelle z_{HF-A} est l'extension quand les molécules sont associées et z_{HF-D} est l'extension quand les molécules sont dissociées ; et
- la comparaison des extensions z_{LF-A}, z_{LF-D}, z_{HF-A}, et z_{HF-D}, en fonction du temps t.

Dans un mode de réalisation plus préféré, la longe possède une longueur d'au moins 700 bp. Encore plus préférentiellement, la longe possède une longueur d'au moins 6000 bp.

Dans certains cas, les étapes a) à c) ou a) à d) telles que décrites ci-dessus peuvent être répétées plusieurs fois, afin de suivre de multiples cycles de dissociation/association. La répétition des étapes est très avantageuse car elle permet d'augmenter le nombre de mesures et ainsi d'améliorer les statistiques et la fiabilité de la caractérisation, notamment en filtrant les résultats sur des critères de reproductibilité. Ainsi, selon un mode préférentiel, les étapes a) à c) du procédé sont répétées plusieurs fois.

Plus particulièrement, l'invention a pour autre objet un procédé de caractérisation d'une interaction entre au moins deux molécules à tester, lesdites molécules à tester étant liées à une molécule d'ADN double-brin, ou à une molécule d'ADN double-brin telle que comprise dans le dispositif de l'invention, comprenant :
a) l'application d'une force constante F_{CF}, à la molécule d'ADN double-brin, qui permet aux molécules à tester de s'associer et de se dissocier ; et
b) la détection d'un changement dans les propriétés conformationnelles de la molécule d'ADN comprenant :
   - la détermination de la dissociation spontanée des molécules à tester au bout du temps t_{CF-A}, et/ou
   - la détermination de l'association spontanée au bout d'un temps T_{CF-D}.

Avantageusement, le procédé de caractérisation d'une interaction comprend l'ajout d'au moins une molécule supplémentaire en solution. Ladite molécule présente en solution peut être la même molécule que l'une des molécules à tester qui sont accrochées aux extrémités de la molécule d'ADN double-brin selon l'invention ou une molécule différente. Avantageusement, la force appliquée lors de la caractérisation d'une interaction par fluctuation spontanée est d'au moins 30 fN, de préférence une force compris entre 30 et 60 fN.

Une « *force physique* » ou « *force* » selon l'invention correspond à toute influence qui fait subir à un objet un certain changement, que ce soit en ce qui concerne son mouvement, sa direction ou sa construction géométrique (e.g. sa conformation). Il apparaîtra clairement à l'homme du métier qu'une force selon l'invention est différente d'autres paramètres physiques tels que, par exemple, la température (qui est une propriété directe de la matière plutôt qu'une influence exercée sur celle-ci). Les forces physiques selon l'invention comprennent des forces telles que la friction, la tension (aussi appelée « *force de traction* »), la rotation, la force normale, la force de résistance d'un fluide, la force appliquée et la force élastique. De manière préférée, la force physique selon l'invention comprend une force de tension, avantageusement la force physique selon l'invention consiste en une force de tension. La force physique selon l'invention peut également comprendre une force de rotation, avantageusement un couple. La force physique peut consister en une force de couple. Dans certains cas, la force physique comprend la tension et la rotation, avantageusement une tension et un couple. La force est exprimée ici en picoNewtons (pN), sauf indication contraire explicite. L' « *application* » de telles forces physiques est bien connue par l'homme de métier, notamment dans le contexte des différents appareils dans lesquels la molécule d'ADN, ou le dispositif comprenant la molécule d'ADN, selon l'invention peut être incorporé (voir par exemple Woodside et al., 2006; les brevets U.S. N° 7,052,650 et 7,244,391 ; WO 2011/147931 ; et Yang et al., 2016).

Par « *extension* »*,* on entend au sens de la présente invention la distance entre deux extrémités d'un polymère. Dans la présente invention, l'extension correspond plus particulièrement à la distance « z » mesurée entre les supports auxquelles la molécule d'ADN double-brin selon l'invention est attachée. L'extension est donc être inférieure ou égale à la longueur de la molécule d'ADN double-brin. L'extension de la molécule d'ADN double brin est exprimée en nm ou µm. La distance z_{LF}, correspond à la distance entre deux extrémités de la molécule d'ADN selon l'invention, de façon préférée la distance entre les supports auxquelles la molécule d'ADN double-brin selon l'invention est attachée, lorsqu'une basse force F_{LF} est appliquée. À titre d'exemple, en-dessous d'un seuil de force appliqué, qui varie selon les molécules à tester (généralement compris entre 0,01 et 0,4 pN), les supports sont proches l'un de l'autre. La distance z_{HF}, correspond à la distance entre deux extrémités de la molécule d'ADN selon l'invention, de façon préférée la distance entre les supports auxquelles la molécule d'ADN double-brin selon l'invention est attachée, lorsqu'une haute force F_{HF} est appliquée. À titre d'exemple, au-dessus d'un seuil de force appliqué, qui varie selon les molécules à tester (généralement compris entre 0,5 et 70 pN), les supports sont éloignés l'un de l'autre. Lorsque les molécules à tester s'associent, deux distances peuvent être déterminées, correspondant à deux états d'extension, z_{HF-A} et z_{HF-D}. Plus précisément, les distances z_{HF-A} et z_{HF-D} correspondent à la distance entre deux extrémités de la molécule d'ADN selon l'invention lorsqu'une haute force F_{HF} est appliquée, et que les molécules à tester sont associées (z_{HF-A}) ou dissociées (z_{HF-D}). La distance z_{HF-D} est supérieure à la distance z_{HF-A}, du fait que les molécules d'ADN double-brin (1) et (2) sont liées l'une à l'autre uniquement par la longe (en non par les molécules à tester), voir aussi schéma aux **Figures 8****,** **10A****.** La conformation de la molécule d'ADN double-brin peut donc être déterminée par des mesures directes de l'extension de celle-ci, par exemple, sous une force, comme décrit ici.

Les différentes distances « z » sont déterminées au cours du temps et en fonction de la force appliquée. Dans certains cas, il peut être avantageux de mesurer les distances de façon précise (e.g. en nm). De façon générale, l'extension de la molécule d'ADN double-brin lorsque les molécules à tester sont associées (A, e.g. z_{HF-A}) correspond approximativement à l'extension d'une molécule linéaire d'ADN double-brin de longueur la somme des longueurs des molécules d'ADN double-brin (1) et (2), à laquelle est appliquée une force F_{HF}. De façon générale, l'extension de la molécule d'ADN double-brin lorsque les molécules à tester sont dissociées (D, e.g. z_{HF-D}) correspond approximativement l'extension d'une molécule linéaire d'ADN double-brin de longueur la somme des la longueur de la longe plus la somme des longueurs des molécules d'ADN double-brin (1) et (2) à l'exclusion des parties se situant entre les molécules à tester et les jonctions, à laquelle est appliquée une force F_{HF}. À haute force, extension et longueur sont presque proportionnelles, ce qui favorise l'établissement de correspondance entre les différents z.

L'extension peut notamment être déduite de la mesure de la distance entre les deux supports (e.g. le support fixe et le support mobile), ce dernier pouvant, par exemple, être localisé par suivi vidéo, comme dans les cas de billes magnétiques, ou par déflection de faisceau laser, comme dans le cas de certains pièges optiques ou de l'AFM. Une force physique est avantageusement appliquée aux deux extrémités attachées aux deux supports lorsque les supports sont éloignés. Lorsque les changements de conformation de la molécule d'ADN double-brin sont observés sous une force physique, par exemple sous une tension, la transition entre l'association et la dissociation des molécules à tester est indiquée par une augmentation de l'extension de la molécule d'ADN double-brin, la distance z_{HF-A} correspondant à un état d'association entre les deux molécules étant plus faible que la distance z_{HF-D} correspondant à un état de dissociation entre les deux molécules.

Toutefois, la détermination des différentes distances « z » n'est pas obligatoire. En effet, dans certains cas, il peut être avantageux de simplement déterminer si chaque état d'extension est présent, afin de déterminer si des molécules à tester sont capables d'interagir. À titre d'exemple, une telle détermination peut être effectuée en identifiant, sur une trace temporelle de z_{HF}, un changement de l'extension qui est attribuée au passage entre la mesure de la distance z_{HF-A} et la mesure de la distance z_{HF-D}. Un tel changement peut, par exemple, être abrupt (e.g. en moins de 3 points d'acquisition, soit 90 millisecondes pour une fréquence d'acquisition vidéo de 30 Hz), telle qu'illustré à la **Figure 10B****.**

Par « *trace temporelle* » on entend le suivi d'une interaction moléculaire (e.g. association/dissociation) en fonction d'une force appliqué et au cours du temps (à titre d'exemple, voir la **Figure 10B** ou la **Figure 11A**).

Dans certains cas, l'extension mesurée (e.g. z_{LF}, z_{HF-A}, et/ou z_{HF-D}) peut être comparée avec une extension prédite par un modèle théorique, telle que le modèle d'élasticité WLC « *worm-like chain* » appliqué aux deux conformations, A et D, pour les deux forces étudiées, F_{LF} et F_{HF}. Ce modèle est particulièrement avantageux pour décrire le comportement élastique des molécules d'ADN double-brin (Bouchiat et al., 1999 et Sarkar et Rybenkov, 2016). Cependant d'autres modèles, tel celui de la chaîne librement jointe, peuvent aussi convenir (Doi, 1996, et Sarkar et Rybenkov, 2016).

Dans un mode de réalisation préféré, une force physique, par exemple une tension, est appliquée à la molécule d'ADN double-brin lorsque les supports sont éloignés. Lorsque la force physique haute est supérieure ou égale à 0,5 pN, 1 pN, 5 pN, 10 pN, 15 pN, 20 pN, 30 pN, 40 pN, 50 pN, ou 60 pN) il devient aisé de distinguer les changements de conformations par mesure de l'extension z, et donc de déterminer si les molécules à tester sont associées ou dissociées (**Figure 8**). Lorsque la force est basse (e.g. inférieure à 0,4 pN) l'extension diminue et les molécules à tester s'associent plus facilement car elles sont plus proches. Cependant, à basse force il est alors impossible de déterminer la conformation (**Figure 8** ; **Figure 10A**).

Avantageusement, la basse force correspond à une force constante (i.e. une seule force basse appliquée). Avantageusement, la force haute correspond à une force constante (i.e. une seule force haute appliquée). Avantageusement, la force appliquée est cyclée entre une force élevée constante (e.g. supérieure ou égale à 0,5 pN) et une basse force constante (e.g. égale ou inférieur à 0,4 pN), comme illustré dans la **Figure 8****.**

À titre alternatif, les fluctuations spontanées de la distance entre les supports, respectivement de la force, sont observées à force appliquée constante (voir, par exemple, Kim et al., 2010), respectivement à distance constante (voir, par exemple Kilchherr et al., 2016), comme illustré dans la Figure 17. Dans un troisième mode de réalisation, la force appliquée peut être augmentée entre une basse force et une force élevée de façon linéaire dans le temps (e.g. une augmentation de la force par 0,01 pN/s) et toute la courbe donnant l'extension en fonction de la force mesurée (voir, par exemple, Kim et al., 2010, Halvorsen et al., 2011 et Kilchherr et al., 2016).

Avantageusement, la force physique appliquée à l'étape a) est de l'ordre de 0,01 pN à 0,4 pN, avantageusement comprise entre 0,01 pN et 0,1 pN. En effet, en dessous d'une force physique de 0,01 pN, il devient difficile de déterminer assez précisément z_{LF} à cause du bruit de fond. Avantageusement, la force physique appliqué à l'étape a) est constante.

Avantageusement, la force physique appliquée à l'étape b) est supérieure ou égale à 0,5 pN, avantageusement comprise entre 0,5 et 70 pN. En effet, à une force physique au-delà de 70 pN, la longe risque de s'ouvrir car les points d'attache aux molécules d'ADN double-brin (1) et (2) sont situés sur des brins opposés. Toutefois, dans certains cas, la force physique appliquée pourrait être supérieure à 70 pN, notamment lorsque des interactions plus fortes doivent être étudiées et/ou que les deux brins de la longe ont été cross-linkés par des agents chimiques (par exemple le psoralène ou le cisplatine). De façon avantageuse, la force physique appliquée restera inférieure au seuil de déformation plastique de l'ADN (e.g. de 70 pN, en l'absence de crosslinking de la longe). Avantageusement, la force physique appliqué à l'étape b) est constante.

Selon un premier mode de réalisation préférentiel, lorsque des études d'interaction font intervenir des petites molécules en tant que molécules à tester, la force physique à l'étape b) est avantageusement comprise entre 0,5 et 70 pN.

Selon un premier mode de réalisation préférentiel, lorsque des études d'interaction font intervenir des protéines en tant que molécules à tester, la force physique à l'étape b) est avantageusement comprise entre 0,5 et 40 pN. En effet, au-delà de ce seuil certaines protéines commencent à se dénaturer.

La force physique appliquée peut varier avec la température, le type de molécule à tester et le tampon, mais l'homme du métier adaptera facilement ladite force physique vis-à-vis de ces paramètres afin de mesurer les interactions moléculaires.

Dans certains cas, un couple peut également être appliqué à la molécule d'ADN double-brin. La molécule d'ADN double-brin selon l'invention est très avantageuse car elle peut être utilisée pour effectuer des études mécaniques de réponse en torsion du complexe formé par les molécules à tester (**Figure 12**).

La comparaison des distances z_{LF}, z_{HF-A}, et z_{HF-D} en fonction du temps (t) permet, de façon avantageuse, de déterminer au moins une des caractéristiques choisies parmi : la constante de vitesse de dissociation, l'énergie d'activation de dissociation, la constante d'équilibre de dissociation. Il est également possible de déterminer, de façon avantageuse, le temps caractéristique de dissociation, qui n'est que l'inverse de la constante de vitesse de dissociation, et la constante d'équilibre d'association, qui n'est que l'inverse de la constante d'équilibre de dissociation. Plus précisément, la comparaison de ces distances au cours du temps donne le « *temps de vie* » de la conformation associée et/ou dissociée des molécules à tester, noté t_{LF-D} pour les expériences d'association et t_{HF-A} pour les expériences de dissociation.

Le « *temps caractéristique de dissociation* » correspond plus particulièrement à la durée de temps pendant laquelle l'extension z de la molécule, correspondant à un état d'association, est détecté. De même, le « *temps caractéristique d'association* » correspond plus particulièrement à la durée de temps pendant laquelle l'extension z de la molécule, correspondant à un état dissocié, est détecté.

Lorsque la force appliquée est cyclée entre une force élevée constante et une basse force constante, une ou plusieurs valeurs « t » peuvent être collectées. Les valeurs « t » sont avantageusement rassemblées sous forme d'histogramme, afin de pouvoir être analysées par ajustement exponentiel et déterminer le temps caractéristique de dissociation τ_{D} le temps caractéristique de dissociation τ_{A}. À titre d'exemple, l'histogramme des t_{LF-D} donne le temps caractéristique d'association τ_{A} en utilisant la formule Probabilité ∝ exp[-t_{LF-D}/τ_{A}] et l'histogramme des t_{HF-A} donne le temps caractéristique d'association τ_{D} en utilisant la formule Probabilité ∝ exp[-t_{HF-A}/τ_{D}]. À partir du temps caractéristique de dissociation, τ_{D}, il est notamment possible de calculer la constante de vitesse de dissociation, k_{D}, par inversion : k_{D} = 1 / τ_{D}.

La « *distance séparant l'état de transition du complexe lors de la dissociation* » correspond à la distance à partir de laquelle la configuration des molécules à tester (e.g. état associé) iront toujours vers une autre configuration (e.g. état dissocié). L'état de transition est une caractéristique bien connue de l'homme de métier (voir par exemple Pilling et Seakins, 1995). À titre d'exemple, en effectuant des expériences pour différentes valeurs de F_{HF} et/ou de la température, l'homme de métier pourra déterminer la distance séparant l'état de transition du complexe lors de la dissociation, ceci en utilisant l'équation d'Arrhenius/Bell (Popa et al., 2011). De même, l'homme de métier pourra déterminer l'énergie d'activation de la réaction de dissociation (Popa et al., 2011).

Dans certains cas, le procédé comprend en outre une étape de comparaison de ladite caractéristique avec une valeur de référence.

Le procédé tel que décrit ci-dessus peut comprendre en outre l'étape suivante :
d) l'ajout d'au moins une troisième molécule.

Lorsque la troisième molécule n'est pas incorporée dans la molécule d'ADN double-brin selon l'invention, elle est avantageusement ajoutée dans l'environnement du dispositif, plus avantageusement en solution aqueuse. Ainsi, selon un mode de réalisation préféré, le procédé de caractérisation d'une interaction a lieu dans un environnement aqueux. Selon un mode de réalisation particulier, la concentration de ladite troisième molécule peut varier au cours du temps. Le procédé selon l'invention peut notamment être utilisé pour caractériser des interactions en la présence ou l'absence d'au moins une troisième molécule qui a une activité agoniste ou antagoniste sur les interactions, telle qu'un cofacteur, un inhibiteur orthostérique ou allostérique, etc.

Dans certains modes de réalisation, une cinétique d'association et de dissociation peut être déterminée en mesurant les longueurs de la molécule d'ADN sur une période de temps, éventuellement dans une ou plusieurs conditions expérimentales (e.g. en changeant la force physique appliquée à l'étape b), le pH, la salinité, le tampon, la température, etc.).

Ainsi, le procédé selon l'invention permet de façon très avantageuse de réaliser des expériences complexes sur une même paire de molécules à tester, par l'introduction d'autres molécules interagissant avec les deux molécules à tester, et/ou par des changements des conditions expérimentales (e.g. force physique, pH, salinité).

De façon avantageuse, le procédé de l'invention permet la mesure du paramètre τ_{D}, qui permet d'évaluer la durée de vie des interactions entre les molécules à tester, ceci par exemple en traçant l'histogramme de temps de vie de la forme associée (Exemple 7).

Cependant il est également avantageux de mesurer τ_{A}, le temps caractéristique de formation des interactions, ceci par exemple en traçant l'histogramme de temps de vie de la forme dissociée (Exemple 7). À longueur de longe fixe, différentes conditions expérimentales peuvent être comparées : présence / absence de certains réactifs (par exemple pour comprendre les mécanismes de recrutement), pH, salinité, température.

Les procédés de caractérisation des interactions moléculaires selon l'invention impliquent la détection des changements de longueur des molécules d'ADN double-brin selon l'invention. Les interactions peuvent être détectées ou déterminées en utilisant un certain nombre de techniques connues dans l'art, dont celles qui permettent aussi la micromanipulation et l'application de force : microscopie à force atomique, pinces optiques, pinces magnétiques, microscopie à force centrifuge, biomembranes force probe, spectroscopie par force acoustique, micromanipulation à l'aide de cantilevers mécaniques ou de micro-aiguilles, etc. La détection des changements de longueur des molécules d'ADN double-brin selon l'invention peut aussi être effectuée à l'aide de techniques ne permettant pas la micromanipulation : « *tethered particule motion* »*,* microscopie de fluorescence (éventuellement en champ évanescent), spectroscopie de fluorescence (éventuellement résolue en espace et/ou en temps) selon des modes de quenching, transfert résonant d'énergie, etc. L'une quelconque de ces techniques peut être utilisée conjointement avec la molécule d'ADN double-brin, le dispositif, et les procédés décrits ici. Ces techniques sont connues dans la technique et certaines sont décrites brièvement ci-dessous (voir aussi, par exemple, Conroy, 2008).

À titre d'exemple, la force entre deux molécules à tester incorporées dans la molécule d'ADN double-brin selon l'invention peut être mesurée par microscopie à force atomique (AFM). Dans certains modes de réalisation, l'AFM peut être utilisé pour mesurer les forces d'étirement et de rupture du lieur à molécule unique. Dans certains modes de réalisation, la force mesurée peut être de l'ordre de quelques pN. Dans certains modes de réalisation, l'AFM est réalisée avec des modes statiques ou dynamiques.

La force entre deux molécules à tester incorporées dans la molécule d'ADN double-brin selon l'invention peut être mesurée à l'aide d'une pince optique (également appelée « *piège à force de gradient à faisceau unique* »). Les pinces optiques utilisent un faisceau laser hautement focalisé pour fournir une force attrayante ou répulsive (typiquement de l'ordre de pN), en fonction du désaccord d'indice de réfraction, pour maintenir physiquement et déplacer des objets diélectriques microscopiques, tels que l'ADN. Dans certains modes de réalisation, des pinces optiques sont utilisées pour manipuler la molécule d'ADN double-brin en exerçant des forces extrêmement faibles via un faisceau laser hautement focalisé. Dans certains modes de réalisation, des pièges optiques peuvent être utilisés pour détecter le déplacement de l'ADN en tant que mesure de la force moléculaire.

La force entre deux molécules à tester incorporées dans la molécule d'ADN double-brin selon l'invention peut être mesurée en utilisant une pince magnétique. Les pinces magnétiques exercent une force et un couple sur une molécule telle que la molécule d'ADN double-brin selon l'invention. L'extension de la molécule correspond à sa réponse à la contrainte appliquée. Avantageusement, un appareil de pince magnétique est équipé d'aimants qui sont utilisés pour manipuler les particules magnétiques, dont la position est mesurée avec une vidéo-microscopie.

La force entre deux molécules à tester incorporées dans la molécule d'ADN double-brin selon l'invention peut être mesurée en utilisant la microscopie à force centrifuge. Le CFM exerce une force sur une molécule telle qu'un complexe d'acide nucléique de l'invention en utilisant une force centrifuge. L'extension de la molécule correspond à sa réponse à la contrainte appliquée. Dans certains modes de réalisation, un complexe est fixé à une extrémité à un support fixe et à l'autre à une particule qui peut être visualisée en utilisant par exemple la microscopie optique. La position de la particule et son mouvement par rapport au support fixe peuvent être observés et mesurés en fonction de la force centrifuge appliquée à la molécule d'ADN double-brin.

D'autres technologies de mesure de force mécanique peuvent être utilisées avec les modes de réalisation décrits ici, par exemple des cantilevers mécaniques, des « *biomembrane force probes* », etc.

La molécule, le dispositif, et le procédé selon l'invention peuvent être utilisés dans un grand nombre d'applications, notamment conjointement avec l'une des techniques décrites ci-dessus. En plus des études des propriétés mécaniques et des interactions entre deux molécules à tester, ils peuvent aussi être utilisés pour détecter des analytes en solution, pour effectuer des études de liaison compétitifs, cribler des molécules, etc.

À titre d'exemple, des tests de liaison compétitifs peut être effectués, notamment par l'introduction de molécules avant ou après l'établissement d'une liaison entre les au moins deux molécules à tester. Dans certains modes de réalisation, une fois que les deux molécules à tester sont liées l'une à l'autre, des formes solubles ou des fragments de la première et/ou de la deuxième molécule à tester peuvent être ajoutés en excès, ceux-ci se liant à la première et/ou à la deuxième molécule à tester afin de rentrer en compétition avec leur contrepartie liée, avant de détecter un changement de liaison (par exemple par la détermination de l'extension z).

Selon un aspect, la molécule, le dispositif, et le procédé selon l'invention peuvent être utilisés pour détecter la présence d'un analyte d'intérêt dans un échantillon, par exemple à des fins diagnostics. Selon cet aspect, la molécule d'ADN comprend au moins deux molécules à tester qui ont une spécificité pour un même analyte. Dans certains cas, les au moins deux molécules à tester peuvent être identiques, à condition qu'elles puissent se lier simultanément à l'analyte. Par exemple, elles peuvent être des anticorps identiques, à condition que l'antigène auquel elles se lient ait plusieurs épitopes qui peuvent être liés par les différents anticorps simultanément et sans interférence. À titre alternatif, les au moins deux molécules à tester peuvent être différentes l'une de l'autre mais avoir une affinité de liaison pour un même analyte. Par exemple, elles peuvent être des anticorps qui se lient à différents épitopes sur le même antigène à condition qu'elles puissent se lier à l'antigène simultanément et sans interférence. La longueur de la molécule d'ADN lorsque les molécules à tester fixent l'analyte ou non peut être utilisée pour déterminer la présence ou l'absence d'un analyte dans un échantillon. Si l'analyte est présent, les molécules à tester se lieront à l'analyte. En l'absence de l'analyte, la liaison ne se produira pas et l'extension correspondra à z_{HF-D}.

Selon un autre aspect, la molécule, le dispositif, et le procédé selon l'invention peuvent être utilisés dans des méthodes de criblage de molécules. Dans ce cas, une seule ou les deux molécules à tester peuvent être connues comme pouvant interagir. Dans certains modes de réalisation, une des deux molécules à tester est un membre d'une bibliothèque de molécules identifié comme pouvant interagir avec l'autre molécule à tester de façon putative, et le procédé est conçu afin de cribler les différentes molécules de la bibliothèque afin d'identifier des molécules ayant une affinité pour une cible particulière (c.-à-d. l'autre molécule à tester). Dans certains modes de réalisation, les molécules à tester sont connues pour avoir une affinité l'une pour l'autre, alors que dans d'autres modes de réalisation, on ne sait pas *a priori* si elles ont une affinité mutuelle ou si le degré d'affinité dans une paire donnée est connu.

### KIT

L'invention a pour autre objet des kits pour la fabrication de la molécule d'ADN double-brin selon l'invention. Un tel kit peut comprendre au moins :
- une molécule d'ADN « *intermédiaire* » ayant des longueurs des molécules (A) et (B) préétablies en fonction des besoins expérimentaux,
- optionnellement, des oligonucléotides et/ou des molécules d'ADN comprenant des groupes fonctionnels,
- optionnellement, une ou plusieurs molécules à tester, avantageusement liées à des oligonucléotides.

Selon un mode de réalisation préféré, le kit comprend au moins la molécule d'ADN double-brin selon l'invention, avec ou sans l'intégration des molécules à tester dans ladite molécule d'ADN.

Selon un autre mode de réalisation préféré, le kit comprend le dispositif selon l'invention, avec ou sans l'intégration des molécules à tester dans la molécule d'ADN double-brin selon l'invention qui est comprise dans ledit dispositif.

Le kit peut en outre contenir au moins un quelconque des autres molécules ou réactifs décrits ici (e.g. une polymérase, une ligase, des dNTPs, un ou plusieurs tampons de réaction, un ou plusieurs supports tels que des billes magnétiques ou des lamelles de verre). Les différents composants du kit peuvent être présents dans des récipients séparés. Lorsque certains composants sont compatibles, ils peuvent être précombinés dans un seul récipient, comme souhaité.

En plus des composants mentionnés ci-dessus, les kits selon l'invention peuvent en outre comprendre des instructions pour utiliser lesdits composants du kit, plus particulièrement pour mettre en oeuvre les procédés de l'invention (e.g. instructions pour synthétiser la molécule d'ADN double-brin selon l'invention, des instructions pour générer le dispositif selon l'invention, etc.).

La pratique de l'invention utilise, sauf indication contraire, des techniques conventionnelles de la chimie des protéines, de la biologie moléculaire, de la microbiologie, de la technologie de l'ADN recombinant et de la pharmacologie, qui font partie des compétences de l'art. De telles techniques sont entièrement expliquées dans la littérature. (Voir Ausubel et al., Current Protocols in Molecular Biology, Eds., John Wiley & Sons, Inc. New York, 1995 ; Remington's Pharmaceutical Sciences, 17ème éd., Mack Publishing Co., Easton, Pa., 1985 ; Sambrook et al., Molecular cloning : Un manuel de laboratoire, 2e édition, Cold Spring Harbor Laboratory Press - Cold Spring Harbor, NY, USA, 1989 ; Lahann, Click Chemistry for Biotechnology and Materials Science, John Wiley & Sons, Chichester, England, 2009).

### EXEMPLES

L'invention est illustrée par les exemples non limitatifs suivants.

### EXEMPLE 1 : PROCEDE DE FABRICATION DE LA MOLECULE D'ADN DOUBLE-BRIN

Le protocole donné ci-dessous correspond à la synthèse des molécules d'ADN utilisées pour les Exemples 8 et 9. En ce qui concerne celles utilisées pour les Exemples 10 et 12, quelques variantes ont été mises en oeuvre, qui sont résumées dans le Tableau 1, ci-dessous.

**Tableau 1 : Résumé des informations concernant les tailles des molécules et leurs séquences, en fonction des Exemples**

| | Exemples 8 et 9 | Exemple 10 | Exemple 8 (longe de 6 kbp) | Exemple 12 |
|---|---|---|---|---|
| SEQ ID NO de la molécule (A) | 9 | 30 | 9 | 45 |
| Taille de la molécule (A) avant la digestion de l'étape A) | 3 000 pb | 2 100 pb | 3 000 pb | 2 100 pb |
| SEQ ID NO de la molécule (B) | 10 | 10 | 43 | 10 |
| Taille de la molécule (B) | Environ 700 pb (689 pb) | Environ 700 pb (689 pb) | Environ 6000 pb (6 058 bp) | Environ 700 pb (689 pb) |
| Taille totale de la molécule d'ADN avant l'étape A) | 3 700 pb | 2 800 pb | 9 000 bp | 2 800 pb |
| Tailles des fragments liés à la longe après la digestion de l'étape A) | 1 500 pb et 1 500 pb | 700 et 1 400 pb | 1 500 pb et 1 500 pb | 700 et 1 400 pb |
| Enzyme servant à digérer la molécule selon SEQ ID NO la 1, marqué à la biotine | Sacl | Xbal | Sacl | Xbal |
| Enzyme servant à digérer la molécule selon SEQ ID NO : 1, marqué à la digoxigénine | Xbal | Sacl | Xbal | Sacl |
| Taille totale de la molécule d'ADN après l'étape B) | Entre 5 700 et 6 300 pb | Entre 4 800 et 5 300 pb | Entre 11 000 et 11 600 bp | Entre 4 800 et 5 300 pb |
| SEQ ID NO de la molécule (1) | 26 ou 27 | 28 ou 29 | 26 ou 27 | 47 ou 48 |
| SEQ ID NO de la molécule (2) | 31 ou 32 | 33 ou 34 | 31 ou 32 | 49 ou 50 |

### ÉTAPE A) LA SYNTHESE DES MOLECULES D'ADN DOUBLE-BRIN (1) ET (2)

La digestion par deux enzymes de restriction de la molécule d'ADN « *intermédiaire* » (illustré à la **Figure 3****,** ayant les séquences de SEQ ID NO : 9 (correspondant à la séquence de la molécule d'ADN (A) et de SEQ ID NO: 10 (correspondant à la séquence de la molécule d'ADN (B)) permet la linéarisation. Par la même occasion, on obtient deux bouts cohésifs non-complémentaires (**Figure 4A**). Dans cette réalisation, ce sont les enzymes *Xbal* et *Sacl* qui sont utilisées.

Plus précisément, après digestion par les enzymes *Xbal* et *Sacl*, un produit d'ADN d'environ 3,7 kpb (kilo paires de bases) constitué de la molécule (B) d'environ 700 pb (correspondant à la longe) et de deux molécules d'ADN double-brin linéaires, (correspondant en parties aux molécules d'ADN double-brin (1) et (2)) de 1,5 kpb est obtenu. Dans chaque molécule d'ADN (1) et (2) chaque extrémité de la longe est attachée à une base nucléotidique qui est situé à environ 40 à 50 pb des bouts étant à l'opposé de ceux générés par la digestion par *Xbal* et *Sacl*.

La digestion par Xbal et Sacl de la molécule d'ADN intermédiaire est effectuée simultanément et pendant 7 à 8 heures à 37°C dans un volume total de 80 µl de solution aqueuse dont les conditions ioniques sont fixées par l'emploi du tampon de réaction « *CutSmart* » selon les recommandations du fabriquant (New England Biolabs). La réaction comprend 6 µg de la molécule d'ADN « *intermédiaire* », 100 unités de Xbal, et 50 unités de Sacl. La réaction est arrêtée en inactivant les enzymes de restriction par passage sur colonne de purification du kit « *Nucleospin Gel and PCR Cleanup* » (Macherey-Nagel).

### ÉTAPE B) : SYNTHESE ET ASSEMBLAGE DES EXTREMITES FONCTIONNALISEES DESTINEES AUX SUPPORTS

Un premier fragment d'ADN double-brin de longueur d'environ 2 300 paires de bases est synthétisé par PCR en incorporant des nucléotides modifiés par la biotine, molécule complémentaire de la streptavidine qui recouvre la bille magnétique faisant office de support. Les oligonucléotides utilisés pour amplifier ce premier fragment (ayant la séquence de SEQ ID NO : 1, amplifié à partir du génome du phage λ) ont les séquences 5'-GCGTATTAGCGACCCATCGTCTTTCTG-3' et 5'-GATGCACGCAATGGTGTAGCAATAATTGC-3', respectivement, correspondant aux séquences de SEQ ID NO : 2 et 3). Plus précisément, la réaction PCR est effectuée dans un volume final de 100 µl de solution aqueuse, distribués en deux tubes de 50 µl chacun. Ces 100 µl incluent 50 ng de matrice (le génome du bactériophage λ), 30 pmol des oligonucléotides d'amorçage, 10 nmol de chaque désoxyribonucléotide triphosphate, 3.5 unités du mélange de polymérases thermostables « *Expand High Fidelity* » (Roche), 5 nmol de biotin-16-dUTP (Roche), et le tampon réactionnel fourni par le fabriquant (Roche) à une concentration finale de 1x selon le protocole du fournisseur (Roche). Le programme PCR employé consiste en une première étape de 2 min à 95°C, suivi par une seconde étape de 15 s à 95°C, suivi par une troisième étape de 30 s à 56°C, suivi par une quatrième étape de 80 s à 72°C, suivi par 25 répétitions de la seconde, troisième, et quatrième étape en succession. A la fin du programme une dernière étape de 3 min à 72°C est effectuée, puis l'échantillon est ramené à 16 °C. Le produit de la réaction est ensuite purifié avec un kit « *PCR Cleanup* » (Macherey- Nagel).

Ce premier fragment est ensuite digéré par Sacl afin de générer deux fragments d'environ 1 300 pb et d'environ 950 pb possédant chacun un bout cohésif complémentaire de celui de la molécule (1). Plus précisément, le produit PCR est tout d'abord quantifié par spectrophotométrie UV. La digestion est ensuite effectuée pendant 2 h à 37°C dans un volume réactionnel de 50 µl de solution aqueuse comprenant 5 µg d'ADN marqué, 100 unités de Sacl (New England Biolabs), et le tampon réactionnel « *CutSmart* » fourni par le fabriquant (New England Biolabs) à une concentration finale de 1x selon le protocole du fournisseur. Les fragments obtenus peuvent ensuite être purifiés par électrophorèse sur gel d'agarose et extraits du gel avec un kit « *Nucleospin Gel and PCR Cleanup* » (Macherey-Nagel). Alternativement, les fragments peuvent également être purifiés simplement avec un kit « *Nucleospin Gel and PCR Cleanup* » (Macherey-Nagel). Les fragments digérés et purifiés sont finalement quantifiés par spectrophotométrie UV.

Un second fragment d'ADN double-brin de longueur d'environ 2 300 paires de bases est synthétisé par PCR en incorporant des nucléotides modifiés par la digoxigénine, molécule complémentaire de l'anticorps qui tapissera la lamelle de verre et qui fera office de second support. Le protocole suivit pour cette PCR est le même que ci-dessus, utilisant les mêmes oligonucléotides (selon les séquences de SEQ ID NO : 2 et 3) amplifiant le même fragment du génome du phage λ, le biotin-16-dUTP étant juste remplacé par la forme stable en conditions alcalines du digoxigénine-11-dUTP (Roche).

Ce second fragment d'ADN double-brin est ensuite digéré par *Xbal* afin de préparer deux fragments d'environ 1 050 pb et d'environ 1 200 pb portant chacun un bout cohésif complémentaire de celui de la molécule (2). Plus précisément, le produit PCR est tout d'abord quantifié par spectrophotométrie. La digestion est ensuite effectuée pendant 2 h à 37°C dans un volume réactionnel de 50 µl de solution aqueuse comprenant 5 µg d'ADN marqué, 250 unités de Xbal (New England Biolabs), et le tampon réactionnel « *CutSmart* » fourni par le fabriquant (New England Biolabs) à une concentration finale de 1x selon le protocole du fournisseur. Les fragments obtenus peuvent ensuite être purifiés par électrophorèse sur gel et extraits du gel avec un kit « *Nucleospin Gel and PCR Cleanup* » (Macherey Nagel). Alternativement, les fragments peuvent également être purifiés simplement avec un kit « *Nucleospin Gel and PCR Cleanup* » (Macherey-Nagel). Les fragments digérés et purifiés sont quantifiés par spectrophotométrie UV.

Le substrat utilisé pour les réactions PCR est ici le génome du phage λ, sans que cela n'ait aucun caractère obligatoire. La longueur des extrémités fonctionnalisées peut varier et aller jusqu'à 10 000 pb mais la valeur d'environ 1 000 pb est un bon compromis entre rendement de production et efficacité d'attachement des molécules d'ADN selon l'invention sous microscope. On peut aussi imaginer des fragments d'ADN plus courts et/ou portant une petite molécule réactive unique à leur extrémité. Finalement, dans d'autres variantes possibles, des groupes fonctionnels autres que la biotine et la digoxigénine peuvent être employés, par exemple des thiols qui se fixeront sur une surface couverte d'or.

La synthèse de la molécule d'ADN double-brin est finalisée par la ligation des fragments marqués à la biotine et à la digoxigénine aux molécules d'ADN (1) et (2), respectivement à l'aide de l'ADN ligase du phage T4 (**Figure 4B**).

Plus précisément, une réaction de ligature dans un volume total de 10 µl a été conduite dans les conditions recommandées par le fabricant (tampon ADN T4 DNA Ligase 1x de New England Biolabs), la molécule d'ADN était à une concentration de 3 nM, les fragments marqués à la biotine et à la digoxigénine étaient chacun à 9 nM ; 200 unités (0,5 µl) d'ADN ligase T4 ont été utilisées pour ligaturer l'ADN pendant 3 heures à température ambiante avant d'inactiver thermiquement la ligase.

Des molécules d'ADN double-brin (1) et (2) ayant les séquences selon le SEQ ID NOs indiquées dans le Tableau 1 ci-dessus sont obtenues.

### EXEMPLE 2 : AMENAGEMENT DES EXTREMITES DESTINEES AUX MOLECULES A TESTER

Les séquences des extrémités ont été choisies de façon à pouvoir générer, par digestion enzymatique, des bouts cohésifs non-complémentaires permettant la liaison par hybridation spécifique puis ligature de deux conjugués oligonucléotide - molécule à tester (voir **Figure** 5).

### Étape A) : Digestion des extrémités

L'enzyme *Nb.BbvCl* a été utilisé pour la digestion des extrémités (**Figure 5A**). Cette enzyme est dite de « *nicking* » car elle ne clive que l'un des deux brins de la double hélice, ce qui permet de générer des bouts cohésifs relativement longs. De plus, les bouts cohésifs formés peuvent posséder toute séquence voulue par le concepteur, dans la limite de quelques bases immuables au niveau du site de reconnaissance. Ceci est à mettre en regard avec les enzymes de restriction classiques avec lesquelles on ne dégage souvent que quatre nucléotides de séquence fixe. Dans le cas présent on génère des bouts 5'-débordants possédant 9 nucléotides. Cette valeur de 9 nucléotides est avantageuse car d'une part assez grande pour permettre un adressage stable et spécifique de chacune des extrémités par reconnaissance moléculaire (les séquences produites ne sont bien-sûr ni identiques, ni complémentaires) et d'autre part assez courte pour éviter tout problème de repliement/hybridation parasite. Cette étape de digestion éventuellement peut être effectuée en même temps que l'étape A de l'Exemple 1.

Plus précisément, la digestion par Nb.BbvCI de la molécule d'ADN intermédiaire est effectuée simultanément à la digestion par Xbal et Sacl de la molécule d'ADN intermédiaire, pendant 7 à 8 heures à 37°C dans un volume total de 80 µl de solution aqueuse dont les conditions ioniques sont fixées par l'emploi du tampon de réaction « *CutSmart* » selon les recommandations du fabriquant (New England Biolabs). La réaction comprend 6 µg de la molécule d'ADN « *intermédiaire* »*,* et, en plus des 100 unités de Xbal, et 50 unités de Sacl employées, 30 unités de Nb.BbvCI (New England Biolabs). La réaction est arrêtée en inactivant les enzymes de restriction par passage sur colonne de purification du kit « *Nucleospin Gel and PCR Cleanup* » (Macherey-Nagel). L'élimination des fragments complémentaires après digestion est relativement aisée, elle s'effectue par purification sur gel d'électrophorèse 1 % à 37°C pendant toute une nuit.

### Étape B) : Liaison des molécules à tester aux extrémités

Les molécules à tester, ici les protéines FRB et FKBP12, sont conjugués à deux oligonucléotides appelés oligo-T^{C}. Chacun de ces derniers possède à son extrémité 3' une séquence complémentaire de l'une de celles présentes aux bouts cohésifs non-complémentaires des extrémités des molécules d'ADN (1) et (2). La conjugaison des protéines aux oligonucléotides a été réalisée par chimie click entre un groupe fonctionnel DBCO porté à l'extrémité 5' des oligo-T^{C} et un groupe fonctionnel azide introduit dans la séquence des protéines par mutagénèse et incorporation d'un acide aminé artificiel, l'azido-phénylalanine, en cours d'expression. La protéine FRB ayant la séquence de SEQ ID NO : 6, et comprenant un group azide à la position 2 020 par substitution de l'acide aminé alanine par la 4-azido-L-phenylalanine, est conjuguée à Oligo-T^{C}₁, ayant la séquence 5'-TATATGAGGC-3'de SEQ ID NO : 4. La protéine FKBP12 ayant la séquence de SEQ ID NO : 7, comprenant un groupe azide à la position 1 par substitution de l'acide aminé méthionine par la 4-azido-L-phenylalanine, est conjuguée à Oligo-T^{C}₂, ayant la séquence 5'-TTGTAAGAGC-3'de SEQ ID NO : 5. La première base « T » en 5' des oligos-T^{C} correspond à DBCO-DT. D'autres systèmes de conjugaison entre oligonucléotides et protéines sont bien entendus possibles : les premiers peuvent ainsi porter, par exemple, un groupe fonctionnel maléimide, carboxylique, ou benzylguanine qui réagira respectivement avec un groupe fonctionnel cystéine, amine, ou SNAP-tag localisé sur les secondes. Ces groupes fonctionnels peuvent aussi être localisés à l'extrémité 3' des oligo-T^{C} à condition de revoir l'aménagement des extrémités pour que l'hybridation des oligonucléotides reste possible. L'exemple 4 ci-dessous décrit une méthode de liaison des molécules à tester aux extrémités utilisant un tel système de conjugaison sans avoir recours à une enzyme de « *nicking* ».

Dans la présente réalisation, l'oligonucléotide est directement lié à la molécule à tester. Il est cependant envisageable de relier les deux espèces au travers d'un espaceur de type biopolymère (e.g. ADN double-brin, ADN simple-brin, ARN, polypeptide, protéine), polymère organique (e.g. PEG) ou même colloïde (e.g. nanoparticule d'or, quantum dot). Les extrémités pourront ainsi être rallongées et la nature de l'interaction pourra être modulée (e.g. élimination de comportement non-spécifique, modification de l'environnement électrostatique).

La dernière étape de la synthèse du dispositif consiste à hybrider les conjugués « *oligonucléotide* - *protéine* » aux bouts cohésifs des extrémités et à effectuer une ligature grâce à l'ADN ligase du phage T4 (voir aussi la **Figure 5B**). Cette ligation est effectuée dans un volume total de 50 µl de solution aqueuse pendant 5 h à 16°C et comprend : 2.5 fmol de la construction d'ADN selon l'invention dont les extrémités sont déjà marquées à la biotine et à la digoxigénine, 2.5 fmoles du complexe nucléoprotéique correspondant à la protéine FKBP12 couplée à Oligo-TC1, et 2.5 fmoles du complexe du complexe nucléoprotéique correspondant à la protéine FRB couplée à Oligo-TC2. La solution aqueuse contient en outre du DTT (2 mM), de l'ATP (1 mM) et 200 unités de T4 DNA ligase (New England Biolabs). La solution aqueuse contient également un agent tamponnant (20 mM de Tris, pH 7.5) et du sel (300 mM NaCl). Aucune étape d'inactivation thermique ou de purification n'est appliquée par la suite.

### EXEMPLE 3 : PROCEDE DE PREPARATION DE LA MOLECULE D'ADN DOUBLE-BRIN « INTERMEDIAIRE »

### Étape A) : Synthèse des jonctions

Cette étape consiste à préparer, par couplage covalent d'oligonucléotides, deux structures en Y (aussi appelées « *jonctions* ») qui feront le lien entre les molécules d'ADN (1) ou (2) et la longe. Plus précisément, pour chaque jonction, un groupe fonctionnel porté par une des extrémités d'un premier oligonucléotide appelé oligo-L est mis à réagir avec un groupe fonctionnel présent au milieu d'un second oligonucléotide appelé oligo-TS (**Figure 6A**). Plus particulièrement, dans le cas présent, un groupe fonctionnel DBCO situé à l'extrémité 5' de l'oligo-L et une fonction azide située au milieu de l'oligo-TS ont été utilisées.

Des fonctions alternatives peuvent cependant être utilisées. Par exemple, l'extrémité 5' réactive de l'oligo-L peut consister en un groupe de type maléimide, auquel cas l'oligo-TS doit porter une modification de type thiol en son milieu. On peut également employer un groupe NHS en 5' de l'oligo-L et un groupe NH₂ au milieu de l'oligo-TS. D'autres chimies pourront aussi être trouvées ou développées à l'avenir, qui seront compatibles avec cette procédure d'assemblage. De plus, pour chaque chimie, un certain nombre de variations sont envisageables : échanger le groupe fonctionnel en 5' de l'oligo-L avec le groupe fonctionnel situé au milieu de l'oligo-TS, positionner en 3' le groupe fonctionnel porté par l'oligo-L, positionner ailleurs que sur une base le groupe fonctionnel intégré au milieu de l'oligo-TS (e.g. sur le squelette phosphate, sur un sucre, sur un espaceur, etc.). Dans nos réalisations les deux jonctions sont de même nature fonctionnelle. Cependant, les deux jonctions peuvent être de nature fonctionnelle différente, par exemple, selon l'une des variantes qui viennent d'être énumérées.

Les séquences des quatre oligonucléotides, i.e. celles des deux oligo-L et des deux oligo-TS ne sont ni identiques, ni complémentaires, afin de pouvoir s'hybrider de façon spécifique. Le nombre de bases de chaque oligo-L doit juste permettre la suite du protocole, il est typiquement égal à 20 à 30 bases. Il en va de même pour le nombre de bases séparant le groupe fonctionnel de l'extrémité 3' de chaque oligo-TS, sa valeur est typiquement égale à 15 à 20 bases. Par contre, dans le présent exemple, le nombre de bases séparant le groupe fonctionnel de l'extrémité 5' de chaque oligo-TS conditionne la longueur des extrémités. Ici, la longueur de l'extrémité est égale à 30 à 60 bases, mais les capacités en synthèse oligonucléotidique permettent d'atteindre des valeurs autour de 150 bases.

Finalement, la présente étape peut correspondre à l'initiation de la mise en place des groupes fonctionnels ou des séquences (e.g. des sites de restriction) destinées à la liaison spécifique des molécules à tester aux extrémités. Plus précisément deux modes de liaison principales sont envisagés : soit par hybridation d'un oligonucléotide tel que détaillé dans l'Exemple 2 **(****Figure 5**), soit par réaction chimique entre groupes fonctionnels spécifiques tel que détaillé dans l'Exemple 4 **(****Figure 7**). Dans le premier cas on choisira judicieusement les séquences afin de pouvoir générer, le moment venu, les bouts cohésifs ad hoc, i.e. capables de réagir de façon orthogonale. Dans le second cas on choisira judicieusement les groupes fonctionnels afin de pouvoir attacher, le moment venu, les molécules à tester de façon orthogonale.

Plus précisément, dans le cas présent, les oligonucléotides oligo-L₁ et oligo-L₂, où la première thymine en 5' est une base DBCO-dT, et les oligonucléotides oligo-TS, et oligo-TS₂, comprenant une base Azido-dT au milieu de l'oligonucléotide, tels que définis ci-dessous dans le Tableau 2, ont été remis en suspension à 10 µg/µl dans du formamide à 37°C. 50 µg de chacun des oligonucléotides Oligo-L₁ et Oligo-TS, ont été combinés. De même, 50 µg chacun de Oligo-L₂ et Oligo-TS₂ ont été combinés. Comme indiqué dans la Tableau 2, les séquences des oligonucléotides qui sont combinés diffèrent lorsque la molécule « *intermédiaire* » est utilisée en aval pour la réalisation des Exemples 8 et 9 ou pour la réalisation des Exemples 10 ou encore 9. Les oligonucléotides combinés ont été incubés 8 h à 37°C et les produits correspondant aux oligonucléotides couplés par de façon covalente ont été séparés sur un gel à 8 % acrylamide: bisacrylamide (29: 1) contenant 8 M d'urée fonctionnant à environ 10V / cm dans du tampon Tris-borate-EDTA. Des tranches de gel correspondant au produit ont été excisées du gel, en utilisant une voie d'imagerie sacrificielle pour effectuer l'ombrage UV permettant d'identifier les bandes pertinentes. L'ADN a été purifié en éluant d'abord l'ADN à partir du fragment de gel broyé pendant une nuit dans un tampon phosphate 10 mM pH 8,0 avec agitation à 4°C. Une cartouche SepPak C18 (Waters) a été conditionnée avec de l'acétonitrile puis de l'eau ; l'éluât a été chargé sur la cartouche, lavé avec de l'eau, et l'oligonucléotide a été libéré de la cartouche en éluant avec de l'acétonitrile. L'acétonitrile a été évaporé par centrifugation sous vide et l'oligonucléotide a été remis en suspension à environ 10 µM.

**Tableau 2 : Oligonucléotides utilisées pour synthétiser les jonctions**

| **Oligonucléotide** | **Séquence*** | **SEQ ID NO** | **Exemple(s)** |
|---|---|---|---|
| Oligo-TS₁ | 5’-GAGAGACCCGGGCACGACTTATCGCCACTGG CAGCAGCCACTGGTAACAGGATTAGCAGAGC-3’ | 11 | 8 (longe de 0,7 et 6 kbp) et 9 |
| Oligo-L₁ | 5'-TGAGCCAAGACGCCTCCATCCATGCA-3' | 12 | 8 (longe de 0,7 et 6 kbp) et 9 |
| Oligo-TS₂ | 5'-GAGAGACCCGGGCACCGTCTCCTTCGAACTTA TTCGCAATGGAGTGTCATTCATCAAGGACG-3' | 13 | 8 (longe de 0,7 et 6 kbp) et 9 |
| Oligo-L₂ | 5'-TCCATGGGCATACTGATCGGTAGGG-3' | 14 | 8 (longe de 0,7 et 6 kbp) et 9 |
| Oligo-TS₁ | 5'-ATATGAGGCTGAGGAAGCGGTTAGCTCCTTC GGTCCTCCGATCGTTGTCAGAAGTAAGTTGGCC GCAG-3' | 15 | 10 |
| Oligo-L₁ | 5'-TCCATGGGCATACTGATCGGTAGGG-3' | 16 | 10, 12 |
| Oligo-TS₂ | 5'-TGTAAGAGCTGAGGCGGTGACCAATATCTACA ACATCAGCCTTGGTATCCAGCGTGATG-3' | 17 | 10 |
| Oligo-L₂ | 5'-*T*GAGCCAAGACGCCTCCATCCATGCA-3' | 18 | 10, 12 |
| Oligo-TS₁ | 5'-ZGTAAGAGCTGAGGCGGTGACCAATATCTACA ACATCAGCCTTGGTATCCAGCGTGATG-3' | 39 | 12 |
| Oligo-TS₂ | 5'-QTCTCAAGCTGAGGAAGCGGTTAGCTCCTTC GGTCCTCCGATCGTTGTCAGAAGTAAGTTGGCC GCAG-3' | 40 | 12 |

| | | | |
|---|---|---|---|
| * la première base « T » indiquée en gras et italique en 5' des oligos-L correspond à DBCO-DT (Glen Research, USA and Trilink, USA) ; la base « T » indiquée en gras et soulignée dans les oligos-TS correspond à Azido-dT (Trilink, USA). Z correspond à O²-benzylcytosine-dT (New England BiolabslTrilink), et Q correspond à O⁶-benzylguanine-dA (New England Biolabs/Trilink). | | | |

### Étape B) : Synthèse de la molécule (A) - le précurseur des molécules d'ADN (1) et (2)

Les deux structures en Y obtenues ci-dessus sont utilisées comme amorces dans une réaction PCR destinée à générer une molécule ADN double-brin qui sera transformé en molécules d'ADN (1) et (2) plus tard (**Figure 6B**). Plus précisément, les séquences à l'extrémité 3' des deux oligo-TS vont d'hybrider sur le substrat et servir à initier la polymérisation. Les deux oligo-L n'interviennent pas et ne sont pas modifiés par la réaction PCR. L'ADN double-brin produit possède donc à chaque extrémité une jonction aboutissant d'une part à un oligo-L et d'autre part à une extrémité dont la fonctionnalisation par une séquence ou groupe fonctionnel spécifique a été initiée. La séquence de cet ADN double-brin peut être choisie par sélection du substrat : le vecteur de clonage Charomid 9-5 ΔSbfl (un dérivé de plasmide Charomid 9-5 à partir duquel le site Sbfl naturel a été éliminé par digestion Sbfl, remplissage et re-ligature, ayant la séquence de SEQ ID NO : 8) a été retenu ici. De plus la longueur du produit est déterminée par la position des amorces sur le substrat : elle est d'environ 3 050 paires de bases dans le cas des Exemple 8 et 9, d'environ 2 100 paires de bases dans le cas des Exemples 10 et 12. Ce chiffre peut en fait aller jusqu'à environ 10 000 paires de bases, limite au-delà de laquelle le rendement des réactions PCR chute.

La réaction PCR est assemblée en combinant 1,25 µg d'ADN matrice (Charomid 9-5 ΔSbfl ; SEQ ID NO : 8), 90 pmoles de l'oligonucléotide en structure « Y » obtenu en couplant Oligo-TS, (SEQ ID NO: 11) et Oligo-L₁ (SEQ ID NO : 12) , 90 pmoles de l'oligonucléotide en structure « Y » obtenu en couplant Oligo-TS₂ (SEQ ID NO : 13) et Oligo-L₂ (SEQ ID NO : 14), 125 nmoles de chacun des quatre désoxyribonucléotides triphosphates, 5 µl de DMSO, 17,5 unités de mélange de polymérases thermostables « *Expand High Fidelity PCR System* » (Roche), 25 µl du tampon de réaction fourni par la fabriquant (Roche), et de l'eau ultrapure afin d'atteindre un volume total de 250 µl. Ce volume est divisé en dix tubes, chacun contenant 25 µl. Ces dix tubes sont soumis à des cycles thermiques selon le protocole qui suit. Les tubes sont chauffés pendant 2 minutes à 94°C. Ensuite, les tubes sont soumis à 8 cycles thermiques, chaque cycle comprenant une étape de 15 secondes à 94°C, suivie d'une étape de 30 secondes à 72°C, suivie d'une étape de 4 minutes à 68°C. À chaque itération de ces 8 cycles la température utilisée pour l'étape de 30 secondes est diminuée de 2°C par rapport à l'itération précédente. Ensuite, les tubes sont soumis à 22 cycles thermiques, chaque cycle comprenant une étape de 15 secondes à 94°C, suivie d'une étape de 30 secondes à 56°C, suivie d'une étape de 4 minutes à 68°C. Ensuite, les tubes sont maintenus pendant 7 minutes à 72°C avant que leur température ne soit ramenée à 16°C afin de terminer la réaction. La molécule d'ADN ainsi produite a ensuite été purifiée par extraction sur colonne à l'aide du kit « *Nucleospin Gel and PCR Cleanup* » (Macherey-Nagel).

### Étape C) : Synthèse et assemblage de la molécule (B), correspondant à la longe

La longe est produite par PCR puis digestion par deux enzymes de restriction du double-brin obtenu, ceci afin de générer deux extrémités cohésives différentes **(****Figure 6D**). Sans raison impérative, c'est le vecteur de clonage Charomid 9-5 ΔSbfl, ayant la séquence de SEQ ID NO : 8, qui a encore une fois été utilisé comme substrat pour l'amplification.

Plus précisément, dans le cas présent la longe (ayant la séquence de SEQ ID NO : 10) a été obtenue par amplification PCR de la matrice Charomid C9-5 ΔSbfl avec les oligonucléotides 5'-GAGAGAACGCGTTACCTGTCCGCCTTTCTCCCTTCGGG (SEQ ID NO : 19) et 5'-GAGAGACCTGCAGGCCTCACTGATTAAGCATTGGTAACTGTCAGACC (SEQ ID NO : 20) (afin de générer un fragment ayant la séquence de SEQ ID NO : 25), digestion par Mlul et Sbfl, et purification par électrophorèse sur gel d'agarose puis extraction sur colonne à l'aide du kit « *Nucleospin Gel and PCR Cleanup* » (Macherey-Nagel). Ces étapes sont réalisées selon les protocoles standards de biologie moléculaire, en suivant les instructions des fabricants.

La longe fabriquée a ici une longueur d'environ 700 paires de bases mais le procédé PCR permet d'aller jusqu'à des valeurs de 10 000 paires de bases si cela est souhaitable. Pour atteindre des longueurs encore plus grandes on pourra avoir recours à de l'ADN « *naturel* » tel que décrit ici. À titre d'exemple, le génome du phage λ fait 48 502 paires de bases.

À titre d'exemple particulier, une longe ayant une longueur d'environ 6 kbp peut être fabriqué par amplification PCR de la matrice phage λ avec des oligonucléotides ayant la séquence de SEQ ID NO 41 et 42 (5'-GAGAGAACGCGTTCCGGATGCGGAGTCTTATCCGTGGAAATC et 5'-GAGAGACCTGCAGGACCAGAGCGGAGATAATCGCGGTGACTCTG, respectivement). Le produit amplifié, ici à bout-francs, est ensuite cloné dans le vecteur pUC18 en utilisant le site de restriction Smal. Le plasmide est introduit dans des cellules *E. coli* et une culture sur un volume de 250 mL est réalisée selon des techniques bien connu dans l'art. Le plasmide est purifié en utilisant le NucleoBond Xtra maxiprep kit (Macherey-Nagel) en suivant les instructions du fabricant puis digéré avec les enzymes de restrictions Mlul et Sbfl. Les produits de digestion sont séparés par électrophorèse sur gel d'agarose et la bande d'intérêt (correspondant au fragment d'environ 6 kbp ayant la séquence de SEQ ID NO : 46 qui sera utilisée en tant que longe) est récupérée. Plus précisément, la bande d'intérêt est découpé du gel d'agarose et purifié sur colonne à l'aide du kit « *Nucleospin Gel and PCR Cleanup* » (Macherey-Nagel) en suivant les instructions du fabricant. Le passage par une étape de clonage permet, avantageusement d'amplifier la matrice dans les bactéries. De plus, lorsque l'ADN est purifié et les fragments digérés ensuite séparés par électrophorèse sur gel d'agarose, des bandes correspondant aux fragments d'ADN sont très bien séparés sur gel, permettant avantageusement d'isoler la longe avec efficacité. Aussi, selon un mode préférentiel, la longe est obtenue par les étapes suivantes : a) insertion d'un fragment d'ADN dans un vecteur b) introduction dudit vecteur dans un cellule hôte, par exemple un bactérie c) culture du cellule hôte permettant avantageusement d'amplifier le nombre de copies du vecteur d) purification dudit vecteur du cellule hôte, et e) isolation dudit fragment d'ADN dudit vecteur. Avantageusement, le vecteur est un vecteur plasmidique, de préférence à nombre de copies élevé.

Les étapes suivantes sont les mêmes, qu'elle que soit la taille de la longe.

### Étape D) : Assemblage de la molécule « intermédiaire »

Deux oligonucléotides appelés oligo-L^{C} sont hybridés sur les oligo-L de la molécule afin de générer des bouts cohésifs complémentaires de ceux flanquant la longe (**Figure 6C**). Une réaction de ligature permet ensuite d'assembler la molécule (B) correspondant à la longe avec la molécule (A) et d'obtenir une molécule d'ADN « *intermédiaire* » (**Figure 6D**).

Afin d'atteindre un rendement non négligeable pour la formation de la molécule d'ADN « *intermédiaire* » (8,5 %), il est avantageux de travailler avec quatre sites de restriction et d'effectuer la ligature, par exemple par l'ADN ligase du phage T4, en présence des quatre enzymes correspondantes. L'utilisation de deux sites de restriction différents, présents à la fois au niveau des jonctions et au niveau des extrémités de la longe permet de limiter la formation de sous-produits issus de réactions de ligation nonsouhaitées comme décrit plus bas. En effet, il est difficile d'ajuster les concentrations afin de diminuer la quantité de sous-produits issus de réactions entre molécules (B) ou entre molécules (A) lorsque seulement deux sites de restriction différents, présents à la fois au niveau des jonctions et au niveau des extrémités de la longe, sont utilisés.

Plus précisément, une des extrémités de la longe est digéré par *Sbfl,* ce qui crée un bout cohésif 3'-débordant de séquence 5'-TGCA-3' précédée des bases GG. Les séquences des oligo-L₂ et oligo-L^{C}₂ sont choisies de façon à obtenir un bout cohésif 3'-débordant de séquence 5'-TGCA-3' précédé de la base A, ce qui correspond au site de restriction de *Nsil.* Lors de la ligature, il est donc possible de former des dimères molécule (B) - molécule (B), molécule (A) - molécule (A) et molécule (B) -molécule (A) car les deux types d'extrémités cohésives peuvent s'apparier entre elles. Cependant, si les enzymes Sbfl et Nsil sont ajoutés au mélange réactionnel les homodimères sont digérés en permanence et les monomères recyclés. Seuls les hétérodimères sont stables et s'accumulent car le site de ligature possède alors une séquence qui ne peut être reconnue par aucune des deux enzymes, i.e. 5'-ATGCAGG-3'. Une stratégie similaire est mise en place à l'autre extrémité de la longe et au niveau de la jonction portant le duplex issu de l'hybridation entre oligo-L₁ et oligo-L^{C}₁ ; ce sont cette fois-ci les enzymes Mlul et Ascl qui sont impliquées.

Plus précisément, les oligonucléotides oligo-L₁ et oligo-L₂ (séquences indiquées ci-dessus, dans la Tableau 2, en fonction du mode de réalisation), intégrés à la molécule (A) selon les étapes A et B, ont été hybridés, respectivement, aux oligonucléotides d'ADN simple-brin oligo-L^{C}₁et oligo-L^{C}₂, en rapport équimolaire à une concentration finale d'environ 300 nM chacun pendant une heure à température ambiante dans du tampon « *SureCut* » 1x (New England Biolabs). Comme illustré dans le Tableau 3, ci-dessous, les séquences des oligonucléotides diffèrent lorsque la molécule « *intermédiaire* » est utilisée en aval pour la réalisation des Exemple 8 et 9 ou pour la réalisation de l'Exemple 10.

**Tableau 3 : Oligonucléotides utilisées pour l'assemblage de la molécule « intermédiaire »**

| **Oligonucléotide** | **Séquence** | **SEQ ID NO** | **Exemple(s)** |
|---|---|---|---|
| Oligo-L^{C}₁ | 5'-TGGATGGAGGCGTCTTGGCTCA-3' | 21 | 8 (longe de 0,7 et 6 kbp) et 9 |
| Oligo-L^{C}₂ | 5'-CGCGCCCTACCGATCAGTATGCCCATGGA-3' | 22 | 8 (longe de 0,7 et 6 kbp) et 9 |
| Oligo-L^{C}₁ | 5'-CGCGCCCTACCGATCAGTATGCCCATGGA-3' | 23 | 10,12 |
| Oligo-L^{C}₂ | 5'-TGGATGGAGGCGTCTTGGCTCA-3' | 24 | 10, 12 |

Ensuite, les molécules d'ADN (A) et (B) préparées comme ci-dessus ont été combinées à une concentration finale d'environ 130 nM chacune dans 30 µl de tampon « *SureCut* » 1x (New England Biolabs), avec 0,5 µl chacun de Sbfl-HF (soit 10 unités), Mlul-HF (soit 10 unités), Nsil-HF (soit 10 unités) et Ascl (soit 5 unités), ADN-ligase HC-T4 (soit 1 000 unités) (New England Biolabs) et 1 mM d'ATP et 2 mM de DTT, et laissés pendant une nuit à température ambiante. La réaction a été arrêtée par inactivation pendant 20 minutes à 65°C.

### EXEMPLE 4 : METHODE ALTERNATIVE DE LIAISON DES MOLECULES A TESTER AUX EXTREMITES

Cette méthode alternative est illustrée dans la Figure 7, et utilise un système de conjugaison entre acides nucléiques et protéines. Dans ce système, les acides nucléiques peuvent être conjugués, par exemple, à une molécule de benzylcytosine (BC) ou de benzylguanine (BG) qui réagira respectivement avec une protéine d'étiquetage CLIP ou SNAP fusionnée avec les protéines. Ladite molécule BC ou BG est liée à une extrémité de l'acide nucléique. Deux exemples sont fournis ci-dessous pour les étapes B et C, portant sur les molécules à tester FRB et FKP12, ou sur la boucle β du récepteur de la glycine (aussi appelé le « *PHLoop* ») et la géphyrine, respectivement.

### Étape A) : Préparation de la molécule d'ADN

La synthèse de la molécule d'ADN pour cette stratégie est la même que précédemment utilisées pour l'Exemple 10 à l'exception de quelques modifications. Premièrement les amorces TS₁ et TS₂ ont maintenant les séquences selon SEQ ID NOs : 39 et 40, respectivement (voir Tableau 2 ci-dessus). Deuxièmement, l'étape de « nicking » par l'enzyme Nb.BbvCI est omise. Troisièmement, l'assemblage des molécules à tester sur la molécule d'ADN ne nécessite plus de ligature mais une simple mise en présence.

### Étape B) : Préparation des molécules à tester

Les molécules à tester sont liées sont fusionnées avec les protéines d'étiquetage CLIP et SNAP, respectivement, comme décrit ci-après.

### FRB et FKBP12 du complexe protéique mTOR

FRB et FKBP12 ont été clonés dans un vecteur pGEX6P-1 dont l'étiquette GST a été respectivement remplacée par les étiquettes SNAP et CLIP respectivement, ceci en faisant appel à une ingénierie basée sur les sites de restriction Ncol et BamHI. Les protéines comportent donc en N-terminal une étiquette 10xHis suivie par une étiquette SNAP ou CLIP puis un site de clivage « *PreScission* » et ont les séquences selon SEQ ID NOs : 35 et 36, respectivement. Les constructions génétiques ont ensuite été introduites dans des cellules *E. coli* BL21 (Invitrogen). Ces cellules ont été cultivées en milieu LB (10 g/l NaCl) additionné de 200 µg/mL ampicilline, ceci jusqu'à atteindre une densité optique à 600 nm compris entre 0,9 et 1,0. De l'IPTG a ensuite été ajouté à une concentration finale de 0,1 mM. La culture est ensuite poursuite sous agitation vigoureuse afin d'obtenir l'expression des protéines, toute la nuit à 20°C pour 10xHis-SNAP-FRB et 4 h à 30°C pour 10xHis-CLIP-FKBP12.

Les protéines 10xHis-SNAP-FRB et 10xHis-CLIP-FKBP12 (ayant la séquence selon SEQ ID NO 35 et 36, respectivement) ont ensuite été purifiées comme il suit. Les cellules BL21 ont été resuspendues dans le tampon de lyse (25 mM Tris-HCl, pH 8,0, 250 mM NaCl, 25 mM imidazole et de l'inhibiteur de protéases (Roche)) et lysées avec un Emulsiflex C5 (Avestin). Le lysat a été clarifié par centrifugation et chargé sur une colonne HisTrap (5mL, GE Healthcare) pré-équilibrée avec le tampon d'accroche (25 mM Tris-HCl, pH 8,0, 250 mM NaCl, 25 mM imidazole) et maintenue à 4°C. La résine a ensuite été lavée avec le tampon d'accroche contenant 50 mM d'imidazole puis la protéine éluée avec le tampon d'accroche contenant 350 mM d'imidazole. La protéine a été dialysée toute la nuit dans un tampon contenant 50 mM Tris-HCl, pH 7,5 et 300 mM NaCl, concentrée, puis purifiée à nouveau sur une colonne Superdex 75 16/600 (« *preparative grade* », GE Healthcare) équilibrée avec ce même tampon. Les échantillons ont finalement été analysés sur gel 4-20% SDS-PAGE, concentrés, et stockés à -80°C. Les concentrations en protéines ont été déterminées en utilisant un test Pierce Coomassie Plus (Bradford) (ThermoFisher Scientific).

### Boucle β du récepteur de la glycine et la géphyrine

La boucle 6 du récepteur de la glycine (aussi appelé ici le « *PHloop* » ; Bedet et al., 2006) ayant la séquence de SEQ ID NO : 44 a été clonée dans un vecteur pGEX6P-1 dont l'étiquette GST a été remplacée par une étiquette CLIP, ceci en faisant appel à une ingénierie basée sur les sites de restriction Ncol et BamHI. La protéine (SEQ ID NO : 37) comporte donc en N-terminal une étiquette 10xHis suivie par une étiquette CLIP puis un site de clivage PreScission. La construction génétique a ensuite été introduite dans des cellules *E. coli* BL21 (Invitrogen). Ces cellules ont été cultivées en milieu LB additionné de 200 µg/mL ampicilline, ceci jusqu'à atteindre une densité optique à 600 nm de 0,7. De l'IPTG a ensuite été ajouté à une concentration finale de 0,1 mM. La culture est ensuite poursuite afin d'obtenir l'expression des protéines pendant 5 h à 25°C.

La protéine CLIP-PHloop a ensuite été purifiée comme il suit. Les cellules BL21 ont été resuspendues dans le tampon de lyse (25 mM sodium phosphate buffer, pH 8,0, 250 mM NaCl, 25 mM imidazole, 12,5 mM 2-mercaptoethanol, 10% glycérol et de l'inhibiteur de protéases (Roche)) et lysées avec un Emulsiflex C5 (Avestin). Le lysat a été clarifié par centrifugation et chargé sur une colonne HisTrap (5mL, GE Healthcare) pré-équilibrée avec le tampon d'accroche (25 mM Tris-HCl, pH 8,0, 250 mM NaCl, 25 mM imidazole, 12,5 mM 2-mercaptoethanol et 10% glycérol) et maintenue à 4°C. La résine a ensuite été lavée avec le tampon d'accroche puis la protéine éluée un gradient 15 - 75 % du tampon d'accroche contenant 500 mM d'imidazole. Les fractions d'intérêt ont ensuite été réunies, concentrées, puis purifiée à nouveau sur une colonne Superdex 75 16/600 (« *preparative grade* », GE Healthcare) équilibrée avec un tampon 20 mM Tris-HCl, pH 8,0, 200 mM NaCl, 1,5 mM 2-mercaptoethanol et 10% glycérol. Les échantillons ont finalement été analysés sur gel 4-20% SDS-PAGE, concentrés, et stockés à -80°C. Les concentrations en protéines ont été déterminées en utilisant un test Pierce Coomassie Plus (Bradford) (ThermoFisher Scientific).

La séquence SNAP a été clonée dans la construction Géphyrine rC4_pQE80L (Grunewald et al., 2018) en utilisant les sites de restriction Sacl et Kpnl. La protéine (SEQ ID NO : 38) comprend en N-terminal une étiquette 6xHis-tag suivie par une site de clivage PreScission et une étiquette SNAP. La construction génétique a ensuite été introduite dans des cellules *E. coli* BL21 RIPL (Invitrogen). Ces cellules ont été cultivées en milieu LB additionné de 200 µg/mL ampicilline et de 50 µg/mL chloramphénicol, ceci jusqu'à atteindre une densité optique à 600 nm de 0,7. De l'IPTG a ensuite été ajouté à une concentration finale de 0,1 mM. La culture est ensuite poursuite afin d'obtenir l'expression des protéines pendant 4 h à 20°C. Les protéines ont été purifiées par chromatographie d'affinité et d'exclusion de taille comme décrit précédemment (Grunewald et al., 2018). Les échantillons ont finalement été analysés sur gel 4-20% SDS-PAGE, concentrés, et stockés à -80°C. Les concentrations en protéines ont été déterminées en utilisant un test Pierce Coomassie Plus (Bradford) (ThermoFisher Scientific).

### Étape C) : Accrochage des molécules à tester aux extrémités de la molécule d'ADN

Les étiquettes CLIP et SNAP réagissent respectivement avec les petites molécules BC et BG situées aux extrémités de la molécule d'ADN (voir aussi Figure 7B). Ces réactions conduisent à la formation d'une liaison covalente thioether (Keppler et al, 2003 ; Gautier et al., 2008). Typiquement, un volume réactionnel de 15 µL contient 2 µM de chaque protéine et 1 nM de molécule d'ADN dans du tampon DB (composition fournie à l'Exemple 6 et correspondant aux expériences sur FRB et FKBP12 décrite dans l'exemple 12). L'incubation a lieu toute la nuit à 25 °C. Le mélange réactionnel est ensuite dilué 20 fois dans le tampon DB, du glycérol est ajouté à une concentration finale de 10% et l'échantillon est aliquoté, congelé dans l'azote liquide puis stocké à -80 °C. La molécule d'ADN fonctionnalisée avec les molécules à tester grâce aux étiquettes protéiques peut ensuite être utilisée de la même façon que celle fonctionnalisées grâce à l'adressage oligonucléotidique de l'Exemple 2. Une telle utilisation est notamment décrite à l'Exemple 5. Plus spécifiquement, juste avant les mesures sous microscope on mélange la molécule d'ADN fonctionnalisée avec les molécules à tester à des billes magnétiques recouvertes de streptavidine. Ce mélange est ensuite injecté dans la cellule fluidique dont les parois sont couvertes d'anti-digoxygénine, et les expériences de spectroscopie de force peuvent

Dans le cas des molécules « *PHloop* » et géphyrine et à titre d'exemple, un second protocole de fonctionnalisation des extrémités est proposé. La molécule d'ADN non encore fonctionnalisée par les molécules à tester est mélangée aux billes magnétiques recouvertes de streptavidine et ce mélange est injecté dans la cellule fluidique dont les parois sont couvertes d'anti-digoxygénine. Après incubation et mise sous tension, les molécules les molécules CLIP-PHloop et SNAP-géphyrine rC4 sont ensuite injectées de façon séquentielle afin de les faire réagir avec, respectivement, les extrémités BC et BG de la molécule d'ADN: d'abord CLIP-PHloop puis après rinçage SNAP-géphyrine rC4. La concentration en chaque molécule est de 500 nM dans du tampon GB (composition fournie à l'Exemple 6) est utilisé. Chaque protéine est incubée pour au moins 2h à 19,2 °C puis on procède à un rinçage extensif avec le tampon GB.

### EXEMPLE 5 : ASSEMBLAGE DE LA MOLECULE D'ADN SUR UN MICROSCOPE

Pour l'assemblage sur le microscope de la molécule d'ADN obtenue à l'Exemple 1, après les éventuelles étapes de digestion enzymatiques décrites dans les Exemples 8 ou 9, ou de celle obtenue aux Exemples 2 ou 4, le mélange réactionnel final est dilué à une concentration nominale d'ADN de 50 pM dans du tampon Tris (TrisCl 10 mM, pH 8). En parallèle des billes magnétiques (de type Dynal MyOne C1 de Thermofisher) ont été préparées en prenant 10 µl de solution stock fournie par le fabricant, en les lavant avec 100 µl de tampon utilisé pour les expériences de micromanipulation, i.e. RB pour les Exemples 8 et 9 et DB pour les Exemples 10 et 12 (voir l'Exemple 6 pour les compositions), en les concentrant avec un aimant, en éliminant le surnageant et en remettant les billes en suspension dans 10 µl du même tampon. Ensuite, 0,5 µl de la solution de molécule d'ADN double-brin selon l'invention est mélangé avec 10 µl de suspension de billes lavées. Après 5 à 10 secondes le volume du mélange est finalement ajusté par ajout de tampon pour arriver à un volume total de 25 µl puis injecté dans la cellule de mesure.

La cellule de mesure consiste en deux lamelles de verre d'épaisseur n°1 (-180 µm) séparées par deux épaisseurs de parafilm (-2 × 80 µm) dans lesquelles un canal (1 mm × 50 mm) a été découpé. Les deux lamelles sont fonctionnalisées avec l'antidigoxigénine et passivées comme décrit dans Duboc et al., 2017. L'une des lamelles a des trous de 2 mm de diamètre situés au-dessus de chaque extrémité du canal et qui sont utilisés pour remplir et drainer le canal rempli de réactifs. La surface, aussi nommée support, est montée sur une platine conçue sur mesure et placée au-dessus d'un objectif de microscope à immersion à huile (PlanA, 100x, NA 1.25, Olympus). Cet objectif fait partie d'un dispositif de piège magnétique (Lionnet *et al.,* 2012 et Sarkar et Rybenkov, 2016) dans lequel une paire d'aimants permanents de haute qualité situés au-dessus de l'échantillon peut être translatée à la verticale de l'échantillon : la force appliquée aux billes magnétiques est augmentée, alternativement réduite, en rapprochant, alternativement éloignant, les aimants de l'échantillon. La paire d'aimants permanents peut aussi effectuer un mouvement de rotation autour de l'axe optique de l'objectif de microscope, ce qui impose un mouvement de rotation à la bille et permet de surenrouler l'ADN. Dans son ensemble, le dispositif de piège magnétique permet de modifier les contraintes mécaniques appliquées à l'ADN. Un logiciel de suivi de particules en temps réel, PicoJai (PicoTwist SARL), permet le suivi par vidéo-microscopie des billes magnétiques avec une résolution nanométrique et en temps réel (dans cette réalisation à environ 30 Hz, mais potentiellement également à 10 kHz).

Un échantillon typique consiste en un champ de vision du microscope contenant environ 30 à 50 molécules d'ADN double-brin individuelles qui peuvent être surveillées simultanément. Le fonctionnement des dispositifs selon l'invention (la solidité de l'attachement aux supports, l'absence d'interactions non-spécifiques avec les supports, etc.) sont systématiquement vérifiées avant toutes caractérisations des molécules à tester. On contrôle par exemple le changement approprié de l'extension des molécules d'ADN double-brin selon l'invention lorsque la force appliquée est variée entre 0,05 pN et environ 1 à 3 pN (e.g. 1,4 pN). Lorsque cela est nécessaire, on peut vérifier en outre qu'une seule molécule d'ADN est attachée entre chaque bille et la surface de verre. Pour cela on s'assure que la position de la bille le long de l'axe optique ne change pas lorsque les aimants sont mis en rotation, ce qui est effectivement attendu lors de la libre rotation autour des jonctions. Par contre ce ne serait pas le cas si deux molécules d'ADN ou plus s'entortillaient l'une autour de l'autre.

### EXEMPLE 6 : CONDITIONS EXPERIMENTALES GENERALES

Les Exemples 8 et 9 ont été réalisés à 34°C dans du tampon de réaction RB (K-Hepes 20 mM pH 7,8, KCl 100 mM, MgCl₂ 5 mM, DTT 1 mM, Tween-20 0,05%, BSA 0,5 mg/ml). Pour les expériences de ligature / réparation, l'ADN ligase du phage T4 (New England Biolabs) est utilisée à une concentration de 200U/ml et les composants NHEJ (préparation en laboratoire) sont utilisés à une concentration de 10 nM Ku70/80 (dimère), 100 pM de DNA-PKcs, 20 nM de PAXX, 20 nM de XRCC4, 20 nM de XLF et 20 nM de ligase IV. Afin de digérer les molécules d'ADN qui avaient subi une ligature / réparation, nous avons perfusé le capillaire avec 100 µl de RB contenant soit 20 unités de Xmal ou 10 unités de Smal (New England Biolabs).

L'Exemple 10 a été réalisé soit à 25°C soit à 30°C dans du tampon de réaction DB (K-Hepes 20 mM pH 7,8, KCl 100 mM, MgCl₂ 5 mM, DTT 2 mM, Tween-20 0,1%, BSA 0,1 mg/ml). Des mesures ont aussi été réalisées à 19,2°C, 21,7°C, 25,4°C et 29,1 °C (voir a figure 15E). La rapamycine (Sigma) est utilisée diluée à 500 nM dans le tampon. Ces conditions sont aussi celles qui ont été utilisées pour l'exemple 12 pour ce qui concerne les mesures impliquant FRB et FKBP12 (Figure 18A-C).

L'Exemple 12 a été réalisé à 19,2°C dans du tampon de réaction GB (Tris 20 mM pH 8,0, NaCl 250 mM, MgCl₂ 5 mM, Tween-20 0,1%, BSA 0,5 mg/mL, 2-mercaptoethanol 5 mM) pour ce qui concerne les impliquant la « *PHloop* » et la géphyrine (**Figure 18D**).

### EXEMPLE 7 : COLLECTION ET ANALYSE DES DONNEES

Les données de pinces magnétiques à l'échelle d'une molécule unique, donnant la position de chaque bille magnétique en fonction du temps et sous des cycles de modulation de la force appliquée, ont été obtenues en utilisant la suite logicielle PicoJai (Picotwist SARL). Ce programme de suivi de particules en temps réel permet d'analyser jusqu'à 100 molécules uniques en parallèle, avec une résolution nanométrique dans les 3 dimensions de l'espace (Lionnet et al., 2012 ; Sarkar et Rybenkov, 2016). Il permet aussi de déterminer la tension appliquée à des molécules d'ADN par analyse des fluctuations browniennes des billes faisant office de support. Dans le cadre des Exemples 8, 9, 10 et 12, les forces F_{HF} et F_{LF} sont inférées à partir de la position le long de l'axe optique des aimants, en ayant recours à une calibration préalable sur des molécules d'ADN plus longues (Charomid 9-11, 11 kpb). L'homogénéité en charge magnétiques des billes utilisées ainsi que la variation lente de force en fonction de position des aimants fait que ce procédé est robuste.

En ce qui concerne les mesures associées à la caractérisation des interactions moléculaires entre molécules à tester, l'extension z des molécules d'ADN selon l'invention a été déterminée par analyse des traces de temps, ceci au cours de cycles variés où la force appliquée F varie en fonction du temps selon un programme temporel établi **(****Figure 8****).** Plus précisément, la mesure du temps de vie, t, pour les molécules à tester sous leur forme dissociée « D » ou sous leur forme associée « A », requiert une variation de F entre une valeur basse, F_{LF}, et une valeur haute, F_{HF}, les cycles temporels suivis étant différents selon le type de mesure, e.g. association ou association **(****Figure 8****).** La valeur de F_{LF} choisie doit permettre une association efficace des molécules à tester tandis que la valeur F_{HF} choisie doit permettre de distinguer les conformations « A » et « D » par mesure de l'extension z. Les événements donnant lieu à une modification de l'extension à haute force entre les formes A et D sont identifiés et le temps de vie associés à ces événements n'entrent dans l'analyse ultérieure que si la valeur « *z_{HF-D}* - *z*_{*HF*-*A*} » observée se situe à moins de 3 écarts-types de l'amplitude attendue (i.e. environ 160 nm dans le cas d'une longe d'environ 700 pb). L'écart-type est celui obtenu en traçant l'histogramme des « z_{HF-D} - *z_{HF-A}* » mesurés et en l'ajustant avec une gaussienne (**Figures 13B** **et** **11B****).**

Les longueurs des passages à basse et haute force, de longueur respective T_{LF} et T_{HF}, sont choisies en fonction d'hypothèses sur les temps caractéristiques d'association et de dissociation, τ_{A} et τ_{D}. L'objectif est ici de pouvoir détecter, avec la plus grande précision possible, le plus grand nombre de transitions conformationnelles sur la durée d'une expérience. Chaque cycle décrit dans la **Figure 8** conduit à la mesure éventuelle d'un temps de vie t, noté t_{LF-D} pour les expériences d'association (**Figure 8A****,** **11A**) et t_{HF-A} pour les expériences de dissociation (**Figure 8B****,** **13A**). Ces cycles sont reproduits en série sur une même molécule et les données sont acquises en parallèle sur une vingtaine de molécules. Les valeurs de t collectées sont ensuite rassemblées sous forme d'histogramme afin de pouvoir être analysées par ajustement exponentiel. L'histogramme des t_{LF-D} donne le temps caractéristique d'association τ_{A} en utilisant la formule Probabilité ∝ exp[-t_{LF-D}/τ_{A}] (**Figure 11C**) et l'histogramme des t_{HF-A} donne le temps caractéristique de dissociation τ_{D} en utilisant la formule Probabilité ∝ exp[-t_{HF-A}/τ_{D}] (**Figures 13C**).

Le temps caractéristique de dissociation peut ensuite être converti en vitesse de réaction de dissociation par inversion : k_{D} = 1/<τ_{D}>, où l'on a moyenné les temps caractéristiques de dissociation sur un ensemble de molécules. En effectuant ensuite des expériences pour différentes valeurs de F_{HF} et/ou de la température l'homme de l'art pourra finalement extraire des données collectées l'énergie d'activation de la réaction de dissociation et la distance séparant l'état de transition du complexe lors de la dissociation, ceci en utilisant l'équation d'Arrhenius/Bell (voir Exemple 10) (Popa et al., 2011).

Alternativement au mode de mesure par cyclage de force, présenté ci-dessus, la caractérisation des interactions moléculaires entre molécules à tester peut être effectuée par l'étude des fluctuations spontanées d'extension à force constante (**Figure 9A**). La valeur choisie pour cette force F_{CF} doit être assez élevée pour que la différence d'extension « z*_{CF-D}* - *z_{CF-A}* » puisse être mesurée ; elle doit cependant être assez faible pour que la proportion de molécules à tester présentes sous forme associée soit suffisante pour effectuer les mesures dans un temps raisonnable. L'extension z des molécules d'ADN selon l'invention est déterminée par analyse des traces temporelles. Les événements donnant lieu à une modification de l'extension entre les formes A et D sont identifiés et il est possible de déterminer un temps de vie t, noté t_{CF-D} pour la forme dissociée et t_{CF-A} pour la forme associée (**Figures 9A****,** **17A**). L'histogramme des t_{LF-D} donne le temps caractéristique d'association τ_{A} en utilisant la formule Probabilité ∝ exp[-t_{LF-D}/τ_{A}] (**Figure 17C**) et l'histogramme des t_{HF-A} donne le temps caractéristique de dissociation τ_{D} en utilisant la formule Probabilité ∝ exp[-t_{HF-A}/τ_{D}] (**Figures 17B**). Le temps caractéristique de dissociation peut ensuite être converti en vitesse de réaction de dissociation par inversion : k_{D} = 1/<τ_{D}>, où l'on a moyenné les temps caractéristiques de dissociation sur un ensemble de molécules.

Si l'on souhaite déterminer la vitesse de réaction d'association on peut effectuer des mesures de fluctuations spontanées en présence d'une des molécules à tester libre en solution (**Figure 9B**). L'analyse des traces temporelles (**Figures 9B****,** **17D**) permet de déterminer, pour les molécules à tester accrochées à la molécule d'ADN, la fraction de temps passé dans l'état associé, (∑t_{CF-A})/tₜₒₜₐₗ. Le tracé des variations de ce paramètre en fonction de Cₗ, la concentration en molécule à tester libre en solution, conduit ensuite à la détermination de C_{eff} et de K₀, où C_{eff} est la concentration effective en molécules à tester lorsqu'elles sont accrochées à la molécule d'ADN et où K₀ = K_{D}/C_{eff} avec K_{D} la constante d'équilibre de dissociation du complexe. Pour obtenir ces deux paramètres les données sont ajustées à la formule (∑t_{CF-A})/tₜₒₜₐₗ = ((1+K₀) + Cₗ /C_{eff} )⁻¹ (**Figure 17F**). Finalement on dérive la constante d'association grâce à k_{A} = k_{D}/K_{D}.

### EXEMPLE 8 : INTERACTION COVALENTE ENTRE DEUX BOUTS COHESIFS D'ADN DOUBLE-BRIN, EN PRESENCE DE L'ADN LIGASE DU PHAGE T4 PUIS DE L'ENZYME DE RESTRICTION SMAI

### Matériels et méthodes

La molécule d'ADN double-brin est fabriquée en suivant les étapes (A) à (C) de l'Exemple 3 (**Figure 6**) suivi par les étapes (A) et (B) de l'Exemple 1 (**Figure 4**), en utilisant une molécule d'ADN ayant les séquences de SEQ ID : 9 et SEQ ID : 10, pour les molécules d'ADN (A) et (B) respectivement. Une digestion enzymatique est ensuite effectuée par Xmal afin de générer un bout cohésif 5'-débordant de séquence 5'-CCGG-3' à chaque extrémité.

Cette digestion est effectuée en combinant 1 µl de solution à 3 nM de la molécule d'ADN selon l'invention issue de l'étape B de l'Exemple 1 et 10 unités de Xmal (New England Biolabs) dans un volume final de 30 µl tamponné par la solution CutSmart (New England Biolabs) selon le protocole du fabriquant. La digestion est effectuée à 37°C sur la nuit. L'enzyme de restriction a été inactivée par la chaleur selon les recommandations du fabricant, et la construction a été diluée 30 fois à une concentration d'ADN nominale de 100 pM et stockée à -20°C.

Les deux bouts cohésifs complémentaires peuvent donc par la suite s'apparier par interaction Watson-Crick. Les molécules à tester sont donc ici les bouts cohésifs des extrémités eux-mêmes. Les molécules d'ADN double-brin selon l'invention sont ensuite couplées à des microbilles magnétiques recouvertes de streptavidine, de diamètre 1 µm (tel que décrit, par exemple à l'Exemple 5). Le tout est finalement introduit dans un capillaire tapissé d'anti-digoxigénine ce qui permet de finaliser l'assemblage du dispositif en fixant les molécules d'ADN double-brin aux supports (**Fig. 2**).

### Résultats

À ce stade, les bouts cohésifs des extrémités ne s'apparient pas de façon stable. Pour la longe de - 0,7 kbp, sous l'effet d'une force alternant entre 0, 04 et 1,4 pN on voit la distance bille - support passer de z_{LF} = 400 nm à z_{HF-D} = 1 080 nm et inversement, signe que les molécules d'ADN double-brin transitent entre les états LF-D, « *Low Force-Dissociated »,* et HF-D, « *High Force* - *Dissociated* » (Figure 10A-B). Cependant, après ajout d'ADN ligase du phage T4, au temps expérimental d'environ 500 s, nous observons que l'extension maximale de la molécule d'ADN double-brin, sous l'effet d'une force de F_{HF} = 1,4 pN, n'est plus que de z_{HF-A} = 880 nm (**Figure 10B**). Cette réduction résulte de la ligature des bouts cohésifs complémentaires lors du premier passage à basse force. Cette réaction conduit à exclure la longe de la mesure d'extension dans l'état HF-A, « *High Force* - *Associated* » et à lui substituer les deux bouts associés en série (**Figure 10A**). Plus précisément, la différence de position de la bille entre les deux états « *High Force »,* déterminée par « *z_{HF-D}* - *z_{HF-A}* », correspond à 170 nm, une valeur en accord avec l'estimation pouvant être tirée du modèle d'élasticité WLC (« *worm-like chain* », c.f. (Bouchiat *et al.,* 1999)) lorsqu'une molécule linéaire d'ADN double-brin passe de 3 610 à 2 990 paires de bases et que la traction est de 1,4 pN. D'autre part, ce même modèle nous indique que la variation de d'extension entre les deux états « *Low Force* », i.e. sous une traction F_{LF} = 0, 04 pN, devrait être noyée dans le bruit de fond de la mesure, ce qui est également mis en évidence ici (**Figure 10B**). Finalement, le rajout de l'enzyme de restriction Smal, au temps expérimental d'environ 1 600 s, permet de couper la liaison covalente entre les bouts cohésifs des extrémités et de retrouver l'extension maximale de la construction, telle que mesurée en début d'expérience (**Figure 10B**).

Des observations similaires sont effectuées lorsque la longe mesure -6 kbp. Cependant, la variation d'extension est beaucoup plus grande : elle est maintenant de 1 800 nm (**Figure 10C**) car on passe de z_{HF-A} = 900 nm à z_{HF-D} = 2 700 nm. Ceci est en accord avec le modèle d'élasticité WLC lorsqu'une molécule linéaire d'ADN double-brin passe de 3 000 à 9 000 paires de bases et que la traction est de 1,1 pN. On note aussi que les états associés et dissociés à basse force peuvent maintenant être résolus. Autre changement de comportement, il faut plus de cycles passés à basse force pour que la ligation ait lieu : ce qui est en accord avec la dilution en molécules à tester inhérente à l'utilisation d'une longe de plus grande taille (Figure 10D-E).

### Discussion

Cet exemple démontre que des molécules d'ADN double-brin selon l'invention, ainsi que des dispositifs comprenant lesdites molécules et leurs supports, peuvent être fabriqués et sont pleinement fonctionnels. Elle valide le principe de la détection d'interaction entre molécules à tester situées aux bouts des extrémités par mesure de la distance entre les extrémités des molécules d'ADN (1) et (2) attachées au supports (e.g. la deuxième extrémité de la première molécule d'ADN double-brin (1) et la deuxième extrémité de la deuxième molécule d'ADN double-brin (2)). De plus des longes de différentes longueurs peuvent être utilisées, ce qui permet très avantageusement de moduler la rapidité avec laquelle les molécules à tester s'associent, ainsi que la différence d'extension qui est à mesurer pour détecter l'interaction.

### EXEMPLE 9 : INTERACTIONS COVALENTE ET NON-COVALENTE ENTRE DEUX BOUTS FRANCS D'ADN DOUBLE-BRIN, EN PRESENCE DES PROTEINES PARTICIPANT AU SYSTEME DE REPARATION NHEJ

### Matériels et méthodes

La molécule d'ADN double-brin est fabriquée en suivant les étapes (A) à (C) de l'Exemple 3 (**Figure 6**) suivi par les étapes (A) et (B) de l'Exemple 1 (**Figure 4**), en utilisant une molécule d'ADN ayant les séquences de SEQ ID : 9 et SEQ ID : 10, pour les molécules d'ADN (A) et (B) respectivement. Une digestion enzymatique est ensuite effectuée par Smal afin de générer un bout franc à chaque extrémité. Cette digestion est effectuée en combinant 1 µl de solution à 3 nM de construction issue de l'étape B de l'Exemple 1 et 20 unités de Smal (New England Biolabs) dans un volume final de 30 µl tamponné par la solution CutSmart (New England Biolabs) selon le protocole du fabriquant. La digestion est effectuée à 25°C sur la nuit. L'enzyme de restriction a été inactivée par la chaleur selon les recommandations du fabricant, et la construction a été diluée 2 fois à une concentration d'ADN nominale de 50 pM et stockée à -20 °C.

Le protocole de préparation est arrêté à ce stade si l'on souhaite étudier le phénomène de réparation jusqu'à la formation des liaisons covalentes reliant chacun des brins de la double-hélice. Par contre, une étape de déphosphorylation des bouts francs des extrémités est réalisée en utilisant l'antarctic phosphatase si l'on souhaite étudier les interactions transitoires entre les deux extrémités d'ADN. Plus précisément, les bouts francs des extrémités de la molécule d'ADN ont été déphosphorylés en combinant, dans un volume de 10 µl de tampon 1x ad hoc fourni par le fournisseur, 2 unités de phosphatase antarctique (New England Biolabs) et 0,6 fmol du produit de digestion obtenu ci-dessus. La réaction a durée au moins 1 heure à 37°C avant d'être inactivée thermiquement selon les recommandations du fabricant.

Encore une fois les molécules à tester sont donc ici les bouts francs des extrémités eux-mêmes.

### Résultats

De la même façon que nous avons étudié la réparation par la ligase du phage T4 des cassures présentant des bouts cohésifs (**Figure 10B**), nous nous intéressons au fonctionnement du système humain NHEJ (« *non-homologous end-joining* ») qui lui répare les cassures présentant des extrémités franches. Ce dernier est plus précisément constitué des protéines Ku70/Ku80, DNA-PKcs, PAXX, XRCC4, XLF et Ligase IV. La **Figure 11A** montre une expérience très similaire à celle effectuée lors de l'Exemple 8. En l'absence de liaison covalente entre les bouts francs, l'extension varie entre z_{LF-D} = 400 nm et z_{HF-D} = 1 080 nm lorsque la force varie entre F_{LF} = 0,05 pN et F_{HF} = 1,4 pN. Cependant, après ajout du système NHEJ, au temps expérimental d'environ 600 s, l'extension maximale de l'ADN sous l'effet d'une force F_{HF} = 1,4 pN n'est plus que de z_{HF-A} = 880 nm. Cette réduction est attribuée à la ligature des bouts francs lors du second passage à basse force. Comme précédemment, le rajout de l'enzyme de restriction Smal après un certain nombre de cycles entre « *High Force* » et « *Low Force* », au temps expérimental d'environ 4 000 s, permet de couper la liaison covalente qui avait été formée et de retrouver l'extension maximale telle qu'initialement mesurée. Lorsque ce protocole est reproduit sur un grand nombre de billes, la mesure de la différence d'extension entre les formes HF-A et HF-D peut être affinée (**Figure 11B**) : la valeur moyenne « *z_{HF-D}* - *z_{HF-A}* » = 161 nm déterminée à F_{HF} =1,4 pN est en accord avec l'estimation trouvée lors de l'Exemple 8 et avec les prédictions dérivées du modèle WLC « *worm-like chain* ». Finalement, on peut aussi tracer l'histogramme du nombre de passages à basse force, n_{LF-D}, nécessaires pour obtenir la ligature (**Figure 11C**). Par ajustement avec une exponentielle cette courbe fournit une estimation du temps caractéristique nécessaire à la formation de liens covalents entre les molécules à tester, τ_{A} = 0,8 ± 0,2 cycle soit 240 ± 60 s si le résultat donné en cycles est multiplié par la durée d'un passage à basse force, à savoir T_{LF} = 300 s.

Ces expériences de ligature covalente ont aussi permis de tester la surenroulabilité des molécules d'ADN double-brin (1) et (2) associées en série (**Figure 11**) ; il n'y a aucune entaille dans les éléments fonctionnels susmentionnés.

Dans un second type d'expériences, nous avons cherché à mesurer le temps caractéristique de dissociation du complexe nucléoprotéique dans lequel les bouts francs ont été déphosphorylés, ce qui empêche le système NHJE de créer des liens covalents. Chaque passage à F_{LF} = 0,05 pN permet aux bouts francs de s'associer et c'est donc la forme HF-A qui est observée lorsque l'on augmente la force à F_{HF} =1,4 pN. Puis, au bout d'un temps noté t_{HF-A}, l'extension de la construction passe à sa valeur maximale, signe que les bouts francs se sont dissociés et que c'est maintenant la longe qui est mise en tension (**Figure 13A**). Le tracé de l'histogramme des différences d'extension entre les formes HF-A et HF-D donne une valeur moyenne de « *z_{HF-D}* - *z_{HF-A}* » = 166 nm (**Figure 13B**). D'autre part un temps caractéristique de dissociation τ_{D} = 2,2 ± 0,3 s peut être extrait de l'histogramme des t_{HF-A} par ajustement exponentiel (**Figure 13C**).

### Discussion

Cet exemple met en évidence l'utilité de la molécule d'ADN selon l'invention, ainsi que des dispositifs comprenant lesdites molécules et leurs supports, pour conduire des études sur les processus de réparation de l'ADN. De plus, le fait de pouvoir injecter divers mélanges de protéines et de pouvoir à chaque fois caractériser les complexes formés (données non présentées) est très avantageuse pour des études de criblage.

D'un point de vue technique, cet exemple illustre :
(i) un protocole de mesure du temps caractéristique d'association lorsqu'une force constante est appliquée à des molécules à tester interagissant entre-elles ;
(ii) un protocole de mesure de temps caractéristique de dissociation lorsqu'une force constante est appliquée à des molécules à tester interagissant entre-elles ; et
(iii) la possibilité d'appliquer, au travers de la rotation des aimants, un couple au complexe formé par les molécules à tester.

### EXEMPLE 10 : INTERACTION NON-COVALENTE ENTRE LES PROTEINES FKBP12 ET FRB, EN PRESENCE DU MEDICAMENT RAPAMYCINE

Cet exemple porte sur un système pharmaceutique pertinent qu'il serait très avantageux de caractériser dans le domaine de la découverte/conception de médicaments.

### Matériels et méthodes

La molécule d'ADN double-brin est celle réalisée selon la description donnée dans l'Exemple 3, suivi par les Exemples 1 et 2 (voir aussi **Figure 6****,** **Figure 4** et **Figure 5**, respectivement pour une illustration de ces étapes), en utilisant une molécule d'ADN ayant les séquences de SEQ ID : 30 et SEQ ID : 10, pour les molécules d'ADN (A) et (B) respectivement. Cela nous permet de greffer spécifiquement les deux molécules à tester, à une extrémité la sous-unité FRB du complexe protéique mTOR et à l'autre la protéine FKBP12.

### Résultats

La drogue rapamycine perturbe l'activité de la voie de signalisation mTOR en se liant à la fois à FRB et à FKBP12.

Dans un premier temps, afin d'estimer τ_{D}, le temps caractéristique de dissociation de ce complexe ternaire, nous avons utilisé un protocole expérimental très semblable à celui mis en oeuvre pour les associations non covalentes impliquant le système NHEJ (**Figure 13**). Les traces temporelles enregistrées à 25°C lors d'une modulation de la force entre les valeurs F_{LF} = 0,05 pN et F_{HF} = 1,4 pN (**Figure 14A**) ont été converties en histogramme des t_{HF-A} dont on a pu ensuite extraire τ_{D} = 31,1 ± 2,3 s par ajustement monoexponentiel (**Figure 14B**). De plus, nous avons estimé la différence d'extension entre les formes HF-A et HF-D « *z_{HF-D}* - *z_{HF-A}* » à environ 170 nm, elle est essentiellement inchangée par rapport aux mesures effectuées lors des Exemples 8 et 9. En effet la longueur de la longe moins celle des extrémités est restée la même dans la molécule d'ADN utilisée dans le présent Exemple 10, soit environ 700 pb, et le modèle « *WLC* » est très proche de la linéarité pour les hautes forces, ce qui explique une contribution identique de ces 700 pb à l'extension totale, ceci malgré l'utilisation de molécules d'ADN (1) et (2) de taille différentes dans les Exemples 8 et 9 et dans le présent Exemple 10.

À partir du temps caractéristique de dissociation, τ_{D}, il est possible de calculer la constante de vitesse de dissociation, k_{D}, par moyennage sur plusieurs molécules et inversion : k_{D} = 1 / <τ_{D}>. Il vient donc dans le cas de la **Figure 14**, qui correspond à une force appliquée F_{HF} = 1,4 pN et à une température T = 298 K, k_{D} = 32,2 × 10⁻³ s⁻¹. De mêmes mesures peuvent être effectuées à une force et/ou à une température différente, comme démontré dans la Figures 15A-D. À titre d'exemple, à T = 303 K nous obtenons ainsi k_{D} = 48,4 × 10⁻³ s⁻¹ pour F_{HF} = 1,4 pN et k_{D} = 64,9 × 10⁻³ s⁻¹ pour F_{HF} = 6 pN. Il est alors possible d'utiliser l'équation d'Arrhenius/Bell, k_{D}(F_{HF},T) = A × exp[-E_{D}/RT] × exp[X_{D}F_{HF}/k_{B}T], pour calculer E_{D}, l'énergie d'activation de la réaction de dissociation, et X_{D}, la distance séparant l'état de transition du complexe lors de la dissociation (Popa et al., 2011). A est ici un préfacteur, R la constante universelle des gaz parfaits et k_{B} la constante de Boltzmann ; R = 8,31 J K⁻¹ mol⁻¹ et k_{B} = 1,38 × 10⁻²³ J K⁻¹. Plus précisément de premières régressions linéaires sur les courbes donnant ln[k_{D}] en fonction de F_{HF} à diverses températures (**Figure 15E**) donnent, après extraction des pentes et des ordonnées à l'origine, X_{D} et ln[k_{D}⁰] en fonction de T (Figures 15F-G). k_{D}⁰ est ici la constante de vitesse de dissociation extrapolée à force nulle : k_{D}⁰(T) = A × exp[-E_{D}/RT]. Par moyennage des différentes valeurs de X_{D}, paramètre théoriquement indépendant de la température, on trouve X_{D} = 4,31 Å. Par régression linéaire sur la courbe donnant ln[k_{D}⁰] en fonction de T on extrait une pente qui est directement reliée à l'énergie d'activation. On trouve E_{D} = 58,8 kJ mol⁻¹.

Dans un second temps, afin d'estimer τ_{D}, le temps caractéristique de dissociation du complexe ternaire et τ_{A}, le temps caractéristique d'association de FRB avec le très stable complexe binaire FKBP12-rapamycine, nous avons utilisé un protocole expérimental reposant sur l'étude des fluctuations spontanées d'extension à force constante (**Figure 9A**). Les traces temporelles enregistrées à 25°C et lorsque l'on applique F_{CF} = 0,04 pN (**Figure 17A**) ont été converties en histogramme des t_{CF-A}, dont on a pu ensuite extraire τ_{D} = 30,4 ± 2,7 s par ajustement monoexponentiel (**Figure 17B**), et en histogramme des t_{CF-D}, dont on a pu ensuite extraire τ_{A} = 13,7 ± 1,1 s par ajustement monoexponentiel (**Figure 17C**). En moyennant les résultats obtenus sur plusieurs molécules d'ADN et en inversant on obtient k_{D} = 34,1 s⁻¹.

Pour déterminer la paire de constantes {k_{A}, k_{D}} on enregistre les fluctuations spontanées d'extension lorsque, à 25°C, on applique F_{CF} = 0,04 pN en présence de concentrations croissantes de FRB en solution, entre 0 et 200 nM (**Figure 9B**, **Figure 17D**). A partir des histogrammes des t_{CF-A} on peut, comme précédemment et pour chaque concentration en FRB, calculer <τ_{D}>. Les résultats obtenus à différentes concentrations (**Figure 17E**) sont ensuite moyennés et la moyenne inversée afin de conduire à k_{D} = 32,7 × 10⁻³ s⁻¹. D'autre part, à partir du suivi temporel des fluctuations pour diverses molécules d'ADN il est avantageusement possible de déterminer, toujours pour chaque concentration en FRB, la fraction de temps passée par les molécules à tester sous forme associée. Ladite fraction de temps passée par les molécules à tester sous forme associée est déterminée selon (∑t_{CF-A})/tₜₒₜₐₗ, où tₜₒₜₐₗ correspond au temps total pendant lequel les fluctuations sont enregistrées et analysées. Les données sont ensuite ajustées à l'aide de (∑t_{CF-A})/tₜₒₜₐₗ = ((1+K₀) + [FRB]/C_{eff} )⁻¹, ce qui conduit à la détermination de C_{eff} et de K₀ (**Figure 17F**). On trouve ici de C_{eff} = 12,3 nM et K₀ = 0,59. Comme on a par ailleurs K₀ = K_{D}/C_{eff} avec K_{D} la constante d'équilibre de dissociation du complexe formé par FRB et FKBP12-rapamycine, il vient K_{D} = 7,26 nM. Finalement on dérive la constante d'association grâce à k_{A} = k_{D}/K_{D} ; on trouve k_{A} = 4,50 10⁶ M⁻¹s⁻¹.

### Discussion

D'un point de vue technique, le protocole de mesure de temps caractéristique de dissociation par cyclage de la force est confirmé. Il permet de déterminer de façon simple la constante de vitesse de dissociation. En effectuant ces mesures à différentes forces hautes et/ou à différentes températures, il est également possible de déterminer l'énergie d'activation de la réaction de dissociation et la distance séparant l'état de transition du complexe lors de la dissociation.

L'étude des fluctuations spontanées d'extension à force constante permet très avantageusement d'obtenir le temps caractéristique de dissociation et le temps caractéristique d'association. En effectuant ces mesures lorsque le tampon contient différentes concentrations en l'un des partenaires moléculaires, il est également avantageusement possible de mesurer la constante d'équilibre de la réaction de dissociation ainsi que la constante de vitesse de la réaction d'association.

### EXEMPLE 11 : MISE EN EVIDENCE DE LA CAPACITE DU DISPOSITIF A DISTINGUER LES INTERACTIONS SPECIFIQUES DES INTERACTIONS NON-SPECIFIQUES

### Matériels et méthodes

Comme à l'exemple 10, la molécule d'ADN double-brin est celle réalisée selon la description donnée dans l'Exemple 3, suivi par les Exemples 1 et 2 (voir aussi **Figure 6**, **Figure 4** et **Figure 5**, respectivement pour une illustration de ces étapes), en utilisant une molécule d'ADN ayant les séquences de SEQ ID : 30 et SEQ ID : 10, pour les molécules d'ADN (A) et (B) respectivement. Les protéines FKBP12 et FRB constituent ici les deux molécules à tester, qui sont greffées aux extrémités des molécules d'ADN (1) et (2) de façon spécifique. Les expériences sont réalisées à 25°C dans le tampon DB.

### Résultats

En plus des conformations A et D classiquement observées, caractérisées par les extensions z_{LF}, z_{HF-A} et z_{HF-D}, la molécule d'ADN reste ici bloquée dans une quatrième conformation pendant un peu moins de 6 000 s. Cette conformation, notée S pour « *Support* », est caractérisée par les extensions z_{LF} et z_{HF-S}, la valeur de ce dernier paramètre indiquant une interaction de l'extrémité portant une des molécules à tester avec un des supports (voir la **Figure 16**).

### Discussion

Cet exemple démontre la capacité du dispositif à distinguer les interactions spécifiques des interactions non-spécifiques (e.g. entre une des molécules à tester et un des supports peuvent être mises en évidence, **Figure 16**). Avantageusement, l'utilisation de molécules d'ADN (1) et (2) de longueurs différentes et judicieusement choisies par rapport à celle de la longe permet de détecter des variations non-spécifiques de l'extension à haute force (e.g. entre une des molécules à tester et un des supports), et donc par conséquent de ne pas analyser les événements concernés.

Une stratégie permettant d'accrocher des molécules à tester de type protéique aux extrémités et d'étudier des réactions d'association/dissociation entre elles, ceci en présence d'une molécule à tester de type petite molécule en solution, a notamment été mise en oeuvre dans ces exemples. Aussi, de façon très avantageuse, la molécule d'ADN selon l'invention, le dispositif comprenant ladite molécule d'ADN et ses supports, ainsi que le procédé de caractérisation d'une ou plusieurs interactions peut être utilisé pour détecter et caractériser des interactions moléculaires, notamment afin d'identifier, de cribler, et/ou de concevoir de nouveaux médicaments, ou d'étudier les interactions moléculaires de médicaments actuels.

### EXEMPLE 12 : UTILISATION D'ETIQUETTES PROTEIQUES DE TYPE SNAP/CLIP POUR L'ACCROCHAGE DES MOLECULES A TESTER SUR LA MOLECULE D'ADN

### Matériels et méthodes

La molécule d'ADN double-brin est celle réalisée selon la description donnée dans l'Exemple 3, avec les modifications et compléments indiqués dans les Exemples 1 et 4 (voir aussi **Figure 6****,** **Figure 4** et **Figure 7**, respectivement pour une illustration de ces étapes). les deux molécules à tester, qui sont greffées aux extrémités des molécules d'ADN (1) et (2) de façon spécifique, sont soit FRB et FKBP12, soit la « PHloop » et la géphyrine.

### Résultats

L'utilisation d'étiquettes SNAP et CLIP pour accrocher les molécules à tester aux extrémités de la molécule d'ADN a tout d'abord été mise en oeuvre pour l'étude de l'interaction de FRB avec FKBP12 médiée par la rapamycine. La force étant cyclée entre valeur haute et basse on obtient des traces temporelles similaires à celles obtenues lorsque la stratégie d'accrochage repose sur la ligature de molécules à tester fonctionnalisées par des oligonucléotides (on comparera la **Figure 18A** à la **Figure 14A**). On peut ensuite tracer les histogrammes des t_{HF-A} **(****Figure 18B**). En répétant ces expériences pour de multiples molécules d'ADN et pour différentes forces hautes, on obtient finalement la variation du logarithme de la constante de vitesse de dissociation en fonction de F_{HF} (**Figure 18C**). On remarquera que les valeurs de ln[k_{D}⁰] et de X_{D} pouvant alors être extraites par ajustement linéaire, respectivement -3.45 ± 0.03 et 4.7 ± 0.2 Å, sont très proches de celles fournies par la stratégie d'accrochage par ligature à la même température, respectivement -3.48 ± 0.01 et 4.4 ± 0.1 Å **(****Figure** 15E).

Dans un second temps, l'utilisation d'étiquettes SNAP et CLIP a été mise en oeuvre pour l'étude des interactions entre la « PHloop » et la géphyrine. Encore une fois des traces temporelles permettant de déterminer les t_{HF-A} ont pu être enregistrées (**Figure 18D**).

### Discussion

Cet exemple démontre la capacité du dispositif à évaluer les interactions entre différentes partenaires moléculaires attachés aux extrémités des molécules d'ADN double brin (1) et (2) par différentes stratégies (ici par l'utilisation des étiquettes de type SNAP et CLIP).

### REFERENCES

Ausubel et al., Current Protocols in Molecular Biology, Eds., John Wiley & Sons, Inc. New York, 1995.
Bedet et al., J. Biol. Chem., 2006 ; 281, 30046-56.Bouchiat et al., Biophys. J. 1999 ; 76: 409-413.
Conroy, "Force Spectroscopy with Optical and Magnetic Tweezers," dans le Handbook of Molecular Force Spectroscopy, pages 23-96 (ed. A. Noy, Springer, 2008).
Doi, Introduction to Polymer Physics, Clarendon Press, 1996.
Duboc et al., Methods Enzymol. 2017 ; 582 : 275-296.
Gao et al., Science 2012 ; 337(6100) :1340-1343.
Gautier et al., Chemistry & biology, 2008 ; 15 : 128-136.
Grunewald et al., eNeuro, 2018, 5. doi : 10.1523/ENEURO.0042-17.2018.
Halvorsen et al., Nanotechnology. 2011 ; 22(49) : 494005.
Hein et al., Pharm Res. 2008 ; 25(10) : 2216-2230.
Keppler et al., Nat. Biotechnol., 2003 ; 21 : 86-89.
Kilchherr et al., Science. 2016 ; 353(6304).
Kim et al., Nature. 2010 ; 466(7309) : 992-5.
Lahann, Click Chemistry for Biotechnology and Materials Science, John Wiley & Sons, Chichester, England, 2009.
Lionnet et al., Cold Spring Harbor Protoc. 2012: 133-138 (2012).
Pilling et Seakins, Reaction kinetics, Oxford University Press (1995).
Popa et al., J Biol Chem. 2011 ; 286 (36) : 31072-31079.
Pingoud et Jeltsch, Nucleic Acids Res. 2001 ; 29 (18) : 3705-3727.
Remington's Pharmaceutical Sciences, 17ème éd., Mack Publishing Co., Easton, Pa., 1985. Restriction Endonucleases Technical Guide », New England Biolabs.
Rognoni et al., Proc Natl Acad Sci USA. 2012 ;109(48) : 19679-84.
Rothemund, Nature. 2006 ; 440(7082) : 297-302.
Sambrook et al., Molecular cloning : Un manuel de laboratoire, 2e édition, Cold Spring Harbor Laboratory Press - Cold Spring Harbor, NY, USA, 1989.
Sarkar et Rybenkov, Frontiers Phys. 2016 ; 4 : 48.
Wiita et al., Proc Natl Acad Sci USA. 2006 ; 103(19) : 7222-7227.
Woodside et al., Proc. Natl. Acad. Sci. U.S.A., 103 (16): 6190-6195, 2006.
Yang et al., Nat Commun. 2016 Mar 17;7:11026. doi: 10.1038/ncomms11026.

## Revendications

1. Molécule d'ADN double-brin comprenant une première molécule d'ADN double-brin (1) reliée à une deuxième molécule d'ADN double-brin (2) par une longe comprenant de l'ADN double-brin, dans laquelle ladite longe est attachée par
(i) au moins une liaison covalente à un nucléotide de la première molécule d'ADN double-brin (1) et par
(ii) au moins une liaison covalente à un nucléotide de la deuxième molécule d'ADN double-brin (2),
selon laquelle ladite liaison covalente de (i) et ladite liaison covalente de (ii) ne sont pas des liaisons phosphodiesters, des liaisons phosphorothioates, des liaisons phosphoramidates ou des liaisons phosphorodiamidate, et ledit nucléotide de (i) et ledit nucléotide de (ii) ne sont pas des nucléotides ultimes de chaque extrémité desdites molécules d'ADN double-brin (1) et (2).

2. Molécule d'ADN double-brin selon la revendication 1, dans laquelle ladite longe est une molécule d'ADN double-brin.

3. Molécule d'ADN double-brin selon la revendication 1 ou 2, dans laquelle ladite longe est attachée à la première molécule d'ADN double-brin (1) par une première liaison covalente entre la première extrémité de ladite longe et une région intermédiaire de la première molécule d'ADN double-brin (1) et à la deuxième molécule d'ADN double-brin (2) par une deuxième liaison covalente entre la deuxième extrémité de ladite longe et une région intermédiaire de la deuxième molécule d'ADN double-brin (2).

4. Molécule d'ADN double-brin selon l'une quelconque des revendications 1 à 3, dans laquelle une première molécule à tester est liée à une première extrémité de la ladite première molécule d'ADN double-brin (1) et une deuxième molécule à tester est liée à une première extrémité de la ladite deuxième molécule d'ADN double-brin (2).

5. Molécule d'ADN double-brin selon la revendication 4, dans laquelle la deuxième extrémité de ladite première molécule d'ADN double-brin (1) est liée à un premier support et la deuxième extrémité de ladite deuxième molécule d'ADN double-brin (2) est liée à un deuxième support, avantageusement dans laquelle au moins un des deux supports est un support mobile.

6. Molécule d'ADN double-brin selon l'une quelconque des revendications 4 ou 5, dans laquelle :
• ladite première molécule d'ADN double-brin (1) et/ou ladite deuxième molécule d'ADN double-brin (2) a une longueur de 300 à 5000 paires de bases, de préférence de 650 à 1500 paires de bases ;
• la première extrémité de la première molécule d'ADN double-brin (1) et/ou la première extrémité de la deuxième molécule d'ADN double-brin (2) à une longueur de 10 à 150 paires de bases, avantageusement de 30 à 50 paires de bases ; et/ou
• ladite longe a une longueur d'environ 300 à environ 50 000 paires de bases, avantageusement d'environ 500 à 10 000 paires de bases, plus avantageusement d'environ 600 à 3000 paires de bases.

7. Molécule d'ADN double-brin selon l'une quelconque des revendications 4 à 6, dans laquelle ladite première et/ou ladite deuxième molécule à tester est sélectionnée dans le groupe composé des molécules suivantes : polymères, acides aminés, peptides, polypeptides, protéines, nucléosides, nucléotides, polynucléotides, oligonucléotides, sucres, polysaccharides, petites molécules, drogues, aptamères, antigènes, anticorps, lipides, lectines, hormones, vitamines, virus, fragments de virus, nanoparticules, molécules cellulaires de surface, et facteurs de transcription.

8. Molécule d'ADN double-brin selon l'une quelconque des revendications 1 à 7, pour une utilisation dans la mesure des interactions entre au moins deux molécules à tester.

9. Dispositif comprenant la molécule d'ADN double-brin selon l'une quelconque des revendications 1 à 8 avec ses supports.

10. Molécule d'ADN double-brin comprenant une première molécule d'ADN double-brin (A) et une deuxième molécule d'ADN double-brin (B), ladite molécule d'ADN double-brin (A) comprenant un site de clivage qui est présent uniquement dans ladite molécule d'ADN double-brin (A), ladite molécule d'ADN double-brin (A) étant reliée à la molécule d'ADN double-brin (B) par deux liaisons covalentes qui ne sont pas des liaisons phosphodiesters, des liaisons phosphorothioates, des liaisons phosphoramidates ou des liaisons phosphorodiamidate, de part et d'autre dudit site de clivage, dans laquelle lesdites liaisons covalentes n'ont pas lieu aux nucléotides ultimes des extrémités de ladite molécule d'ADN double-brin (A).

11. Procédé de fabrication de la molécule d'ADN double-brin selon l'une quelconque des revendications 1 à 8, **caractérisé en ce qu'**il comprend l'étape suivante :
a) le clivage de la molécule d'ADN double-brin (A) selon la revendication 10 audit site de clivage, générant ainsi une molécule d'ADN double-brin comprenant une première molécule d'ADN double-brin (1) et une deuxième molécule d'ADN double-brin (2).

12. Procédé de caractérisation d'une interaction entre au moins deux molécules à tester liées à une molécule d'ADN double-brin selon l'une quelconque des revendications 1 à 8 ou telle que comprise dans le dispositif de la revendication 9, comprenant :
a) l'application d'une force physique basse, F_{LF}, à la molécule d'ADN double-brin, qui permet aux molécules à tester de s'associer ;
b) l'application d'une force physique haute, F_{HF}, à la molécule d'ADN double-brin, qui permet de déterminer si les molécules à tester sont associées ou dissociées ; et
c) la détection d'un changement dans les propriétés conformationnelles de la molécule d'ADN comprenant :
- la détermination de l'extension z_{LF} entre la deuxième extrémité de la première molécule d'ADN double-brin (1) et la deuxième extrémité de la deuxième molécule d'ADN double-brin (2) à l'étape a) ;
- la détermination des extensions z_{HF-A} et z_{HF-D} entre la deuxième extrémité de la première molécule d'ADN double-brin (1) et la deuxième extrémité de la deuxième molécule d'ADN double-brin (2), à l'étape b), dans laquelle z_{HF-A} est l'extension quand les molécules sont associées et z_{HF-D} est l'extension quand les molécules sont dissociées ; et
- la comparaison des extensions z_{LF}, z_{HF-A}, et z_{HF-D}, en fonction du temps t.

13. Procédé selon la revendication 12, ledit procédé comprenant en outre l'étape supplémentaire suivante :
d) la détection d'un changement dans les propriétés conformationnelles de la molécule d'ADN comprenant :
- la détermination des extensions z_{LF-A} et z_{LF-D} entre la deuxième extrémité de la première molécule d'ADN double-brin (1) et la deuxième extrémité de la deuxième molécule d'ADN double-brin (2) à l'étape a), dans laquelle z_{LF-A} est l'extension quand les molécules sont associées et z_{LF-D} est l'extension quand les molécules sont dissociées;
- la détermination des extensions z_{HF-A} et z_{HF-D} entre la deuxième extrémité de la première molécule d'ADN double-brin (1) et la deuxième extrémité de la deuxième molécule d'ADN double-brin (2), à l'étape b) , dans laquelle z_{HF-A} est l'extension quand les molécules sont associées et z_{HF-D} est l'extension quand les molécules sont dissociées ; et
- la comparaison des extensions z_{LF-A}, z_{LF-D}, z_{HF-A}, et z_{HF-D}, en fonction du temps t.

14. Procédé de caractérisation d'une interaction entre au moins deux molécules à tester liées à une molécule d'ADN double-brin selon l'une quelconque des revendications 1 à 8 ou telle que comprise dans le dispositif de la revendication 9, comprenant :
a) l'application d'une force constante F_{CF}, à la molécule d'ADN double-brin, qui permet aux molécules à tester de s'associer et de se dissocier ; et
b) la détection d'un changement dans les propriétés conformationnelles de la molécule d'ADN comprenant :
- la détermination de la dissociation spontanée des molécules à tester au bout du temps t_{CF-A}, et/ou
- la détermination de l'association spontanée au bout d'un temps t_{CF-D}.

15. Procédé selon l'une quelconque des revendications 12 à 14, selon lequel la caractérisation de l'interaction comprend la détermination d'au moins une des caractéristiques choisies parmi : le temps caractéristique d'association, le temps caractéristique de dissociation, la constante de vitesse de dissociation, l'énergie d'activation de dissociation, la distance séparant l'état de transition du complexe lors de la dissociation, et la constante d'équilibre de dissociation.

## Patentansprüche

1. Doppelsträngiges DNA-Molekül, umfassend ein erstes doppelsträngiges DNA-Molekül (1), das mit einem zweiten doppelsträngigen DNA-Molekül (2) durch eine Leine verbunden ist, die Doppelstrang-DNA umfasst, wobei die Leine befestigt ist durch
(i) mindestens eine kovalente Bindung an ein Nukleotid des ersten doppelsträngigen DNA-Moleküls (1) und durch
(ii) mindestens eine kovalente Bindung an ein Nukleotid des zweiten doppelsträngigen DNA-Moleküls (2),
wobei die kovalente Bindung von (i) und die kovalente Bindung von (ii) keine Phosphodiester-Bindungen, Phosphorothioat-Bindungen, Phosphoramidat-Bindungen oder Phosphordiamidat-Bindungen sind und das Nukleotid von (i) und das Nukleotid von (ii) keine letzten Nukleotide jedes Endes der doppelsträngigen DNA-Moleküle (1) und (2) sind.

2. Doppelsträngiges DNA-Molekül nach Anspruch 1, wobei die Leine ein doppelsträngiges DNA-Molekül ist.

3. Doppelsträngiges DNA-Molekül nach Anspruch 1 oder 2, wobei die Leine am ersten doppelsträngigen DNA-Molekül (1) durch eine erste kovalente Bindung zwischen dem ersten Ende der Leine und einer Übergangsregion des ersten doppelsträngigen DNA-Moleküls (1) und am zweiten doppelsträngigen DNA-Molekül (2) durch eine zweite kovalente Bindung zwischen dem zweiten Ende der Leine und einer Übergangsregion des zweiten doppelsträngigen DNA-Moleküls (2) befestigt ist.

4. Doppelsträngiges DNA-Molekül nach einem der Ansprüche 1 bis 3, wobei ein erstes zu testendes Molekül mit einem ersten Ende des ersten doppelsträngigen DNA-Moleküls (1) verbunden ist und ein zweites zu testendes Molekül mit einem ersten Ende des zweiten doppelsträngigen DNA-Moleküls (2) verbunden ist.

5. Doppelsträngiges DNA-Molekül nach Anspruch 4, wobei das zweite Ende des ersten doppelsträngigen DNA-Moleküls (1) mit einem ersten Träger verbunden ist und das zweite Ende des zweiten doppelsträngigen DNA-Moleküls (2) mit einem zweiten Träger verbunden ist, wobei in vorteilhafter Weise mindestens einer der zwei Träger ein beweglicher Träger ist.

6. Doppelsträngiges DNA-Molekül nach einem der Ansprüche 4 oder 5, wobei:
• das erste doppelsträngige DNA-Molekül (1) und/oder das zweite doppelsträngige DNA-Molekül (2) eine Länge von 300 bis 5000 Basenpaaren, vorzugsweise von 650 bis 1500 Basenpaaren hat;
• das erste Ende des ersten doppelsträngigen DNA-Moleküls (1) und/oder das erste Ende des zweiten doppelsträngigen DNA-Moleküls (2) eine Länge von 10 bis 150 Basenpaaren, in vorteilhafter Weise von 30 bis 50 Basenpaaren hat; und/oder
• die Leine eine Länge von zirka 300 bis zirka 50000 Basenpaaren, in vorteilhafter Weise von zirka 500 bis 10000 Basenpaaren, in vorteilhafterer Weise von zirka 600 bis 3000 Basenpaaren hat.

7. Doppelsträngiges DNA-Molekül nach einem der Ansprüche 4 bis 6, wobei das erste und/oder das zweite zu testende Molekül aus der Gruppe ausgewählt ist, die aus den folgenden Molekülen zusammengesetzt ist: Polymere, Aminosäuren, Peptide, Polypeptide, Proteine, Nukleoside, Nukleotide, Polynukleotide, Oligonukleotide, Zucker, Polysaccharide, kleine Moleküle, Drogen, Aptamere, Antigene, Antikörper, Lipide, Lektine, Hormone, Vitamine, Viren, Virusfragmente, Nanopartikel, Zelloberflächenmoleküle und Transkriptionsfaktoren.

8. Doppelsträngiges DNA-Molekül nach einem der Ansprüche 1 bis 7 für eine Verwendung beim Messen der Interaktionen zwischen mindestens zwei zu testenden Molekülen.

9. Vorrichtung, umfassend das doppelsträngige DNA-Molekül nach einem der Ansprüche 1 bis 8 mit seinen Trägern.

10. Doppelsträngiges DNA-Molekül, das ein erstes doppelsträngiges DNA-Molekül (A) und ein zweites doppelsträngiges DNA-Molekül (B) umfasst, wobei das doppelsträngige DNA-Molekül (A) eine Spaltstelle umfasst, die nur im doppelsträngigen DNA-Molekül (A) vorhanden ist, wobei das doppelsträngige DNA-Molekül (A) mit dem doppelsträngigen DNA-Molekül (B) durch zwei kovalente Bindungen, die keine Phosphodiester-Bindungen, Phosphorothioat-Bindungen, Phosphoramidat-Bindungen oder Phosphordiamidat-Bindungen sind, beiderseits der Spaltstelle verbunden ist, wobei die kovalenten Bindungen nicht an den letzten Nukleotiden der Enden des doppelsträngigen DNA-Moleküls (A) stattfinden.

11. Verfahren zur Herstellung des doppelsträngigen DNA-Moleküls nach einem der Ansprüche 1 bis 8, **dadurch gekennzeichnet, dass** es den folgenden Schritt umfasst:
a) das Spalten des doppelsträngigen DNA-Moleküls (A) nach Anspruch 10 an der Spaltstelle, wodurch ein doppelsträngiges DNA-Molekül, das ein erstes doppelsträngiges DNA-Molekül (1) und ein zweites doppelsträngiges DNA-Molekül (2) umfasst, erzeugt wird.

12. Verfahren zur Charakterisierung einer Interaktion zwischen mindestens zwei zu testenden Molekülen, die an ein doppelsträngiges DNA-Molekül nach einem der Ansprüche 1 bis 8 gebunden sind oder wie in der Vorrichtung von Anspruch 9 umfasst, umfassend:
a) das Anwenden einer schwachen physikalischen Kraft F_{LF} auf das doppelsträngige DNA-Molekül, die es den zu testenden Molekülen erlaubt, sich zu vereinen;
b) das Anwenden einer starken physikalischen Kraft F_{HF} auf das doppelsträngige DNA-Molekül, die erlaubt zu ermitteln, ob die zu testenden Moleküle vereint oder getrennt sind; und
c) das Ermitteln einer Änderung in den Konformationsmerkmalen des DNA-Moleküls, umfassend:
- das Bestimmen der Extension z_{LF} zwischen dem zweiten Ende des ersten doppelsträngigen DNA-Moleküls (1) und dem zweiten Ende des zweiten doppelsträngigen DNA-Moleküls (2) in Schritt a);
- das Bestimmen der Extensionen z_{HF-A} und z_{HF-D} zwischen dem zweiten Ende des ersten doppelsträngigen DNA-Moleküls (1) und dem zweiten Ende des zweiten doppelsträngigen DNA-Moleküls (2) in Schritt b), wobei z_{HF-A} die Extension ist, wenn die Moleküle vereint sind, und z_{HF-D} die Extension ist, wenn die Moleküle getrennt sind; und
- das Vergleichen der Extensionen z_{LF}, z_{HF-A} und z_{HF-D} in Abhängigkeit von der Zeit t.

13. Verfahren nach Anspruch 12, wobei das Verfahren ferner den folgenden zusätzlichen Schritt umfasst:
d) das Ermitteln einer Änderung in den Konformationsmerkmalen des DNA-Moleküls, umfassend:
- das Bestimmen der Extensionen z_{LF-A} und z_{LF-D} zwischen dem zweiten Ende des ersten doppelsträngigen DNA-Moleküls (1) und dem zweiten Ende des zweiten doppelsträngigen DNA-Moleküls (2) in Schritt a), wobei z_{LF-A} die Extension ist, wenn die die Moleküle vereint sind, und z_{LF-D} die Extension ist, wenn die Moleküle getrennt sind; und
- das Bestimmen der Extensionen z_{HF-A} und z_{HF-D} zwischen dem zweiten Ende des ersten doppelsträngigen DNA-Moleküls (1) und dem zweiten Ende des zweiten doppelsträngigen DNA-Moleküls (2) in Schritt b), wobei z_{HF-A} die Extension ist, wenn die Moleküle vereint sind und z_{HF-D} die Extension ist, wenn die Moleküle getrennt sind; und
- das Vergleichen der Extensionen z_{LF-A}, z_{LF-D}, z_{HF-A} und z_{HF-D} in Abhängigkeit von der Zeit t.

14. Verfahren zur Charakterisierung einer Interaktion zwischen mindestens zwei zu testenden Molekülen, die an ein doppelsträngiges DNA-Molekül nach einem der Ansprüche 1 bis 8 gebunden sind oder wie in der Vorrichtung von Anspruch 9 umfasst, umfassend:
a) das Anwenden einer konstanten Kraft F_{CF} auf das doppelsträngige DNA-Molekül, die es den zu testenden Molekülen erlaubt, sich zu vereinen und sich zu trennen; und
b) das Ermitteln einer Änderung in den Konformationsmerkmalen des DNA-Moleküls, umfassend:
- das Bestimmen der spontanen Trennung der zu testenden Moleküle am Ende der Zeit t_{CF-A}, und/oder
- das Bestimmen der spontanen Vereinigung am Ende der Zeit t_{CF-D}.

15. Verfahren nach einem der Ansprüche 12 bis 14, wobei die Charakterisierung der Interaktion das Bestimmen von mindestens einem der Merkmale umfasst, die ausgewählt sind aus: der charakteristischen Vereinigungszeit, der charakteristischen Trennungszeit, der Trennungsgeschwindigkeitskonstante, der Trennungsaktivierungsenergie, dem Abstand, der den Transitionszustand vom Komplex bei der Trennung trennt und der Trennungsgleichgewichtskonstante.

## Claims

1. Double-stranded DNA molecule comprising a first double-stranded DNA molecule
(1) connected to a second double-stranded DNA molecule (2) by a tether comprising double-stranded DNA, wherein said tether is attached by
(i) at least one covalent bond to a nucleotide of the first double-stranded DNA molecule (1) and by
(ii) at least one covalent bond to a nucleotide of the second double-stranded DNA molecule (2),
wherein said covalent bond of (i) and said covalent bond of (ii) are not phosphodiester bonds, phosphorothioate bonds, phosphoramidate bonds or phosphorodiamidate bonds, and said nucleotide of (i) and said nucleotide of (ii) are not the ultimate nucleotides of each extremity of said double-stranded DNA molecules (1) and (2) .

2. Double-stranded DNA molecule according to claim 1, wherein said tether is a double-stranded DNA molecule.

3. Double-stranded DNA molecule according to claim 1 or 2, wherein said tether is attached to the first double-stranded DNA molecule (1) by a first covalent bond between the first extremity of said tether and an intermediate region of the first double-stranded DNA molecule (1) and to the second double-stranded DNA molecule (2) by a second covalent bond between the second extremity of said tether and an intermediate region of the second double-stranded DNA molecule (2).

4. Double-stranded DNA molecule according to any one of claims 1 to 3, wherein a first test molecule is linked to a first extremity of said first double-stranded DNA molecule (1) and a second test molecule is linked to a first extremity of said second double-stranded DNA molecule (2).

5. Double-stranded DNA molecule according to claim 4, wherein the second extremity of said first double-stranded DNA molecule (1) is linked to a first support and the second extremity of said second double-stranded DNA molecule (2) is linked to a second support, preferably wherein at least one of the two supports is a movable support.

6. Double-stranded DNA molecule according to any one of claims 4 or 5, wherein:
• said first double-stranded DNA molecule (1) and/or said second double-stranded DNA molecule (2) has a length of 300 to 5000 base pairs, preferably 650 to 1500 base pairs;
• the first extremity of the first double-stranded DNA molecule (1) and/or the first extremity of the second double-stranded DNA molecule (2) at a length of 10 to 150 base pairs, preferably 30 at 50 base pairs; and/or
• said tether has a length of about 300 to about 50,000 base pairs, preferably about 500 to 10,000 base pairs, more preferably about 600 to 3000 base pairs.

7. Double-stranded DNA molecule according to any one of claims 4 to 6, wherein said first and/or second test molecule is selected from the group consisting of the following molecules: polymers, amino acids, peptides, polypeptides, proteins, nucleosides, nucleotides, polynucleotides, oligonucleotides, sugars, polysaccharides, small molecules, drugs, aptamers, antigens, antibodies, lipids, lectins, hormones, vitamins, viruses, virus fragments, nanoparticles, cell surface molecules, and transcription factors.

8. Double-stranded DNA molecule according to any one of claims 1 to 7, for use in measuring interactions between at least two test molecules.

9. Device comprising the double-stranded DNA molecule according to any one of claims 1 to 8 with its supports.

10. Double-stranded DNA molecule comprising a first double-stranded DNA molecule (A) and a second double-stranded DNA molecule (B), said double-stranded DNA molecule (A) comprising a cleavage site which is present only in said double-stranded DNA molecule (A), said double-stranded DNA molecule (A) being connected to the double-stranded DNA molecule (B) by two covalent bonds which are not phosphodiester bonds, phosphorothioate bonds, phosphoramidate bonds or phosphorodiamidate bonds, on either side of said cleavage site, wherein said covalent bonds do not occur at the ultimate nucleotides of the extremities of said double-stranded DNA molecule (A).

11. Process for manufacturing the double-stranded DNA molecule according to any one of claims 1 to 8, **characterized in that** it comprises the following step:
a) cleaving the double-stranded DNA molecule (A) according to claim 10 at said cleavage site, thereby generating a double-stranded DNA molecule comprising a first double-stranded DNA molecule (1) and a second double-stranded DNA molecule (2).

12. Method of characterizing an interaction between at least two test molecules linked to a double-stranded DNA molecule according to any one of claims 1 to 8 or as comprised in the device of claim 9, comprising:
a) applying a low physical force, F_{LF}, to the double-stranded DNA molecule, which allows the test molecules to associate;
b) applying a high physical force, F_{HF}, to the double-stranded DNA molecule, which makes it possible to determine whether the test molecules are associated or dissociated; and
c) detecting a change in the conformational properties of the DNA molecule comprising:
- determining z_{LF} extension between the second extremity of the first double-stranded DNA molecule (1) and the second extremity of the second double-stranded DNA molecule (2) in step a);
- determining z_{HF-A} and z_{HF-D} extensions between the second extremity of the first double-stranded DNA molecule (1) and the second extremity of the second double-stranded DNA molecule (2), in step b), wherein z_{HF-A} is the extension when the molecules are associated and z_{HF-D} is the extension when the molecules are dissociated; and
- comparing z_{LF}, z_{HF-A}, and z_{HF-D} extensions, as a function of time t.

13. Method according to claim 12, said method further comprising the following additional step:
d) detecting a change in the conformational properties of the DNA molecule comprising:
- determining z_{LF-A} and z_{LF-D} extensions between the second extremity of the first double-stranded DNA molecule (1) and the second extremity of the second double-stranded DNA molecule (2) in step a), wherein z_{LF-A} is the extension when the molecules are associated and z_{LF-D} is the extension when the molecules are dissociated;
- determining z_{HF-A} and z_{HF-D} extensions between the second extremity of the first double-stranded DNA molecule (1) and the second extremity of the second double-stranded DNA molecule (2), in step b), wherein z_{HF-A} is the extension when the molecules are associated and z_{HF-D} is the extension when the molecules are dissociated; and
- comparing z_{LF-A}, z_{LF-D}, z_{HF-A}, and z_{HF-D} extensions, as a function of time t.

14. Method of characterizing an interaction between at least two test molecules linked to a double-stranded DNA molecule according to any one of claims 1 to 8 or as comprised in the device of claim 9, comprising:
a) applying a constant force F_{CF}, to the double-stranded DNA molecule, which allows the test molecules to associate and dissociate; and
b) detecting a change in the conformational properties of the DNA molecule comprising:
- determining the spontaneous dissociation of the test molecules after time t_{CF-A}, and/or
- determining the spontaneous association after time t_{CF-D}.

15. Method according to any one of claims 12 to 14, wherein the characterization of the interaction comprises determining at least one of the characteristics selected from: the characteristic association time, the characteristic dissociation time, the dissociation rate constant, the dissociation activation energy, the distance separating the transition state from the complex during dissociation, and the equilibrium dissociation constant.
